(19) Europäisches Patentamt
European Patent Office
Office européen des brevets

(11) Publication number : **0 346 899 B1**

(12) # EUROPEAN PATENT SPECIFICATION

(45) Date of publication of patent specification : **31.08.94 Bulletin 94/35**

(21) Application number : **89110883.9**

(22) Date of filing : **15.06.89**

(51) Int. Cl.[5] : **G03C 7/32**, G03C 7/26, // C07D401/02 , (C07D401/02, 249:00, 213:00)

(54) **Silver halide color photographic light-sensitive material.**

(30) Priority : **16.06.88 JP 148906/88**

(43) Date of publication of application : **20.12.89 Bulletin 89/51**

(45) Publication of the grant of the patent : **31.08.94 Bulletin 94/35**

(84) Designated Contracting States : **DE FR GB NL**

(56) References cited :
**EP-A- 0 287 833**
**GB-A- 2 032 914**

(73) Proprietor : **FUJI PHOTO FILM CO., LTD.**
**210 Nakanuma**
**Minami Ashigara-shi**
**Kanagawa 250-01 (JP)**

(72) Inventor : **Shimada, Yasuhiro c/o Fuji Photo Film Co., Ltd.**
**No. 210, Nakanuma**
**Minami Ashigara-shi Kanagawa (JP)**

(74) Representative : **Hansen, Bernd, Dr.rer.nat. et al**
**Hoffmann, Eitle & Partner**
**Patentanwälte**
**Postfach 81 04 20**
**D-81904 München (DE)**

EP 0 346 899 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

### FIELD OF THE INVENTION

The present invention relates to a silver halide color photographic light-sensitive material, and more particularly to a silver halide color photographic light-sensitive material which is excellent in sharpness and color reproducibility.

### BACKGROUND OF THE INVENTION

In the field of color photographic light-sensitive materials based on a substractive process, it has been hitherto known to employ compounds which are capable of releasing a photographically useful group upon reaction with an oxidation product of a developing agent. Such a released, photographically useful groups include, for example, development inhibitors, fogging agents, bleach accelerating agents, dyes and fluorescent whitening agents, which are employed for their respective purposes. Exemplary methods are described in U.S. Patent 4,248,962.

Couplers which release a development inhibitor from non-color forming coupler moieties which do not substantially form a dye are also known and described, for example, in U.S. Patents 3,632,345, 3,958,993, 3,928,041, 4,187,110, 4,259,437 and 4,226,934.

However, these couplers described in such patents are not sufficient in view of their reaction rate with the oxidation products of developing agents, and thus further improvement has been desired.

Further, couplers described in JP-A-52-82423 (the term "JP-A" as used herein means an "unexamined published Japanese patent application") have a sufficiently high coupling rate, but provide a dye having a large molecular absorption coefficient. Accordingly, they are not suitable for non-color forming couplers.

Couplers capable of releasing a photographically useful group are ordinarily employed in a combination with image forming couplers. Therefore, they can be employed only for the purpose of releasing a photographically useful group other than that which forms a dye, i.e., it is not always necessary to form a dye. Specifically, couplers which are substantially non-color forming, which form a dye having a small molecular absorption coefficient, or which form a dye that is unstable and immediately decomposes can be employed in any of a blue, green or red-sensitive layer and an intermediate layer without limitation.

### SUMMARY OF THE INVENTION

Therefore, an object of the present invention is to provide a silver halide color photographic material which is excellent in sharpness.

Another object of the present invention is to provide a silver halide color photographic material of high sensitivity without damaging its color reproducibility and desilvering properties.

Other objects of the present invention will become apparent from the following detailed description and examples.

The above-described objects of the present invention can be accomplished by a silver halide color photographic light-sensitive material comprising a support having thereon at least one silver halide emulsion layer, wherein the silver halide color photographic material contains a compound represented by the following general formula (II):

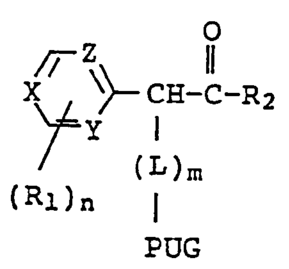

(II)

wherein X, Y and Z, which may be the same or different, each represents a methine group or a nitrogen atom provided that at least one of X, Y and Z is a nitrogen atom; $R_1$ represents a group capable of substituting on the hetero ring; $R_2$ represents an organic moiety; L represents a group capable of being cleaved from

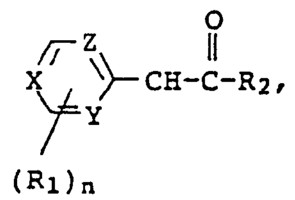

when the compound represented by the general formula (II) is reacted with an oxidation product of a developing agent and capable of releasing PUG after the cleavage from the methine group; PUG represents a photographically useful group; m represents an integer from 0 to 2; and n represents an integer from 0 to 4, when m or n represents 2 or more, two or more $R_1$'s or L's may be the same or different.

The compounds represented by the general formula (II) can be substantially employed as non-color forming couplers since dyes obtained therefrom upon the reaction with the oxidation products of developing agents have their maximum absorption in a comparative shorter wavelength region and a small molecular absorption coefficient.

Now, the compound represented by the general formula (II) will be described in greater detail below.

In the general formula (II), the group represented by $R_1$ is preferably a halogen atom (for example, chlorine, or bromine), an aliphatic group, an aromatic group, a heterocyclic group, a cyano group, an alkoxy group (for example, methoxy, ethoxy, or 2-methoxyethoxy), an aryloxy group (for example, phenoxy, or 4-tert-butylphenoxy), an aliphatic or aromatic acyloxy group (for example, acetoxy), a carbamoyloxy group, an aliphatic or aromatic sulfonyloxy group, an aliphatic or aromatic acylamino group (for example, acetamide, propylamido, or tert-butylamido), an aliphatic or aromatic sulfonamido group, a carbamoyl group, an aliphatic or aromatic acyl group (for example, acetyl, or benzoyl), an aliphatic or aromatic sulfonyl group, an alkoxycarbonyl group (for example, methoxycarbonyl, or butyloxycarbonyl), or an aryloxycarbonyl group. When n represents 2 or more, two or more $R_1$'s may be the same or different, and when n represents 2, two $R_1$'s may combine with each other to form a cyclic structure.

In the general formula (II), the organic group represented by $R_2$ is preferably an aromatic group, a heterocyclic group, an aliphatic group, $-OR_3$ or

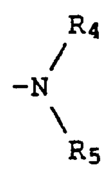

(wherein $R_3$ and $R_4$ each represents an aromatic group, a heterocyclic group or an aliphatic group; $R_5$ represents an aromatic group, a heterocyclic group, an aliphatic group or a hydrogen atom; or $R_4$ and $R_5$ may combine with each other to form a cyclic structure.

The aliphatic group as used herein is an aliphatic hydrocarbon group having from 1 to 40 carbon atoms, preferably from 1 to 20 carbon atoms in the hydrocarbon moiety thereof, and may be saturated or unsaturated, chain-like or cyclic, straight or branched chain, substituted or unsubstituted. Representative examples of the unsubstituted aliphatic group include a methyl group, an ethyl group, a propyl group, an isopropyl group, a butyl group, a tert-butyl group, an isobutyl group, a tert-amyl group, a hexyl group, a cyclohexyl group, a 2-ethylhexyl group, an octyl group, a 1,1,3,3-tetramethylbutyl group, a decyl group, a dodecyl group, a hexadecyl group, or an octadecyl group.

The aromatic group as used herein is an aromatic group having from 6 to 20 carbon atoms, and preferably an unsubstituted or substituted phenyl group or an unsubstituted or substituted naphthyl group.

The heterocyclic group as used herein is a heterocyclic group having from 1 to 20 carbon atoms, preferably from 1 to 7 carbon atoms, and containing at least one of a nitrogen atom, an oxygen atom and a sulfur atom, as a hetero atom, and preferably a three-membered to eight-membered, substituted or unsubstituted heterocyclic group. Representative examples of the heterocyclic groups include a 2-pyridyl group, a 4-pyridyl group, a 2-thienyl group, a 2-furyl group, a 2-imidazolyl group, a pyrazinyl group, a 2-pyrimidinyl group, a 1-imidazolyl group, a 1-indolyl group, a phthalimido group, a 1,3,4-thiadiazol-2-yl group, a benzoxazol-2-yl group, a 2-quinolyl group, a 2,4-dioxo-1,3-imidazolidin-5-yl group, a 2,4-dioxo 1,3-imidazolidin-3-yl group, a succinimido group, a phthalimido group, a 1,2,4-triazol-2-yl group, or a 1-pyrazolyl group.

The aliphatic group, aromatic group and heterocyclic group as described above may have one or more substituents. Suitable examples of the substituents include a mono-valent organic group, for example, a halogen atom (for example, chlorine, fluorine, or bromine), an alkyl group (for example, methyl, ethyl, tert-octyl, tert-amyl, n-nonyl, or methoxymethyl), an alkoxy group (for example, methoxy, n-octyloxy, n-decyloxy, or n-pentadecyloxy), an aryloxy group (for example, phenoxy, or tert-octylphenoxy), an alkoxycarbonyl group (for example, methoxycarbonyl, n-dodecyloxycarbonyl, or n-hexadecyloxycarbonyl), an aryloxycarbonyl group (for example, phenoxycarbonyl, or 2,4-di-tert-amylphenoxycarbonyl), an aliphatic or aromatic sulfonamido group (for example, methanesulfonamido, n-butanesulfonamido, n-hexadecanesulfonamido, or benzenesulfonamido), a sulfamoyl group (for example, N,N-di-n-octylsulfamoyl group, or N-n-hexadecylsulfamoyl), an amino group (for example, unsubstituted amino, ethylamino, or di-n-octylamino), a carbamoyl group (for example, di-n-octylcarbamoyl, or diethylcarbamoyl), an aliphatic or aromatic acylamino group (for example, 2,4-di-tert-amylphenoxyacetamido, or n-pentadecylphenoxyacetamido), an aliphatic or aromatic sulfonyl group (for example, methylsulfonyl, or n-dodecylsulfonyl), a cyano group, an aryl group (for example, phenyl), an aralkyl group (for example, benzyl), a nitro group, a hydroxy group, a carboxy group, an aliphatic or aromatic acyl group (for example, acetyl), or a heterocyclic group, preferably those which are described for $R_3$ and $R_4$ (for example, n-octadecylsuccinimido).

In the general formula (II), the group represented by L include known linking groups described below.

(1) A group utilizing a cleavage reaction of hemiacetal.

Examples of these groups include those as described, for example, in U.S. Patent 4,146,396, JP-A-60-249148 (U.S. Patent 4,698,297) and JP-A-60-249149 (U.S. Patent 4,652,516), and are represented by the following general formula (T-1):

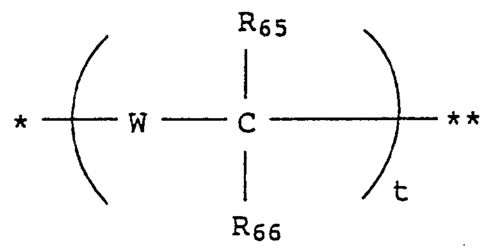

(T-1)

wherein a bond indicated by * denotes the position at which the group is connected to the upper side group in the general formula (II); a bond indicated by ** denotes the position at which the group is connected to the lower side group in the general formula (II); W represented an oxygen atom, a sulfur atom or a group of

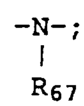

$R_{65}$ and $R_{66}$ each represents a hydrogen atom or a substituent; $R_{67}$ represents a substituent; t represents 1 or 2; and when t represents 2, two

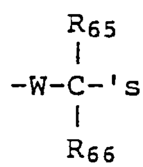

may be the same or different.

Representative examples of the substituents represented by $R_{65}$, $R_{66}$ or $R_{67}$ include a group of $R_{69}$, a group of $R_{69}CO$-, a group of $R_{69}SO_2$-, a group of

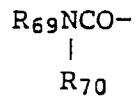

and a group of

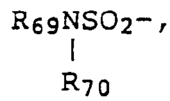

etc., wherein $R_{69}$ represents an aliphatic group, an aromatic group or a heterocyclic group as defined above; and $R_{70}$ represents a hydrogen atom, an aliphatic group, an aromatic group or a heterocyclic group as defined above.

Each of the groups represented by $R_{65}$, $R_{66}$ and $R_{67}$ may also represents a divalent group and be connected with each other to form a cyclic structure.

Specific examples of the groups represented by the general formula (T-1) are set forth below.

$*\text{-}OCH_2\text{-}**$

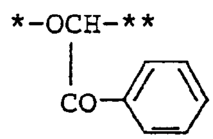

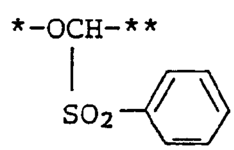

$*\text{-}SCH_2\text{-}**$

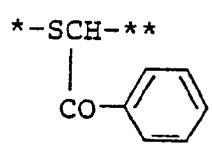

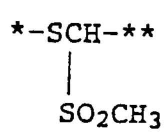

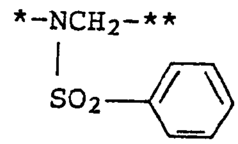

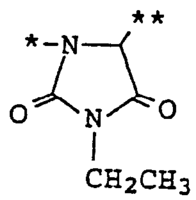

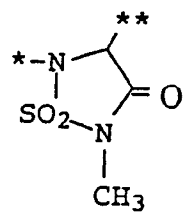

(2) A group causing a cleavage reaction utilizing an intramolecular nucleophilic displacement reaction.

Examples of these groups include the timing groups described in U.S. Patent 4,248,962, and are represented by the following general formula (T-2):

*-Nu-Link-E-** (T-2)

wherein a bond indicated by * denotes the position at which the group is connected to the upper side group in the general formula (II); a bond indicated by** denotes the position at which the group is connected to the lower side group in the general formula (II); Nu represents a nucleophilic group such as an oxygen atom or a sulfur atom; E represents an electrophilic group which is able to cleave the bond indicated by ** upon a nucleophilic attack of Nu; and Ling represents a linking group which connects Nu with E in a stereochemical position capable of causing an intramolecular nucleophilic displacement reaction between Nu and E.

Specific examples of the groups represented by the general formula (T-2) are set forth below.

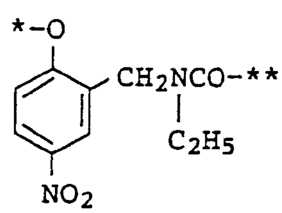

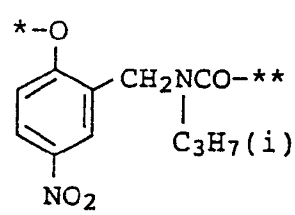

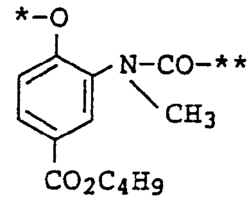

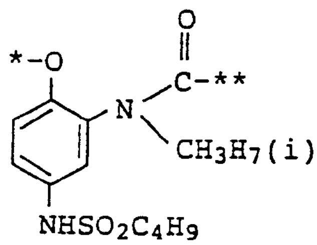

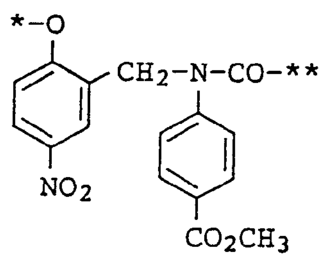

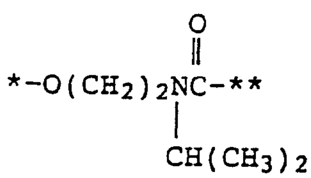

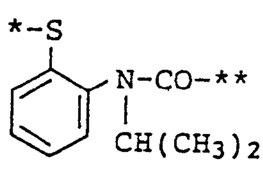

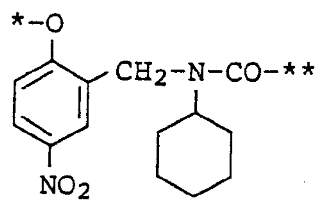

(3) A group causing a cleavage reaction utilizing an electron transfer reaction via a conjugated system.

Examples of these groups include those descried in U.S. Patents 4,409,323 and 4,421,845, and are represented by the following general formula (T-3):

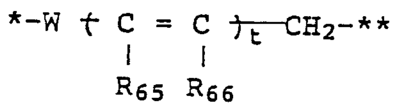

(T-3)

wherein a bond indicated by *, a bond indicated by **, W, $R_{65}$, $R_{66}$ and t each has the same meaning as defined in the general formula (T-1) above.

Specific examples of the groups represented by the general formula (T-3) are set forth below.

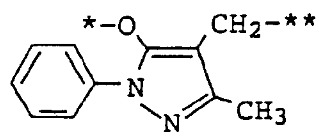

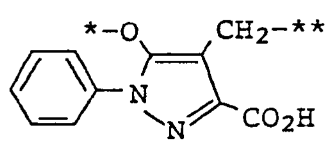

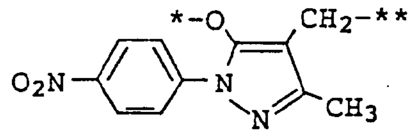

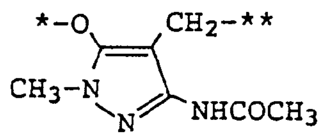

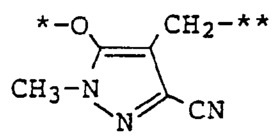

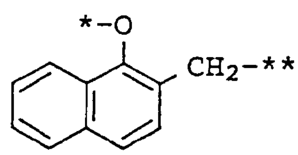

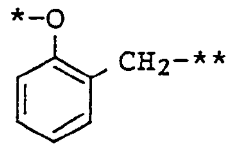

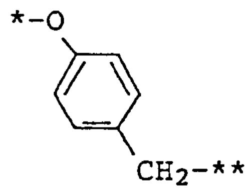

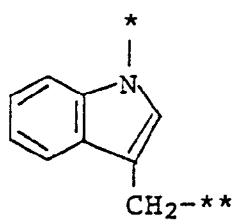

(4) A group utilizing a cleavage reaction of an ester upon hydrolysis.

Examples of these groups include those described in West German Patent Application (OLS) No. 2,626,315, and are represented by the following formula (T-4) or (T-5):

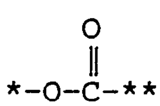

(T-4)

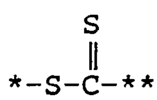

(T-5)

wherein a bond indicated by * and a bond indicated by ** each has the same meaning as defined in the general formula (T-1) above.

(5) A group utilizing a cleavage reaction of an iminoketal.

Examples of these groups include those described in U.S. Patent 4,546,073, and are represented by the following general formula (T-6):

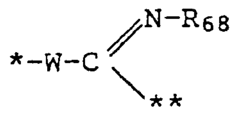

(T-6)

wherein a bond indicated by *, a bond indicated by ** and W each has the same meaning as defined in the general formula (T-1); and $R_{68}$ has the same meaning as defined for $R_{67}$ in the general formula (T-1) above.

Specific examples of the groups represented by the general formula (T-6) are set forth below.

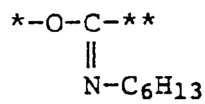

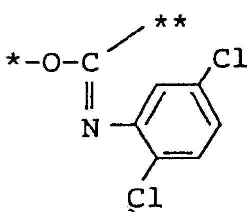

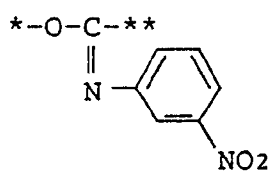

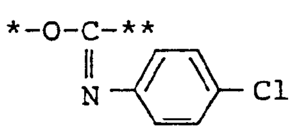

In the general formula (II), PUG represents a photographically useful group. Suitable examples of the photographically useful groups include those which provide development inhibitors, development accelerators, bleach restraining agents, bleach accelerating agents, developing agents, fixing agents, silver halide solvents, silver-complex forming agents, hardening agents, tanning agents, tanning agents, toning agent, fogging agents, antifogging agents, chemical sensitizers, desensitizers, photographic dyes or precursors thereof, and couplers (including competing couplers, color forming couplers, and development inhibitor releasing couplers).

In the case wherein PUG in the general formula (II) represents a development inhibitor, suitable examples of the releasing groups include those described, for example, in U.S.Patents 4,390,618 and 4,724,199, JP-A-59-168444, JP-A-60-128440, JP-A-60-191241, JP-A-61-212842, JP-A-62-166340, JP-A-62-168147, JP-A-62-205344 and JP-A-63-73245.

In the case wherein PUG in the general formula (II) represents a bleach accelerating agent, suitable examples of the releasing groups include those as described, for example, in JP-A-61-201247 (EP-A-193,389), JP-A-62-173467, JP-A-63-70239, JP-A-63-70854, JP-A-63-70851, JP-A-63-70852, JP-A-63-70853 and JP-A-63-73245.

In the general formula (II), the group represented by PUG is preferably a development inhibitor. Suitable examples of the development inhibitors include a phenyltetrazolylthio group, a 1,3,4-thiadiazolyl-2-thio group, a 1,3,4-oxadiazolyl-2-thio group, a 1,3,4-triazolyl-2-thio group, a benzimidazolyl-2-thio group, a benzoxazolyl-2-thio group, benzothiazolyl-2-thio group, a benzotriazolyl group, a benzoindazolyl group and an imidazolylthio group. These groups may be substituted with one or more substituents as those for the aromatic group represented by $R_2$ described above on the positions capable of being substituted.

In the general formula (II), a more preferred PUG is a group providing a compound having a development inhibiting function when being released as PUG. A development inhibitor which is capable of being decomposed (or changed into) a compound having substantially no effect on photographic properties after being discharged into a color developing solution is particularly preferred.

Examples of these development inhibitors include those as described in U.S. Patent 4,477,563, JP-A-60-218644, JP-A-60-221750, JP-A-60-233650 and JP-A-61-111743.

Preferred examples of the development inhibitors include those represented by the following general formulas (D-1), (D-2), (D-3), (D-4), (D-5), (D-6), (D-7), (D-8), (D-9), (D-10), (D-11) and (D-12):

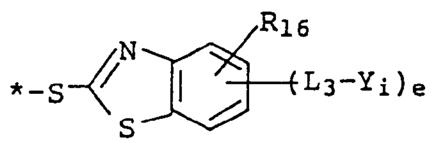

(D-1)

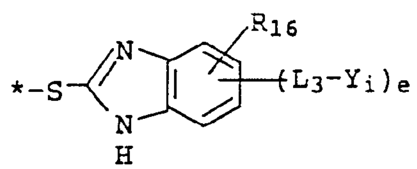

(D-2)

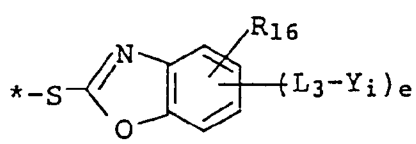

(D-3)

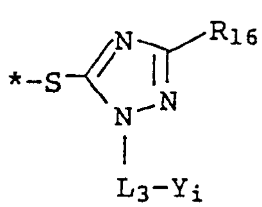

(D-4)

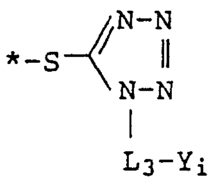

(D-5)

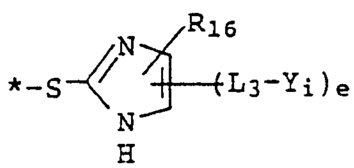

(D-6)

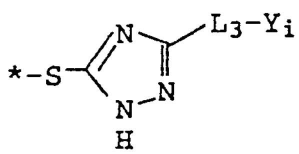

(D-7)

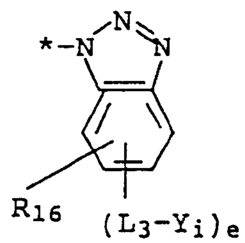

(D-8)

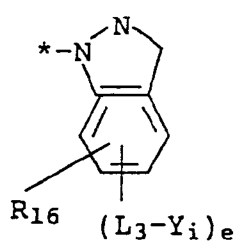

(D-9)

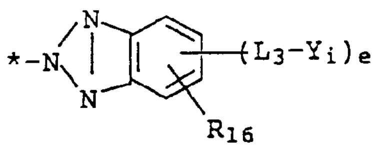

(D-10)

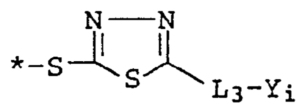

(D-11)

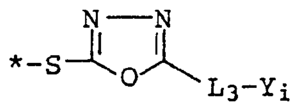

(D-12)

wherein a bond indicated by * denotes the position at which the group is connected to the methine group or the linking group (L) in the general formula (II); $R_{16}$ represents a hydrogen atom or a substituent; e represents 1 or 2; $L_3$ represents a group containing a chemical bond which is capable of being cleaved in a developing solution; and $Y_i$ represents a substituent capable of generating the development inhibiting function and is selected from an aliphatic group, an aromatic group or a heterocyclic group.

The development inhibitor described above which is released diffuses in a photographic layer while exhibiting the development inhibiting function and a part thereof discharges into the color developing solution. The development inhibitor discharged into the color developing solution rapidly decomposes at the chemical bond, included in $L_3$, to releases the group represented by $Y_i$ (for example, hydrolysis of an ester bond) upon a reaction with a hydroxy ion or hydroxylamine generally present in the color developing solution; whereby the compound changes into a compound having a large water-solubility and a small development inhibiting function, and thus the development inhibiting function substantially disappears.

While $R_{16}$ in the above described formulae is preferably a hydrogen atom, it may also be a substituent. Representative examples of the substituent include an aliphatic group (for example, methyl, or ethyl), an aliphatic or aromatic acylamino group (for example, acetamido, or propionamido), an alkoxy group (for example, methoxy, or ethoxy), a halogen atom (for example, chlorine, or bromine), a nitro group, or an aliphatic or aromatic sulfonamido group (for example, methanesulfonamido).

The linking group represented by $L_3$ in the above described general formulae includes a chemical bond which is cleaved in a developing solution. Suitable examples of such chemical bonds include those described in the table below. These chemical bonds are cleaved with a nucleophilic reagent such as hydroxy ion or hydroxylamine, etc. which is a component of the color developing solution.

TABLE

| Chemical bond Included in $L_3$ | Groups and Product Produced by Cleavage Reaction of Chemical Bond (Reaction With $^{\ominus}OH$) |
|---|---|
| $-COO-$ | $-COOH$ + $HO-$ |
| H<br>$-NCOO-$ | $-NH_2$ + $HO-$ |
| $-SO_2O-$ | $-SO_2H$ + $HO-$ |
| $-OCH_2CH_2SO_2-$ | $-OH$ + $CH_2=CHSO_2-$ |
| $-OCO-$<br>‖<br>O | $-OH$ + $CO_2$ + $HO-$ |
| $-NHCCO-$<br>‖ ‖<br>O O | $-NH_2$ + $HO-$ |

The chemical bond group shown in the table above is connected directly or through an alkylene group and/or a phenylene group with a heterocyclic moiety constituting development inhibitor or a benzene ring constituting development inhititor (in the case of benzene condensed ring) and connected directly with $Y_i$. When the chemical bond group is connected through an alkylene group and/or a phenylene group, the alkylene group and/or phenylene group may contain an ether bond, an amido bond, a carbonyl group, a thioether bond, a sulfon group, a sulfonamido bond or a ureido bond at the divalent group.

The aliphatic group represented by $Y_i$ is a hydrocarbon group having from 1 to 20 carbon atoms, preferably from 1 to 10 carbon atoms, and may be saturated or unsaturated, straight chain, branched chain or cyclic, or substituted or unsubstituted. A substituted hydrocarbon group is particularly preferred.

The aromatic group represented by $Y_i$ is a substituted or unsubstituted phenyl group or a substituted or unsubstituted naphthyl group.

The heterocyclic group represented by $Y_i$ is a substituted or unsubstituted four-membered to eight-membered heterocyclic group containing at least one of a sulfur atom, an oxygen atom and a nitrogen atom as a hetero atom.

Specific examples of the heterocyclic groups include a pyridyl group, an imidazolyl group, a furyl group, a pyrazolyl group, an oxazolyl group, a thiazolyl group, a thiadiazolyl group, a triazolyl group, a diazolidinyl group, and a diadinyl group.

Suitable examples of the substituents for the substituted aliphatic group, aromatic group or heterocyclic groups include a halogen atom, a nitro group, an alkoxy group having from 1 to 10 carbon atoms, an aryloxy group having from 6 to 10 carbon atoms, an alkanesulfonyl group having from 1 to 10 carbon atoms, an arylsulfonyl group having from 6 to 10 carbon atoms, an alkanamido group having from 2 to 10 carbon atoms, an anilino group, a benzamido group, a carbamoyl group, an alkyl carbamoyl group having from 2 to 10 carbon atoms, an aryl carbamoyl group having from 7 to 10 carbon atoms, an alkylsulfonamido group having from 1 to 10 carbon atoms, an arylsulfonamido group having from 6 to 10 carbon atoms, an alkylthio group having from 1 to 10 carbon atoms, an arylthio group having from 6 to 10 carbon atoms, a phthalimido group, a succinimido group, an imidazolyl group, a 1,2,4-triazolyl group, a pyrazolyl group, a benzotriazolyl group, a furyl group, a benzothiazolyl group, an alkylamino group having from 1 to 10 carbon atoms, an alkanoyl group having from 2 to 10 carbon atoms, a benzoyl group, an alkanoyloxy group having from 2 to 10 carbon atoms, a benzoyloxy group, a perfluoroalkyl group having from 1 to 5 carbon atoms, a cyano group, a tetrazolyl group, a hydroxy group, a mercapto group, an amino group, an alkylsulfamoyl group having from 1 to 10 carbon atoms, an arylsulfamoyl group having from 6 to 10 carbon atoms, a morpholino group, an aryl group having from 6 to 10 carbon atoms, a pyrrolidinyl group, a ureido group, a urethane group, an alkoxycarbonyl group having from

2 to 10 carbon atoms, an aryloxycarbonyl group having from 7 to 10 carbon atoms, an imidazolidinyl group and an alkylidene amino group having from 1 to 10 carbon atoms.

The compound represented by the general formula (II) according to the present invention can be applied to a multilayer multicolor photographic material containing on a support at least three light-sensitive layers having spectral sensitivity different from each other for the main purpose of improving sharpness, color reproducibility or graininess. A multilayer natural color photographic material generally possesses at least one red-sensitive silver halide emulsion layer, at least one green-sensitive silver halide emulsion layer and at least one blue-sensitive silver halide emulsion layer, respectively, on a support. The order of the layers can be appropriately varied, if desired. The compounds according to the present invention can be added to any appropriate layer including not only a light-sensitive emulsion layer but also a layer adjacent thereto, for example, an intermediate layer. Further, the compounds according to the present invention can be added to any light-sensitive layer of appropriate sensitivity, for example, a high-sensitive layer or a low-sensitive layer.

The amount of the compound represented by the general formula (II) according to the present invention to be added can be varied depending on the structure of the compound. However, it is preferred to use in a range from $1 \times 10^{-6}$ mol to 0.5 mol, particularly from $1 \times 10^{-5}$ mol to $1 \times 10^{-1}$ mol per mol of silver (silver halide calculated as silver and colloidal silver) present in the same layer to which it is to be added, or a layer adjacent thereto directly.

The compound represented by the general formula (II) according to the present invention can be employed in a combination with a color image forming coupler in a silver halide emulsion layer. In such a case, a molar ratio of the compound according to the present invention/color image forming coupler is generally from 0.01/99.99 to 50/50, preferably from 1/99 to 30/70.

Specific examples of the compounds, according to the present invention are set forth below, but the present invention should not be construed as being limited thereto.

(1)

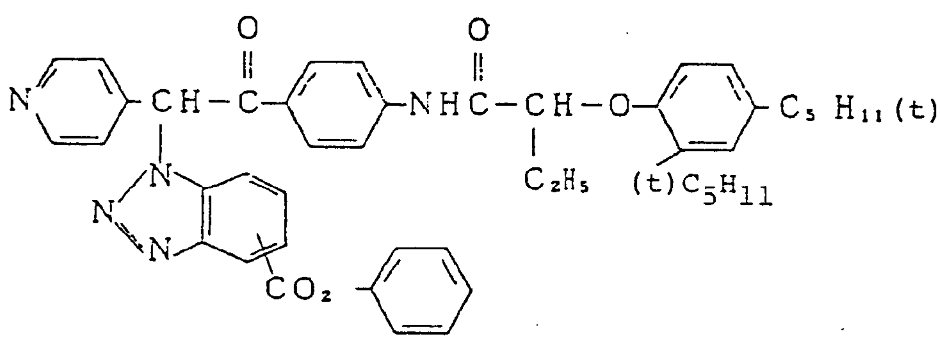

(2)

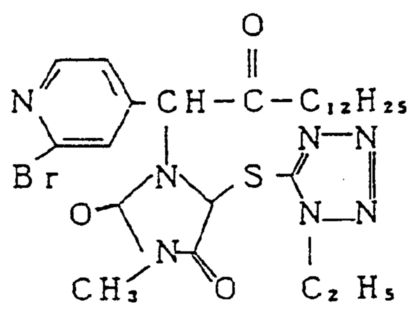

(3)

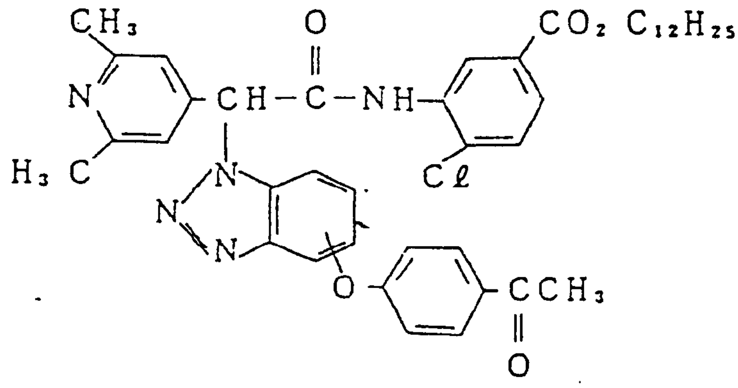

(4)

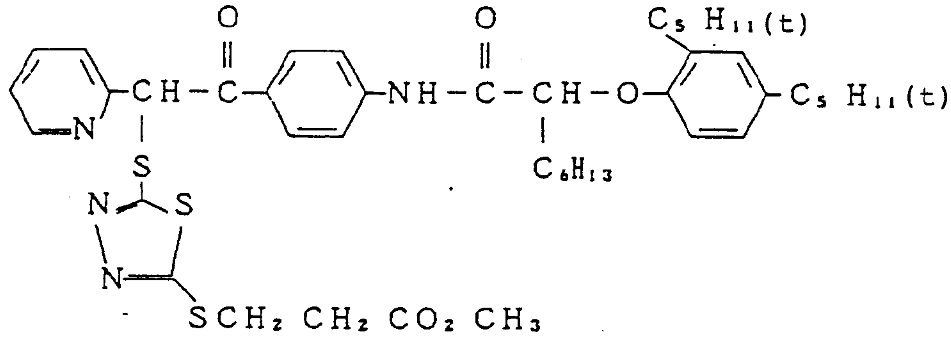

(5)

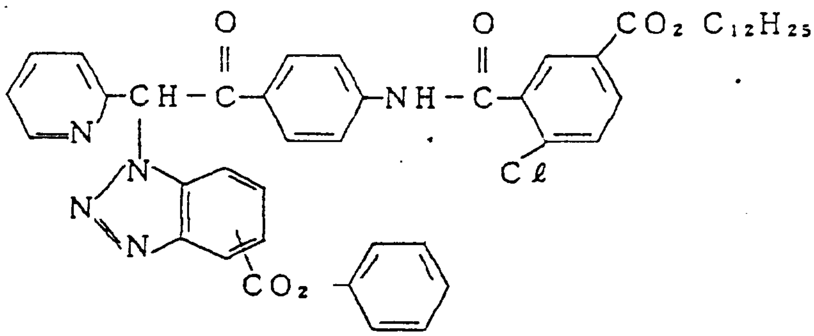

(6)

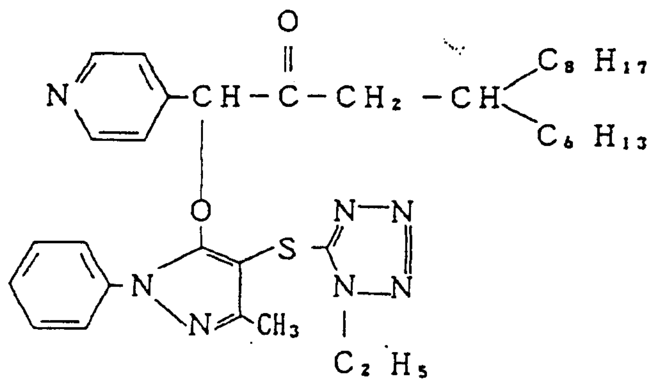

(7)

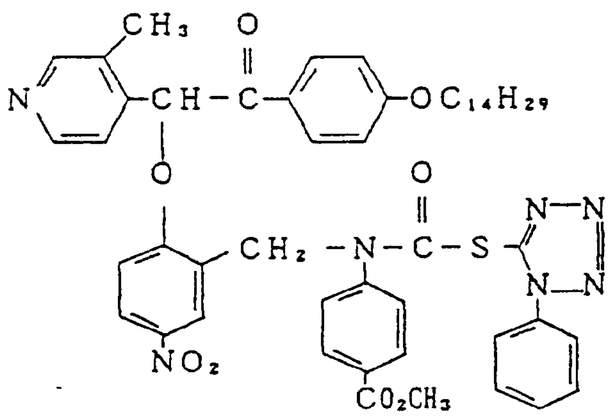

(8)

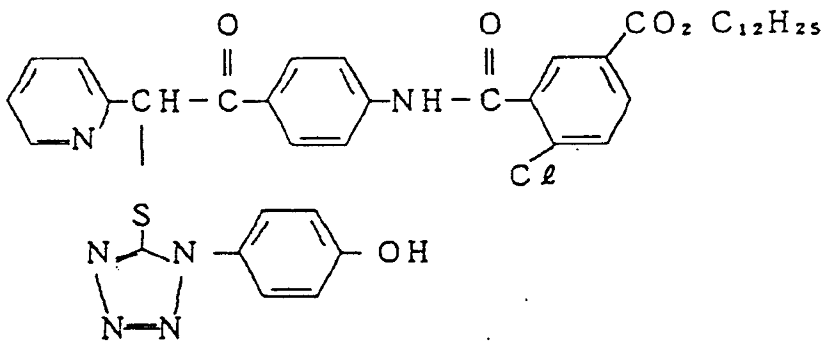

(9)

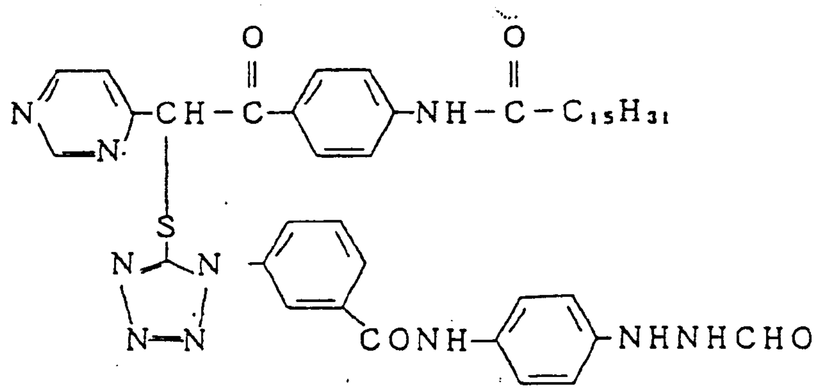

(10)

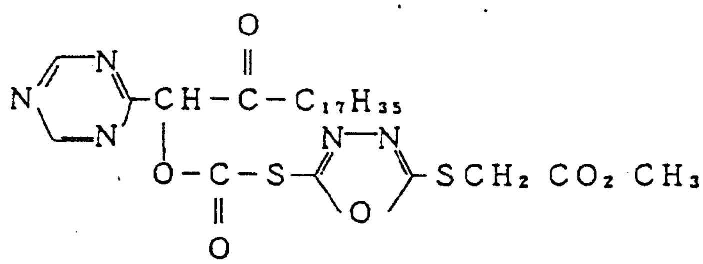

(11)

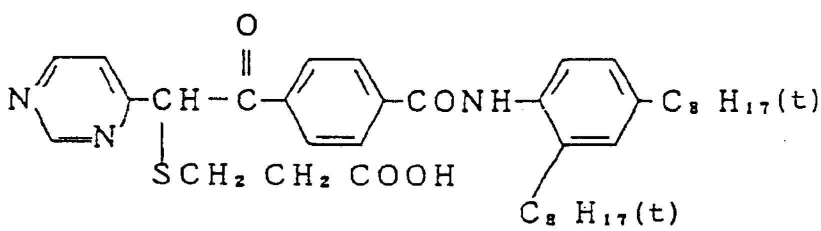

(12)

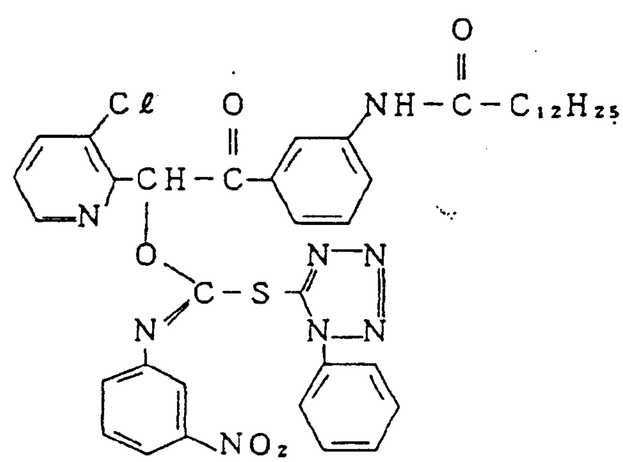

(1 3)

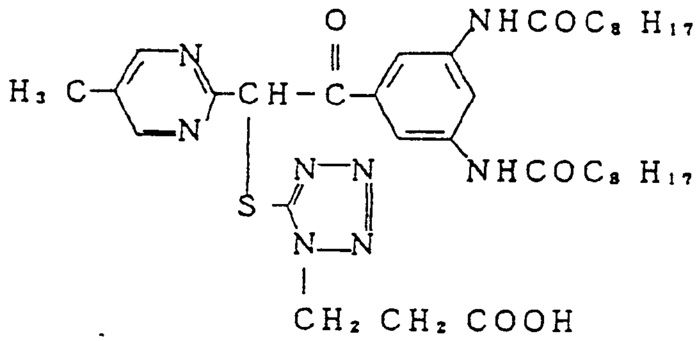

(1 4)

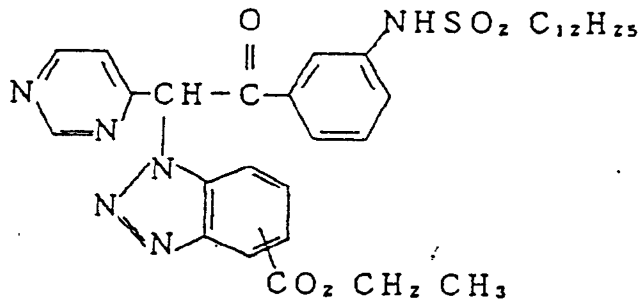

(1 5)

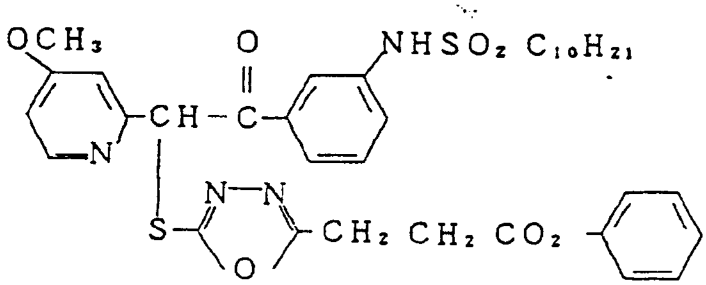

(1 6)

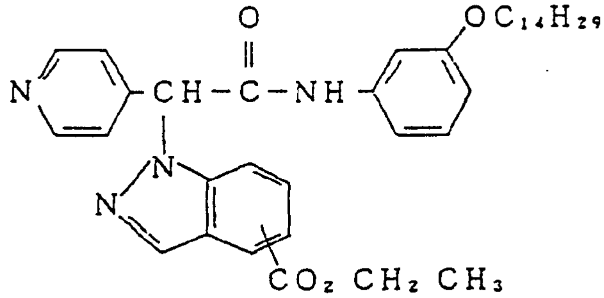

(17)

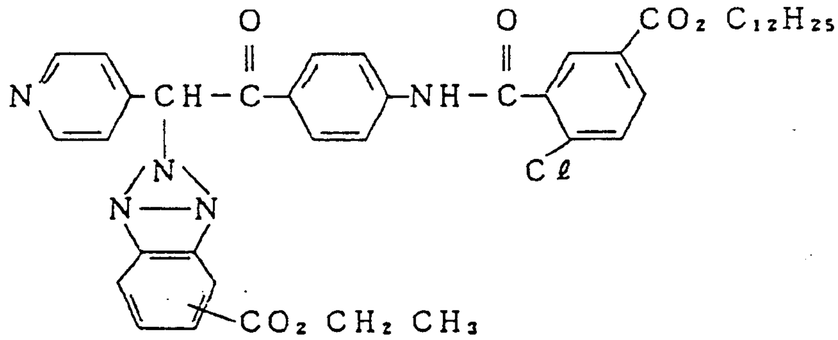

(18)

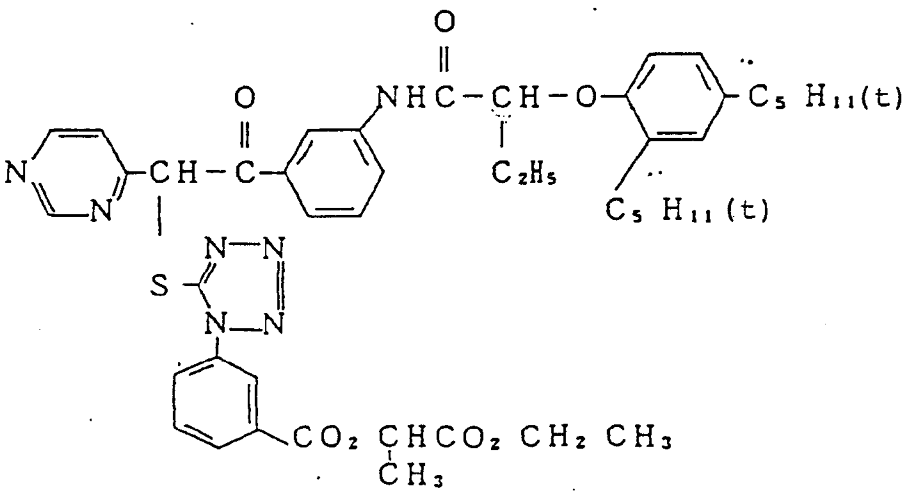

(19)

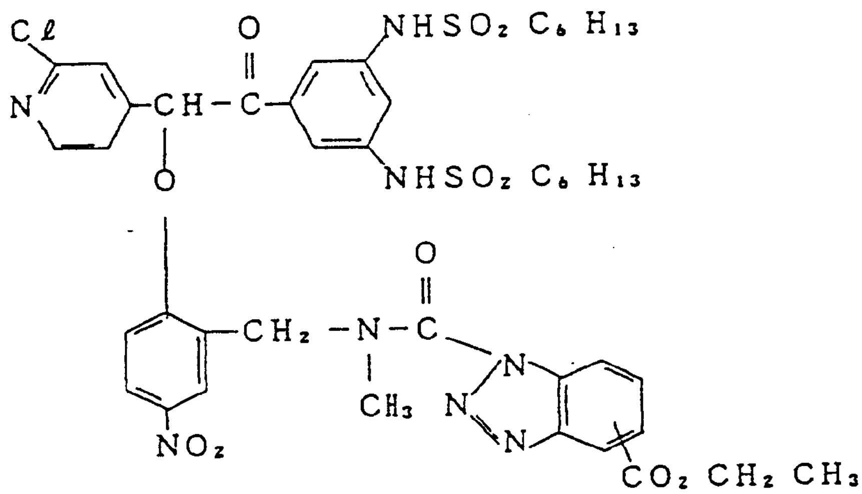

(20)

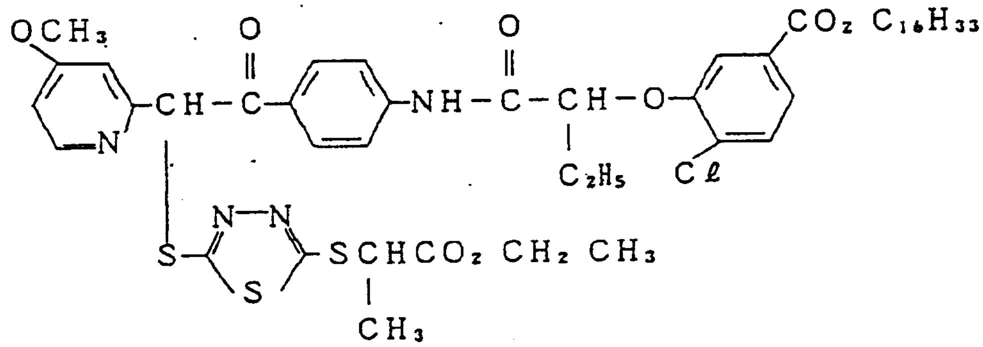

(21)

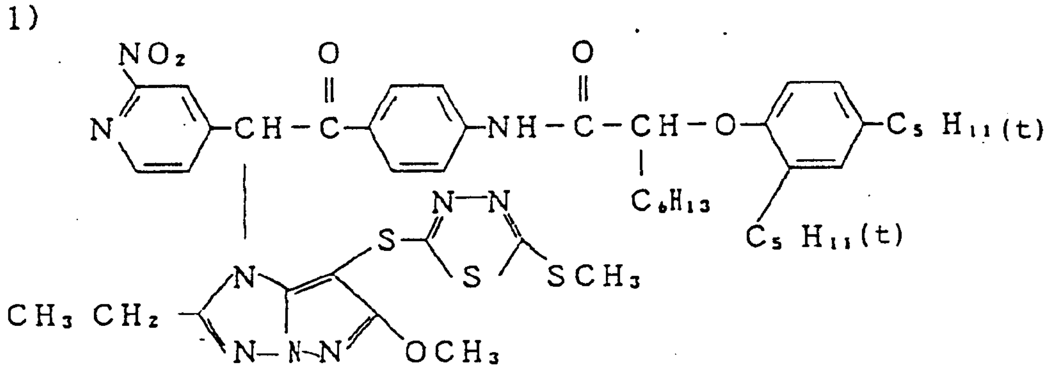

(22)

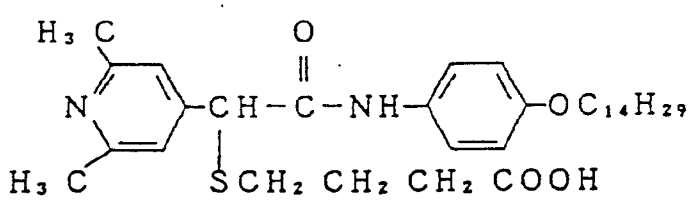

(23)

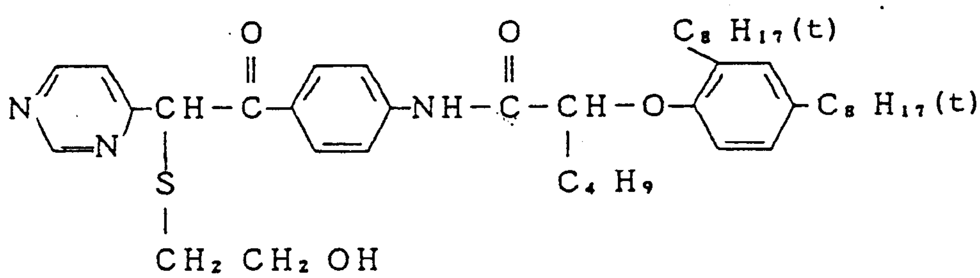

(24)

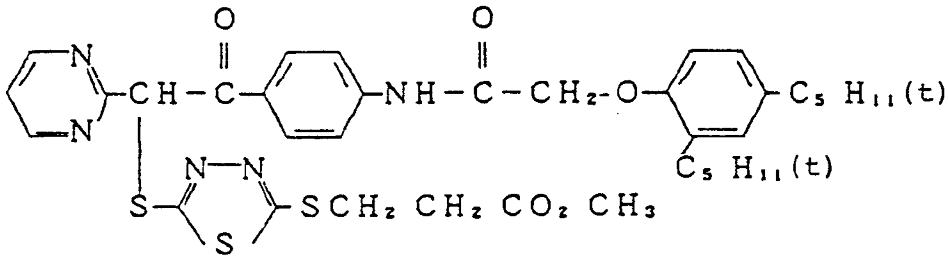

(25)

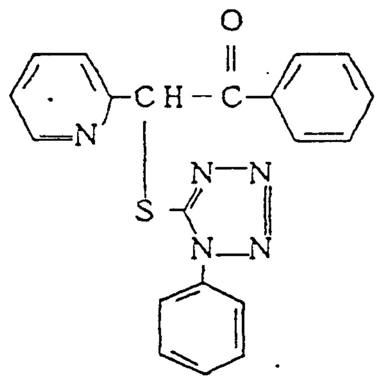

(26)

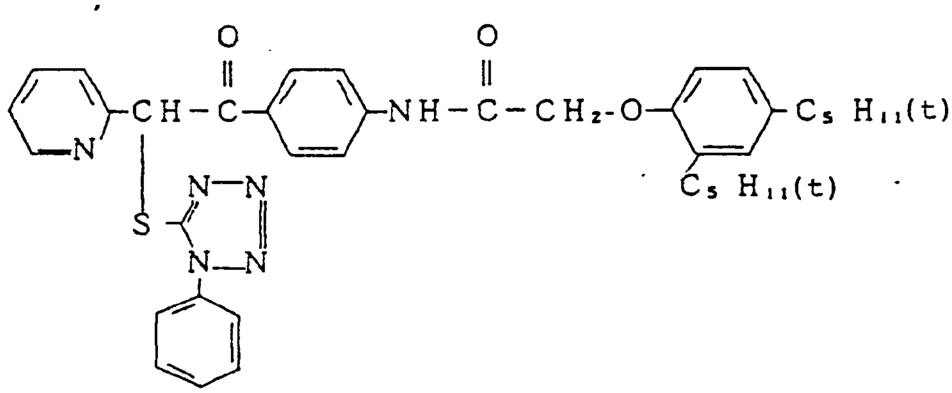

(27)

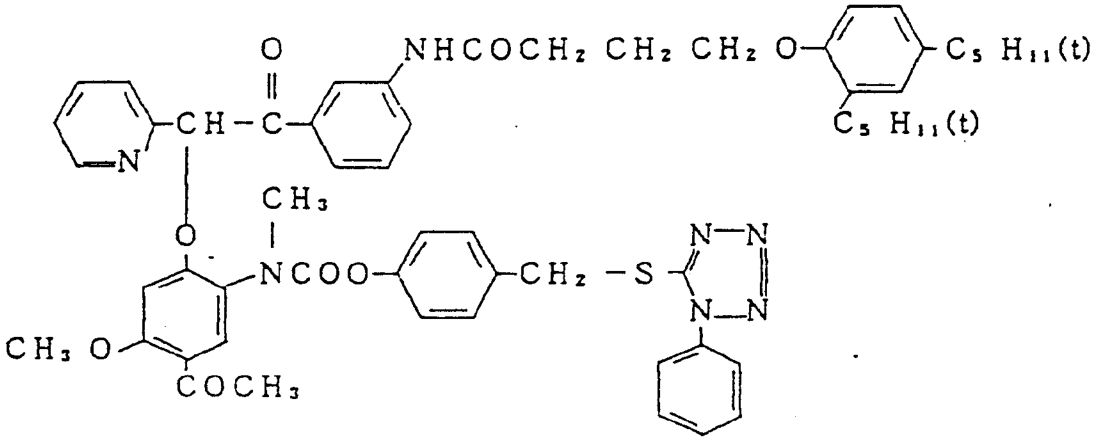

(28)

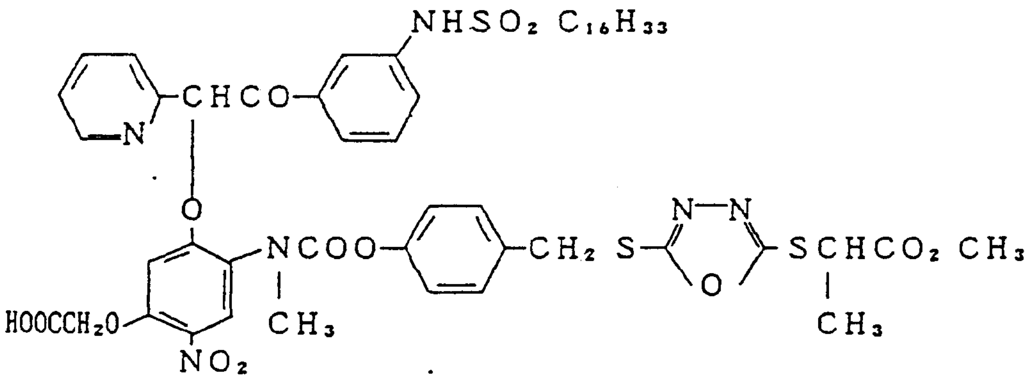

Typical synthesis examples of compounds of the present invention are illustrated below. Other compounds encompassed herein can be synthesized in a similar manner.

SYNTHESIS EXAMPLE 1

Synthesis of Compound (1)

Compound (1) was synthesized according to the route schematically shown below.

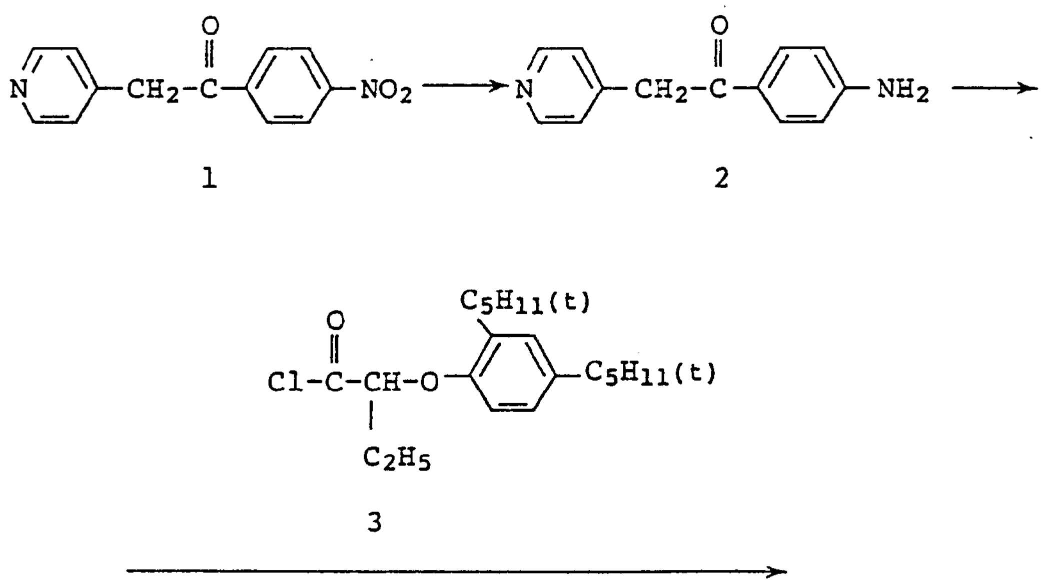

1

2

3

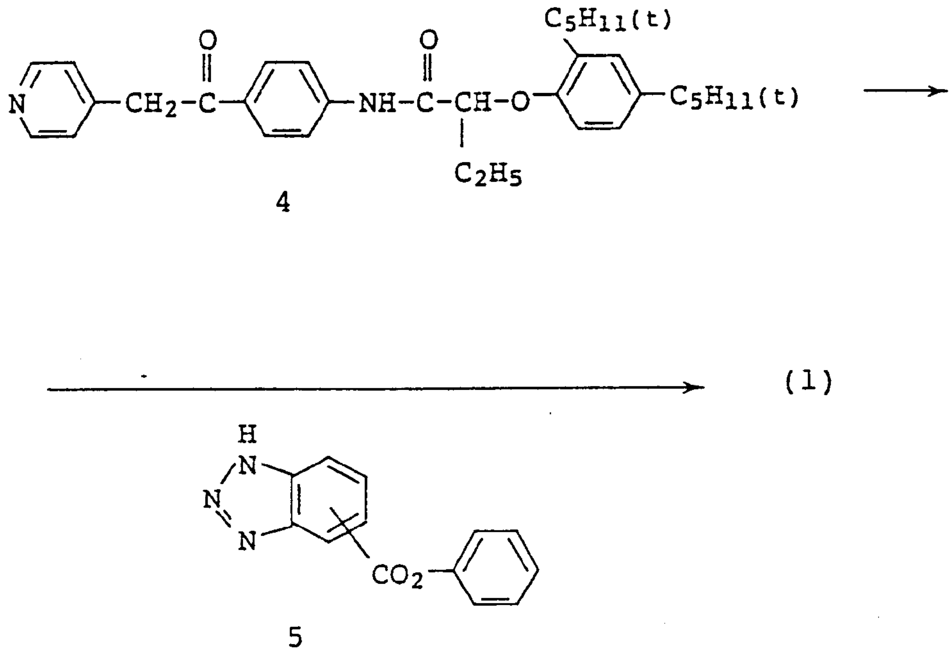

4

(1)

5

Step (1): Synthesis of Intermediate Compound 4

56.0 g of reduced iron and 3.6 g of ammonium chloride were added to a solution of 150 ml of isopropanol, 20 ml of water and 3.6 ml of acetic acid. After the mixture was refluxed by heating for 20 minutes, 15.0 g of Compound 1 was added thereto little by little. After the completion of the addition, the mixture was refluxed for 1 hour, and then filtered while hot to remove iron. To the filtrate was added dropwise 50 ml of acetonitrile containing 22.7 g of Compound 3 dissolved therein. After the reaction, 500 ml of ethyl acetate was added to the reaction mixture and then the mixture was washed with water. The organic layer was dried, and the solvent was distilled off under reduced pressure. The residue was recrystallized from a solvent mixture of ethyl acetate and hexane to obtain 24.2 g of Intermediate Compound 4.

Step (2): Synthesis of Compound (1)

To 100 ml of chloroform solution, containing 20.0 g of Intermediate Compound 4 dissolved therein, was added dropwise 20 ml of chloroform solution containing 6.2 g of bromine dissolved therein, while maintaining a reaction mixture temperature of 10°C. After the reaction, water was added to the reaction mixture, in order to wash the same. Then, the chloroform layer was gradually added dropwise to 50 ml of dimethyl formamide solution containing 9.3 g of Compound 5 and 10.7 ml of triethylamine dissolved therein. After the reaction, 200 ml of ethyl acetate was added to the reaction mixture, and the mixture thoroughly washed with an aqueous solution of sodium hydrogencarbonate. After the neutralization, the organic layer was dried with magnesium sulfate, and the solvent was distilled off under a reduced pressure. The residue was recrystallized from a solvent mixture of ethyl acetate and hexane to obtain 17.6 g of desired Compound (1).

SYNTHESIS EXAMPLE 2

Synthesis of Compound (5)

Compound (5) was synthesized according to the route schematically shown below.

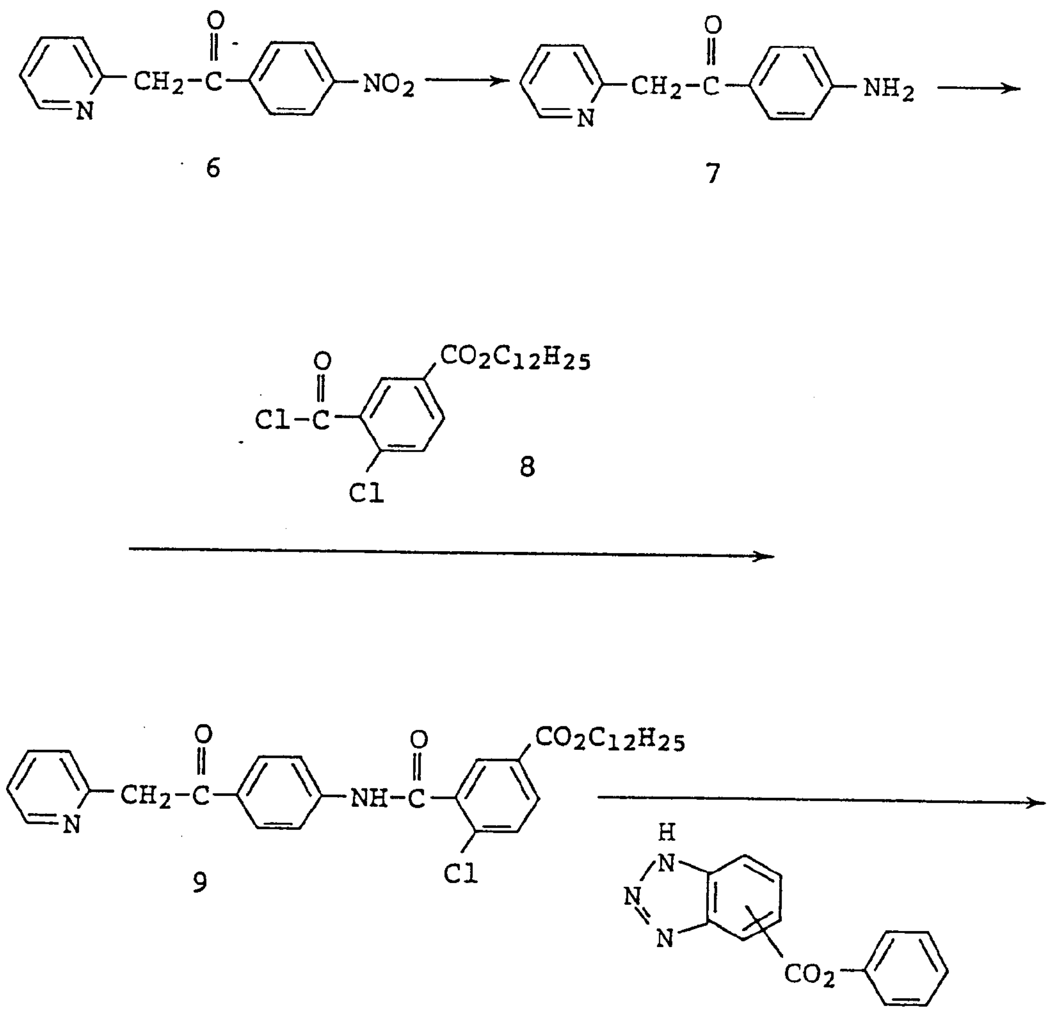

6 7 8 9 5

Step (1): Synthesis of Intermediate Compound 9

In the same manner as described in Step (1) of Synthesis Example 1, except using 20.0 g of Compound 6, 31.5 g of Intermediate Compound 9 was obtained.

Step (2): Synthesis of Compound (5)

In the same manner as described in Step (2) of Synthesis Example 1 except for using 30.0 g of Intermediate Compound 9, Compound (5) was synthesized. The oily product obtained, by the conventional after-treatment, was recrystallized from a solvent mixture of dichloromethane and hexane to obtain 26.4 g of desired Compound (5).

Reference Experiment

Dyes 10 and 11 which were obtained from Compounds (25) and (26) according to the present invention described above were synthesized. The maximum absorption wavelength, and the molecular absorption coefficient (ε) at the maximum absorption wavelength of each dye were measured. The results are shown in Table 1.

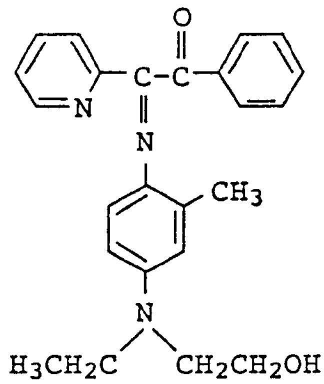

10

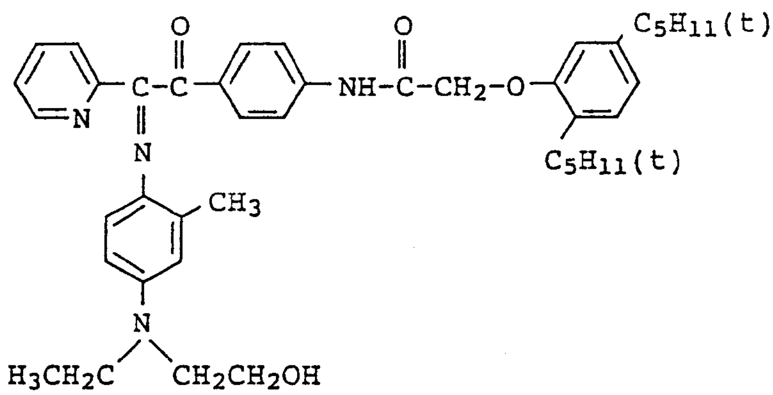

11

TABLE 1

| Dye No. | Maximum Absorption Wavelength (nm) | Molecular Absorption Coefficient (ε) |
|---|---|---|
| 10 | 419.2 | $6.60 \times 10^3$ |
| 11 | 422.3 | $7.68 \times 10^3$ |

From the results shown in Table 1, it can be seen that the dyes obtained from compounds according to the present invention and the oxidation product of the developing agent, have their absorption maxima in a comparative shorter wavelength region and small molecular absorption coefficients.

Accordingly, compounds of the present invention can be employed in any of a blue, green and red sensitive layer and an intermediate layer without limitation, since they act as substantially non-color forming couplers.

In the photographic emulsion layer of the photographic light-sensitive material used in the present invention, a preferably employed silver halide is silver iodobromide, silver iodochloride or silver iodochlorobromide, each containing about 30 mol% or less of silver iodide. Silver iodobromide containing from about 2 mol% to about 25 mol% of silver iodide is particularly preferred.

Silver halide grains in the silver halide emulsion may have a regular crystal structure, for example, a cubic octahedral or tetradecahedral structure, an irregular crystal structure, for example, a spherical or tabular structure, a crystal defect, for example, a twin plane, or a composite structure thereof.

The particle size of silver halide utilized may be varied and includes particles from fine grains, having about 0.2 micron diameter or less, to large size grains, having about a 10 micron diameter of projected area. Further, polydispersed emulsion and monodispersed emulsion of the same may be used.

The silver halide photographic emulsion which can be used in the present invention can be prepared using known methods, for example, those as described in Research Disclosure, No. 17643 (December, 1978), pages 22 to 23, "I. Emulsion Preparation and Types" and ibid., No. 18716 (November, 1979), page 648, P. Glafkides, Chimie et Physique Photographique, Paul Montel (1967), G.F. Duffin, Photographic Emulsion Chemistry, The Focal Press (1966), and V.L. Zelikman et al., Making and Coating Photographic Emulsion, The Focal Press (1964).

Monodispersed emulsions as described, for example, in U.S. Patents 3,574,628 and 3,655,394, and British Patent 1,413,748 are preferably used in the present invention.

Further, tabular silver halide grains having an aspect ratio of about 5 or more can be employed in the present invention. The tabular grains may be easily prepared by the method as described, for example, in Gutoff, Photographic Science and Engineering, Vol. 14, pages 248 to 257 (1970), U.S. Patents 4,434,226, 4,414,310, 4,433,048 and 4,439,520, and British Patent 2,112,157.

The crystal structure of silver halide grains may be uniform, composed of different halide compositions between the inner portion and the outer portion, or may have a stratified structure.

Further, silver halide emulsions in which silver halide grains having different compositions are connected upon epitaxial junctions, or silver halide emulsions in which silver halide grains are connected with compounds other than silver halide, such as silver thiocyanate, or lead oxide may also be employed.

Moreover, a mixture of grains having a different crystal structure may be used.

The silver halide emulsion used in the present invention are usually conducted with physical ripening, chemical ripening and spectral sensitization. Various kinds of additives which can be employed in these steps are described in Research Disclosure, No. 17643, (December, 1978) and ibid., No. 18716 (November, 1979). Concerned items thereof are summarized in the table shown below.

Further, known photographic additives which can be used in the present invention are also described in the above mentioned literatures, and concerned items thereof are summarized in the table below.

| | Kind of Additives | RD 17643 | RD 18716 |
|---|---|---|---|
| 1. | Chemical Sensitizers | Page 23 | Page 648, right column |
| 2. | Sensitivity Increasing Agents | | - ditto - |
| 3. | Spectral Sensitizers and Supersensitizers | Pages 23 to 24 | Page 648, right column to page 649, right column |
| 4. | Whitening Agents | Page 24 | |
| 5. | Antifoggants and Stabilizers | Pages 24 to 25 | Page 649, right column |
| 6. | Light-Absorbers, Filter Dyes and Ultra-violet Ray Absorbers | Pages 25 to 26 | Page 649, right column to page 650, left column |
| 7. | Antistaining Agents | Page 25, right column | Page 650, left column to right column |
| 8. | Dye Image Stabilizers | Page 25 | |
| 9. | Hardeners | Page 26 | Page 651, left column |
| 10. | Binders | Page 26 | - ditto - |
| 11. | Plasticizers and Lubricants | Page 27 | Page 650, right column |
| 12. | Coating Aids and Surfactants | Pages 26 to 27 | - ditto - |
| 13. | Antistatic Agents | Page 27 | - ditto - |

In the present invention, various color couplers can be employed and specific examples thereof are described in the patents cited in Research Disclosure, No. 17643, "VII-C" to "VII-G".

As yellow couplers used in the present invention, for example, those as described in U.S. Patents 3,933,501, 4,022,620, 4,326,024 and 4,401,752, JP-B-58-10739, British Patents 1,425,020 and 1,476,760.

As magenta couplers used in the present invention, 5-pyrazolone type and pyrazoloazole type compounds are preferred. Magenta couplers as described in U.S. Patents 4,310,619 and 4,351,897, European Patent 73,636, U.S. Patents 3,061,431 and 3,725,067, Research Disclosure, No. 24220 (June, 1984), JP-A-60-33552, Research Disclosure, No. 24230 (June, 1984), JP-A-60-43659, and U.S. Patents 4,500,630 and 4,540,654 are particularly preferred.

As cyan couplers used in the present invention, phenol type and naphthol type couplers are exemplified. Cyan couplers as described in U.S. Patents 4,052,212, 4,146,396, 4,228,233, 4,296,200, 2,369,929, 2,801,171, 2,772,162, 2,895,826, 3,772,002, 3,758,308, 4,334,011 and 4,327,173, West German Patent Application (OLS) No. 3,329,729, European Patent 121,365A, U.S. Patents 3,446,622, 4,333,999, 4,451,559 and 4,427,767, and European Patent 161,626A are preferred.

As colored couplers for correcting undesirable absorptions of dyes formed, those as described in Research Disclosure, No. 17643, "VII-G"", U.S. Patent 4,163,670, JP-B-57-39413, U.S. Patents 4,004,929 and 4,138,258, and British Patent 1,146,368 are preferably employed.

As couplers capable of forming appropriately diffusible dyes, those as described in U.S. Patent 4,366,237, British Patent 2,125,570, European Patent 96,570, and West German Patent Application (OLS) No. 3,234,533 are preferably employed.

Typical examples of polymerized dye forming couplers are described in U.S. Patents 3,451,820, 4,080,211 and 4,367,282, and British Patent 2,102,173.

The couplers which can be used in the present invention can be introduced into the photographic light-sensitive material according to various known dispersing methods.

Suitable examples of organic solvents having a high boiling point which can be employed in an oil droplet-in-water type dispersing method are described, for example, in U.S. Patent 2,322,027.

Examples of steps of latex dispersion method, effects thereof and latexes for impregnation are disclosed, for example, in U.S. Patent 4,199,363, West German Patents (OLS) 2,541,274 and 2,541,230.

Suitable supports which can be used in the present invention are described, for example, in Research Disclosure, No. 17643, page 28 and ibid., No. 18716, page 647, right column to page 648, left column, as mentioned above.

A color developing solution which can be used in development processing of the color photographic light-sensitive material according to the present invention is preferably an alkaline aqueous solution containing an aromatic primary amine type color developing agent as a main component. As the color developing agent, while an aminophenol type compound is also useful, a p-phenylenediamine type compound is preferably employed. Typical examples of the p-phenylenediamine type compounds include 3-methyl-4-amino-N,N-diethylaniline, 3-methyl-4-amino-N-ethyl-N-β-hydroxyethylaniline, 3-methyl-4-amino-N-ethyl-N-β-methanesulfonamidoethylaniline, 3-methyl-4-amino-N-ethyl-N-β-methoxyethylaniline, or sulfate, hydrochloride or p-toluenesulfonate thereof.

Two or more kinds of color developing agents may be employed in a combination thereof, depending on the purpose.

The color developing solution can ordinarily contain pH buffering agents, such as carbonates, borates or phosphates of alkali metals; and development inhibitors or anti-fogging agents such as bromides, iodides, benzimidazoles, benzothiazoles, or mercapto compounds. Further, if desired, the color developing solution may contain various preservatives such as hydroxylamine, diethylhydroxylamine, sulfites, hydrazines, phenylsemicarbazides, triethanolamine, catechol sulfonic acids, or triethylenediamine(1,4-diazabicyclo[2,2,2]octane); organic solvents such as ethyleneglycol, or diethylene glycol; development accelerators such as benzyl alcohol, polyethylene glycol, quarternary ammonium salts, or amines; dye forming couplers; competing couplers; fogging agents such as sodium borohydride; auxiliary developing agents such as 1-phenyl-3-pyrazolidone; viscosity imparting agents; and various chelating agents represented by aminopolycarboxylic acids, aminopolyphosphonic acids, alkylphosphonic acids, or phosphonocarboxylic acids. Representative examples of the chelating agents include ethylenediaminetetraacetic acid, nitrilotriacetic acid, diethylenetriaminepentaacetic acid, cyclohexanediaminetetraacetic acid, hydroxyethyliminodiacetic acid, 1-hydroxyethylidene-1,1-diphosphonic acid, nitrilo-N,N,N-trimethylenephosphonic acid, ethylenediamine-N,N,N′,N′-tetramethylenephosphonic acid, ethylenediamine-di(o-hydroxyphenylacetic acid), and salts thereof.

In case of conducting reversal processing, color development is usually conducted after black-and-white development. In a black-and-white developing solution, known black-and-white developing agents, for example, dihydroxybenzenes such as hydroquinone, 3-pyrazolidones such as 1-phenyl-3 pyrazolidone, or aminophenols such as N-methyl-p-aminophenol may be employed individually or in a combination.

The pH of the color developing solution or the black-and-white developing solution is usually in a range from 9 to 12.

After color development, the photographic emulsion layers are usually subjected to a bleach processing. The bleach processing can be performed simultaneously with a fix processing (bleach-fix processing), or it can be performed independently from the fix processing. Further, for the purpose of a rapid processing, a processing method wherein after a bleach processing a bleach-fix processing is conducted may be employed.

As bleaching agents which can be employed, ferric complex salts of aminopolycarboxylic acid (such as ethylenediaminetetraacetic acid, diethylenetriaminepentaacetic acid, cyclohexanediaminetetraacetic acid, or 1,3-diaminopropanetetraacetic acid) are preferred, and ferric complex salt of 1,3-diaminopropanetetraacetic acid is particularly preferred in view of rapid processing.

The pH of the bleaching solution or bleach-fixing solution is preferably in a range from 3 to 7, particularly preferably from 4 to 6 in view of rapid processing.

After a desilvering step, the silver halide color photographic material according to the present invention is generally subjected to a water washing step and/or a stabilizing step.

An amount of water required for the water washing step may be set in a wide range depending on characteristics of photographic light-sensitive materials (due to elements used therein, for example, couplers, etc.),

uses thereof, temperature of washing water, a number of water washing tanks (stages), a replenishment system such as countercurrent or orderly current, or other various conditions. A relationship between a number of water washing tanks and an amount of water in a multi-stage countercurrent system can be determined based on the method as described in Journal of the Society of Motion Picture and Television Engineers, Vol. 64, pages 248 to 253 (May, 1955).

A pH of the washing water used in the processing of the photographic light-sensitive materials according to the present invention is usually from 4 to 9, preferably from 5 to 8. Temperature of washing water and time for a water washing step can be variously set depending on characteristics or uses the of photographic light-sensitive materials. However, one generally selects a range of from 15 to 45°C and a period from 20 sec. to 10 min., and preferably a range of from 25 to 40°C and a period from 30 sec. to 5 min.

The photographic light-sensitive material of the present invention can also be directly processed with a stabilizing solution in place of the above-described water washing step. In such a stabilizing process, any of known methods as described, for example, in JP-A-57-8543, JP-A-58-14834 and JP-A-60-220345 can be employed.

Further, it is possible to conduct the stabilizing process subsequent to the above-described water washing process. One example thereof is a stabilizing bath containing formalin and a surface active agent, which is employed as a final bath in the processing of color photographic light-sensitive materials for photography. To such a stabilizing bath, various chelating agents and antimolds may also be added.

In the present invention, various kinds of processing solutions can be employed in a temperature range from 10 to 50°C. Although a standard temperature is from 33 to 38°C, it is possible to carry out the processing at higher temperatures in order to accelerate the processing whereby the processing time is shortened, or at lower temperatures in order to achieve improvement in image quality and to maintain stability of the processing solutions.

In accordance with the present invention, silver halide color photographic material excellent in image sharpness without losing sensitivity. Further, these silver halide color photographic materials are suitable for pradical use since their sensitivity and desilvering property are not deteriorated.

The present invention will be explained in greater detail with reference to the following examples, but the present invention should not be construed as being limited thereto.

## EXAMPLE 1

### Preparation of Sample 101:

On a cellulose triacetate film support, provided with a subbing layer, were coated layers, having the composition set forth below, to prepare a multilayer color light-sensitive material which was designated Sample 101.

With respect to the compositions of the layers, coating amounts of silver halide and colloidal silver are shown by $g/m^2$ units in terms of silver, the coating amounts of couplers, additives and gelatin are shown by $g/m^2$ unites, and the coating amounts of sensitizing dyes are shown by mol per mol of silver halide present in the same layer.

First Layer: Antihalation Layer

| | |
|---|---|
| Black colloidal silver | 0.2 |
| Gelatin | 1.3 |
| ExM-9 | 0.06 |
| UV-1 | 0.03 |
| UV-2 | 0.06 |
| UV-3 | 0.06 |
| Solv-1 | 0.15 |
| Solv-2 | 0.15 |
| Solv-3 | 0.05 |

Second Layer: Intermediate Layer

| | |
|---|---|
| Gelatin | 1.0 |
| UV-1 | 0.03 |
| ExC-4 | 0.02 |

| | |
|---|---|
| ExF-1 | 0.004 |
| Solv-1 | 0.1 |
| Solv-2 | 0.1 |

Third Layer: Low-Speed Red-Sensitive Emulsion Layer

| | |
|---|---|
| Silver iodobromide emulsion (AgI: 4 mol%, uniform AgI type, diameter corresponding to sphere: 0.5 µm, coefficient of variation of diameter corresponding to sphere: 20%, tabular grain, diameter/thickness ratio: 3.0) | 1.2 g (as silver) |
| Silver iodobromide emulsion (AgI: 3 mol%, uniform AgI type, diameter corresponding to sphere: 0.3 µm, coefficient of variation of diameter corresponding to sphere: 15%, spherical grain, diameter/thickness ratio: 1.0) | 0.6 g (as silver) |
| Gelatin | 1.0 |
| ExS-1 | $4\times10^{-4}$ |
| ExS-2 | $5\times10^{-5}$ |
| ExC-1 | 0.05 |
| ExC-2 | 0.50 |
| ExC-3 | 0.03 |
| ExC-4 | 0.12 |
| ExC-5 | 0.01 |
| ExC-8 | 0.03 |

Fourth Layer: High-Speed Red-sensitive Emulsion Layer

| | |
|---|---|
| Silver iodobromide emulsion (AgI: 6 mol%, internal high AgI type, with core/shell AgI molar ratio of 1:1, diameter corresponding to sphere: 0.7 µm, coefficient of variation of diameter corresponding to sphere: 15%, tabular grain, diameter/thickness ratio: 5.0) | 0.7 g (as silver) |
| Gelatin | 1.0 |
| ExS-1 | $3\times10^{-4}$ |
| ExS-2 | $2.3\times10^{-5}$ |
| ExC-6 | 0.11 |
| ExC-7 | 0.05 |
| ExC-4 | 0.05 |
| Solv-1 | 0.05 |
| Solv-3 | 0.05 |

Fifth layer: Intermediate Layer

| | |
|---|---|
| Gelatin | 0.5 |
| Cpd-1 | 0.1 |
| Solv-1 | 0.05 |

Sixth Layer: Low-Speed Green-Sensitive Emulsion Layer

| | |
|---|---|
| Silver iodobromide emulsion (AgI: 4 mol%, surface high AgI type, with core/shell AgI molar ratio of 1:1, diameter corresponding to sphere: 0.5 µm, coefficient of variation of diameter corresponding to sphere: 15%, tabular grain, diameter/thickness ratio: 4.0) | 0.35 g (as silver) |

| | |
|---|---|
| Silver iodobromide emulsion (AgI: 3 mol%, uniform AgI type, diameter corresponding to sphere: 0.3 μm, coefficient of variation of diameter corresponding to sphere: 25%, spherical grain, diameter/thickness ratio: 1.0) | 0.20 (as silver) |
| Gelatin | 1.0 |
| ExS-3 | $5 \times 10^{-4}$ |
| ExS-4 | $3 \times 10^{-4}$ |
| ExS-5 | $1 \times 10^{-4}$ |
| ExM-8 | 0.4 |
| ExM-9 | 0.07 |
| ExM-10 | 0.02 |
| ExN-1 | 0.03 |
| Solv-1 | 0.3 |
| Solv-4 | 0.05 |

Seventh Layer: High-Speed Green-Sensitive Emulsion Layer

| | |
|---|---|
| Silver iodobromide emulsion (AgI: 4 mol%, internal high AgI type, with core/shell AgI molar ratio of 1:3, diameter corresponding to sphere: 0.7 μm, coefficient of variation of diameter corresponding to sphere: 20%, tabular grain, diameter/thickness ratio: 5.0) | 0.8 (as silver) |
| Gelatin | 0.5 |
| ExS-3 | $5 \times 10^{-4}$ |
| ExS-4 | $3 \times 10^{-4}$ |
| ExS-5 | $1 \times 10^{-4}$ |
| ExM-8 | 0.1 |
| ExM-9 | 0.02 |

| | |
|---|---|
| ExY-11 | 0.03 |
| ExC-2 | 0.03 |
| ExM-14 | 0.04 |
| Solv-1 | 0.2 |
| Solv-4 | 0.01 |

Eighth Layer: Intermediate Layer

| | |
|---|---|
| Gelatin | 0.5 |
| Cpd-1 | 0.05 |
| Solv-1 | 0.02 |

Ninth Layer: Donor Layer of Interimage Effect to Red-Sensitive Layer

| | |
|---|---|
| Silver iodobromide emulsion (AgI: 2 mol%, internal high AgI type, with core/shell AgI molar ratio of 2:1, diameter corresponding to sphere: 1.0 µm, coefficient of variation of diameter corresponding to sphere: 15%, tabular grain, diameter/thickness ratio: 6.0) | 0.35 (as silver) |
| Silver iodobromide emulsion (AgI: 2 mol%, internal high AgI type with core/shell AgI molar ratio of 1:1, diameter corresponding to sphere: 0.4 µm, coefficient of variation of diameter corresponding to sphere: 20%, tabular grain, diameter/thickness ratio: 6.0) | 0.20 (as silver) |
| Gelatin | 0.5 |
| ExS-3 | $8 \times 10^{-4}$ |
| ExY-13 | 0.11 |
| ExM-12 | 0.03 |
| ExM-14 | 0.10 |
| Solv-1 | 0.20 |

Tenth Layer: Yellow Filter Layer

| | |
|---|---|
| Yellow colloidal silver | 0.05 |
| Gelatin | 0.5 |
| Cpd-2 | 0.13 |
| Solv-1 | 0.13 |

| | |
|---|---|
| Cpd-1 | 0.10 |

Eleventh Layer: Low-Speed Blue-sensitive Emulsion Layer

| | |
|---|---|
| Silver iodobromide emulsion (AgI: 4.5 mol%, uniform AgI type, diameter corresponding to sphere: 0.7 µm, coefficient of variation of diameter corresponding to sphere: 15%, tabular grain, diameter/thickness ratio: 7.0) | 0.3 (as silver) |
| Silver iodobromide emulsion (AgI: 3 mol%, uniform AgI type, diameter corresponding to sphere: 0.3 µm, coefficient of variation of diameter corresponding to sphere: 25%, tabular grain, diameter/thickness ratio: 7.0) | 0.15 (as silver) |
| Gelatin | 1.6 |
| ExS-6 | $2\times10^{-4}$ |
| ExC-16 | 0.05 |
| ExC-2 | 0.10 |
| ExC-3 | 0.02 |
| ExY-13 | 0.07 |
| ExY-15 | 1.0 |
| Solv-1 | 0.20 |

Twelfth Layer: High-Speed Blue-sensitive Emulsion Layer

| | |
|---|---|
| Silver iodobromide emulsion (AgI: 10 mol%, internal high AgI type, diameter corresponding to sphere: 1.0 µm, coefficient of variation of diameter corresponding to sphere: 25%, multiple twin tabular grain, diameter/thickness ratio: 2.0) | 0.5 (as silver) |
| Gelatin | 0.5 |
| ExS-6 | $1\times10^{-4}$ |
| ExY-15 | 0.20 |
| ExY-13 | 0.01 |
| Solv-1 | 0.10 |

Thirteenth Layer: First Protective Layer

| | |
|---|---|
| Gelatin | 0.8 |
| UV-4 | 0.1 |
| UV-5 | 0.15 |
| Solv-1 | 0.01 |
| Solv-2 | 0.01 |

Fourteenth Layer: Second Protective Layer

| | |
|---|---|
| Fine grain silver iodobromide emulsion (AgI: 2 mol%, uniform AgI type, diameter corresponding to sphere: 0.07 μm) | 0.5 (as silver) |
| Gelatin | 0.45 |
| Polymethyl methacrylate particle (diameter: 1.5 μm) | 0.2 |
| H-1 | 0.4 |
| Cpd-5 | 0.5 |
| Cpd-6 | 0.5 |
| CpD-8 | 0.2 |

Each layer described above further contained a stabilizer for emulsion (Cpd-3: 0.04 g/m$^2$) and a surface active agent (Cpd-4: 0.02 g/m$^2$) as a coating aid in addition to the above-described compounds.

The compounds used for the preparation of the light-sensitive material are illustrated below.

UV-1

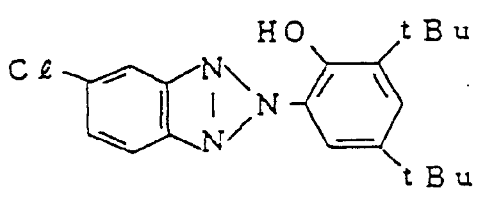

UV-2

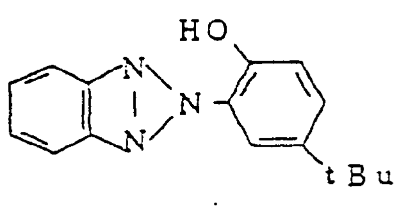

UV-3

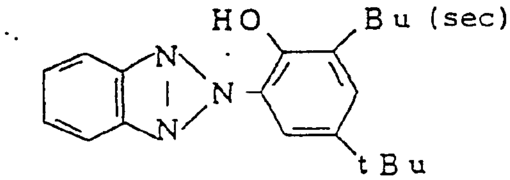

UV-4

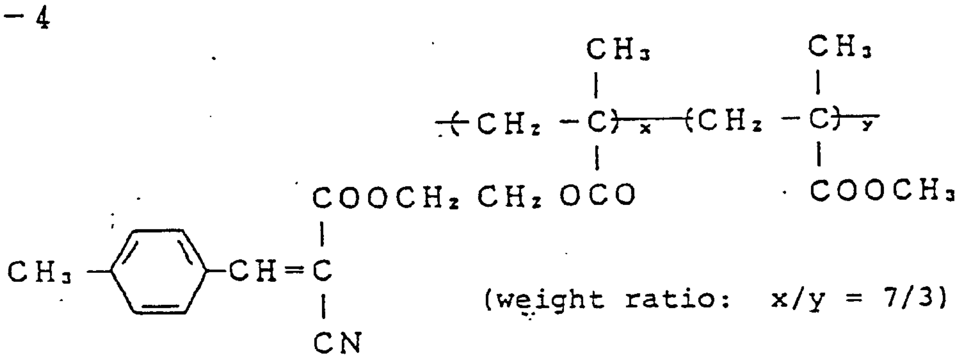

(weight ratio: x/y = 7/3)

UV-5

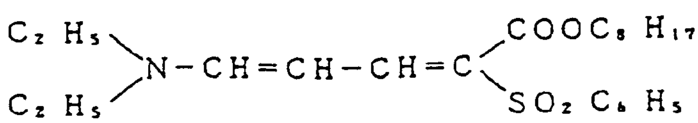

Solvent-1 Tricresyl phosphate

Solvent-2 Dibutyl phthalate

Solv-3

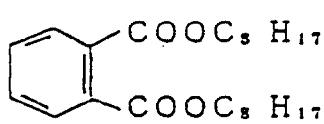

Solv-4

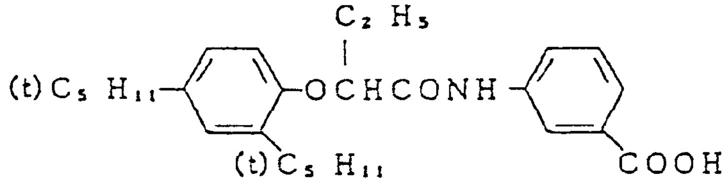

Cpd-1

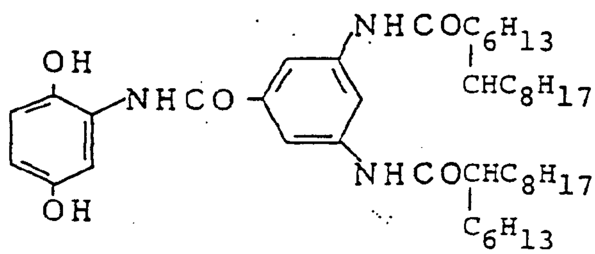

Cpd-2

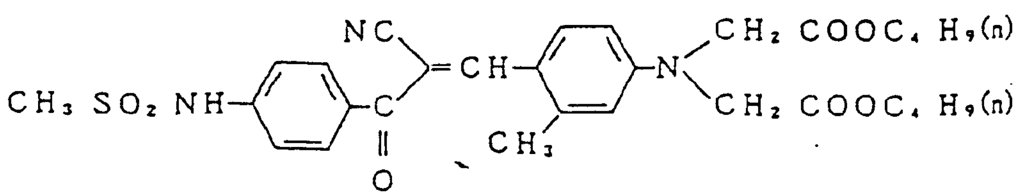

Cpd-3

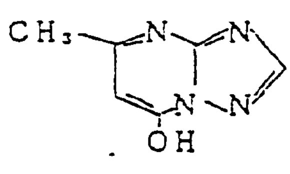

Cpd-4

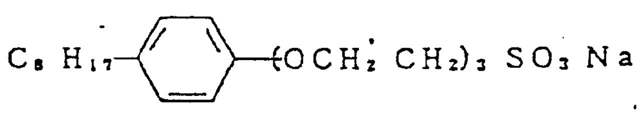

Cpd-5

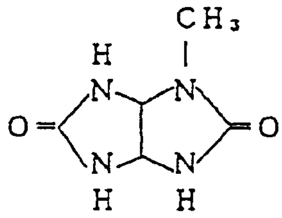

Cpd-6

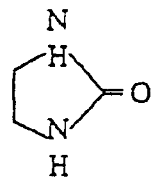

Cpd-8

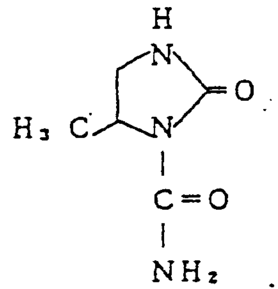

ExC-1

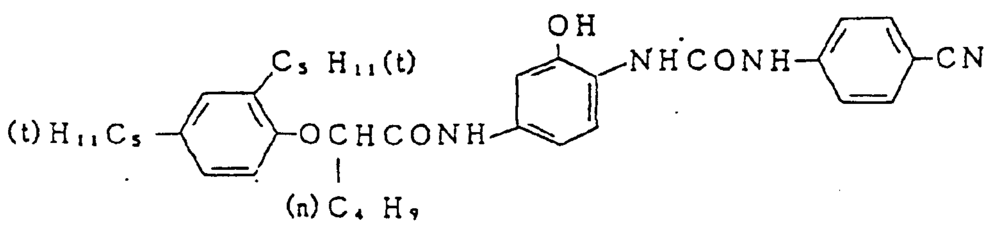

ExC-2

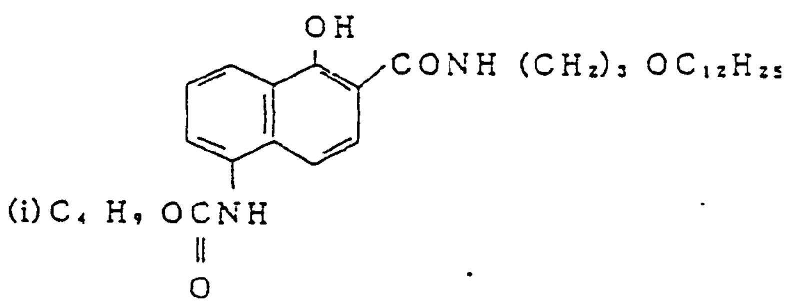

ExC-3

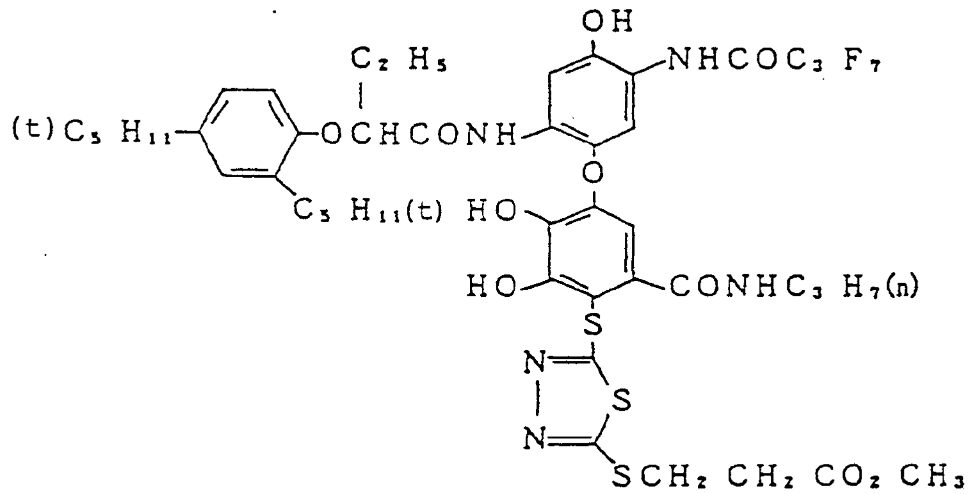

ExC-4

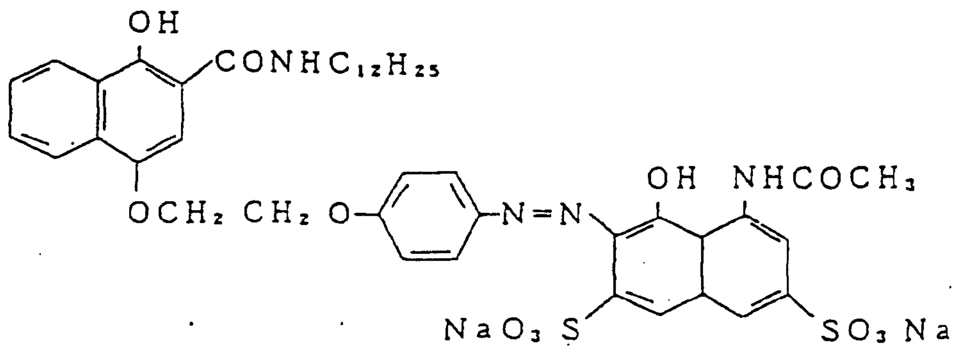

ExC-8

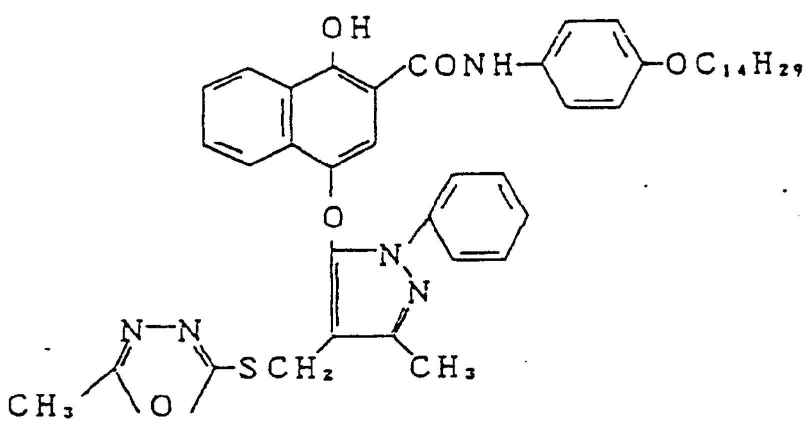

ExC-5

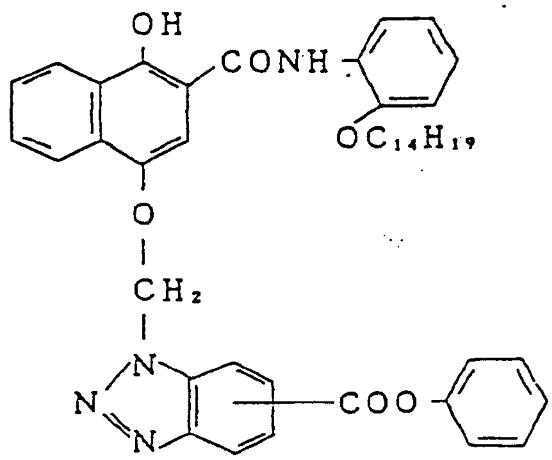

ExC-6

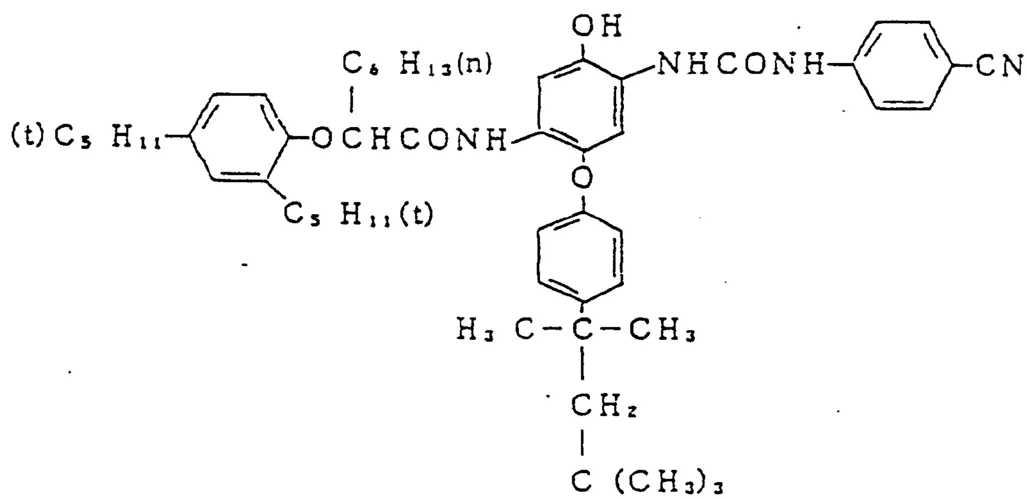

ExC-7

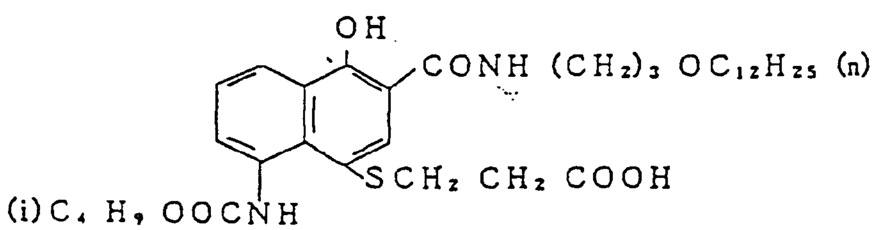

ExM-8

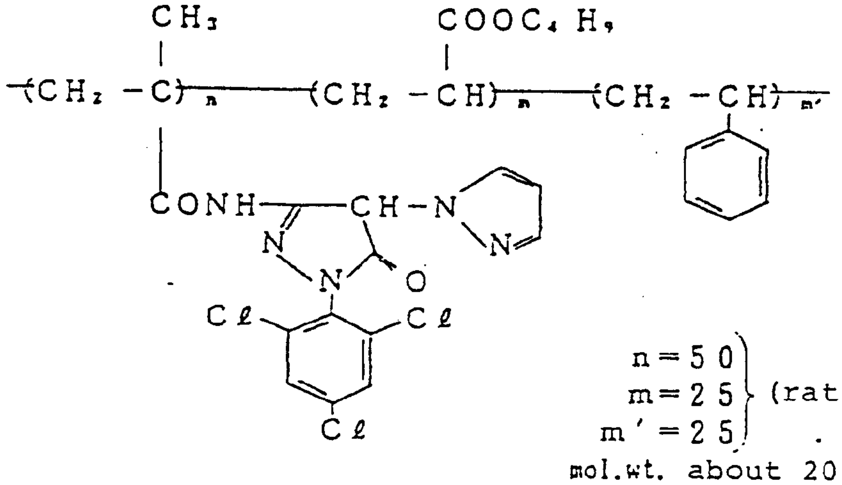

n = 50
m = 25 (ratio by weight)
m' = 25

mol.wt. about 20,000

ExM-9

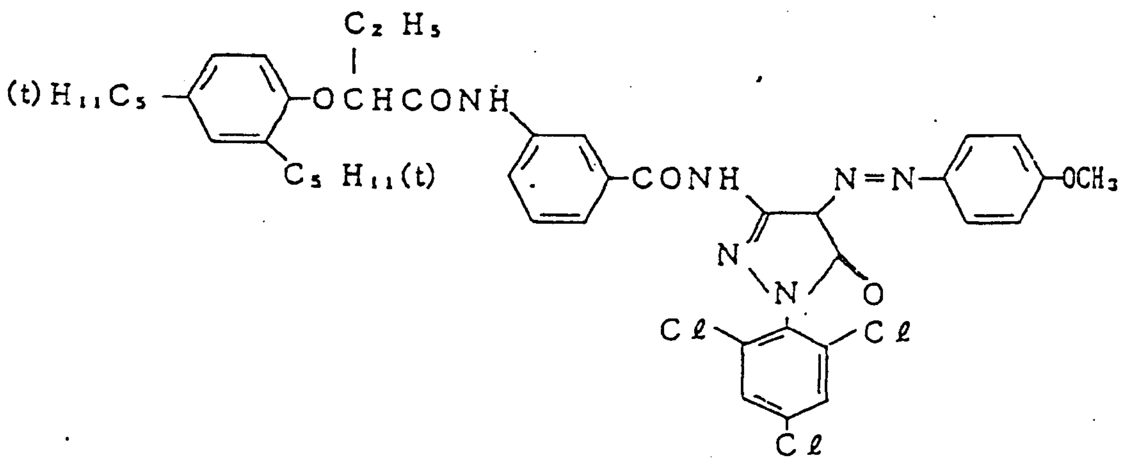

ExM-10

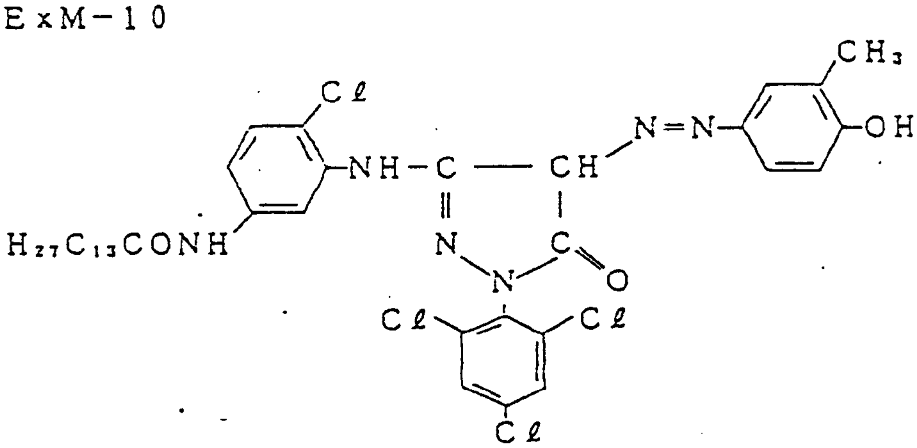

ExY-11

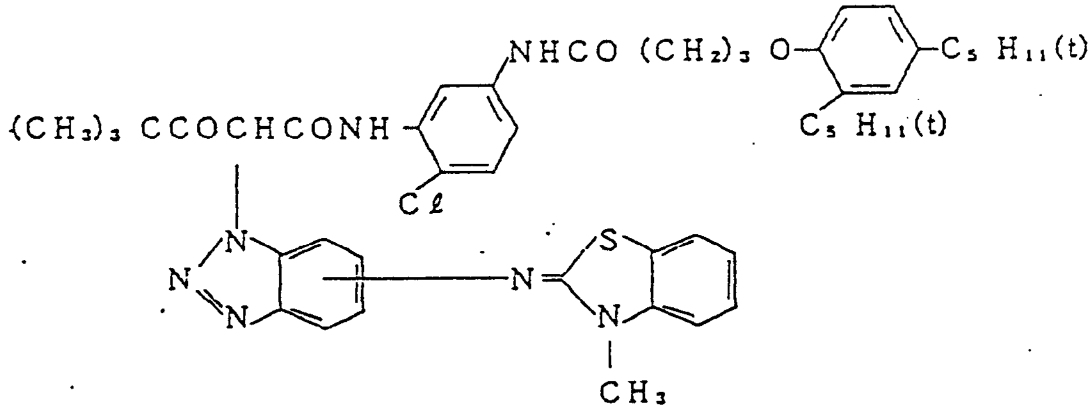

ExN-1

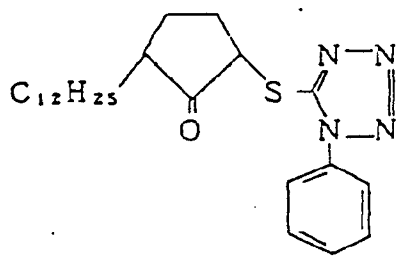

(Coupler disclosed in U.S. Patent 3,958,993)

ExM-12

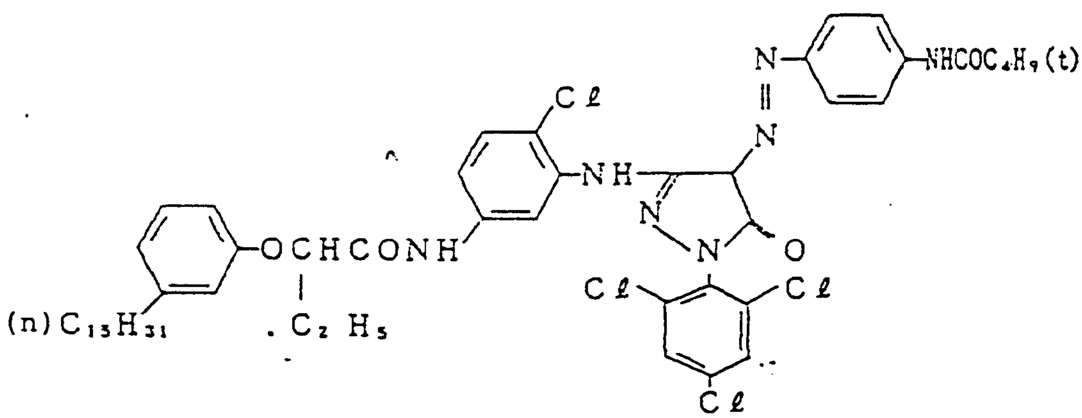

ExY-13

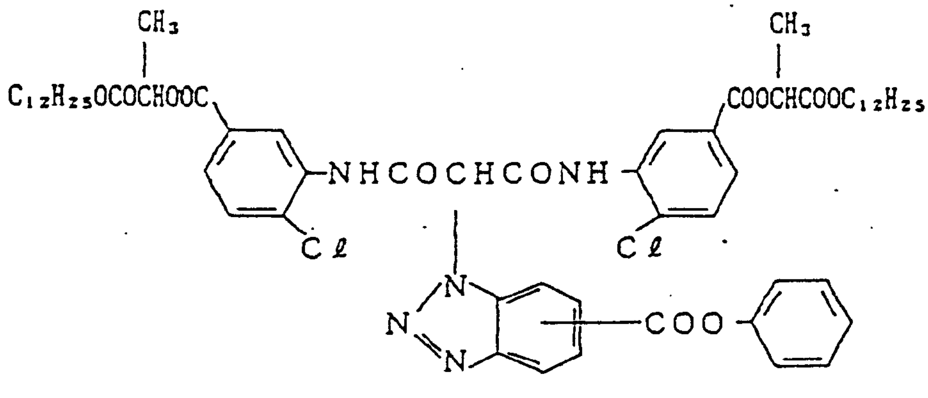

ExM-14

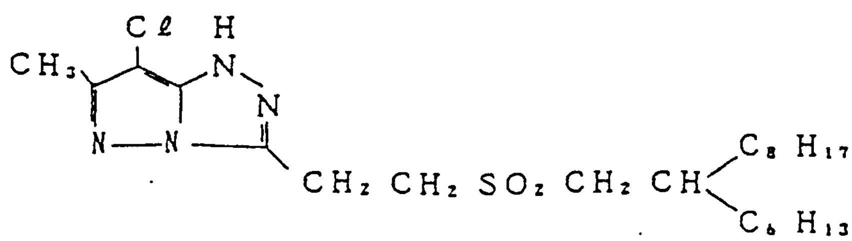

ExY-15

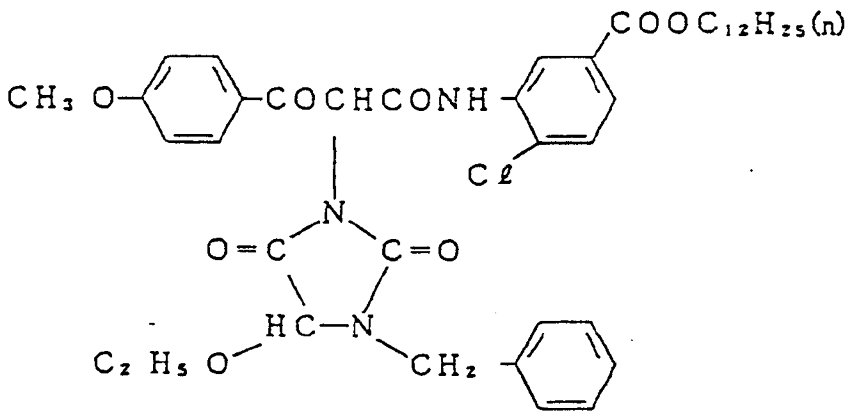

ExC-16

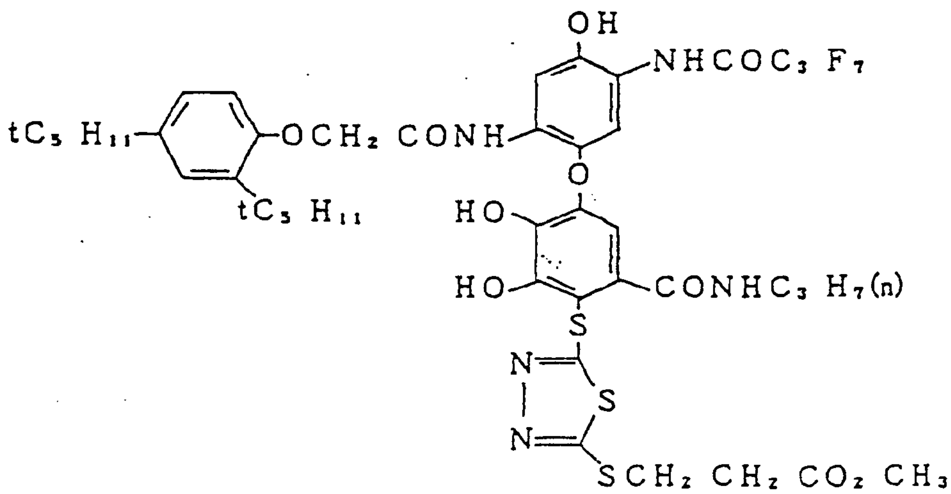

ExS-1

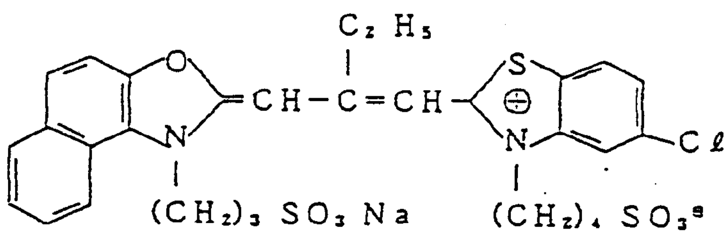

ExS-2

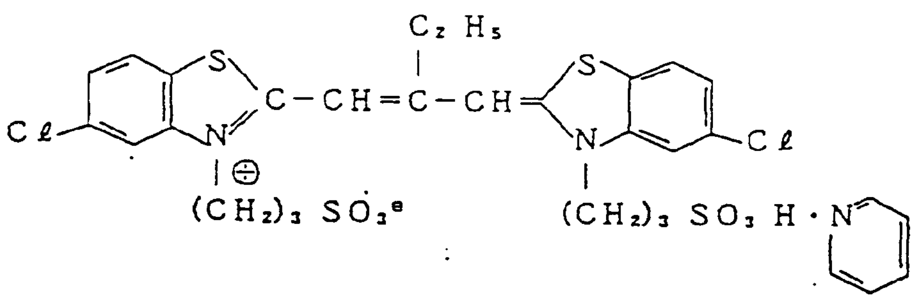

ExS-3

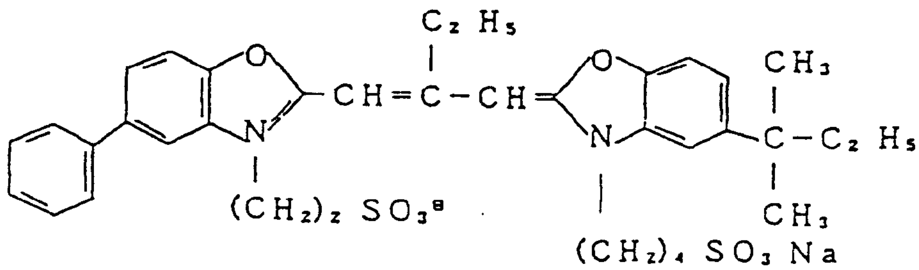

ExS-4

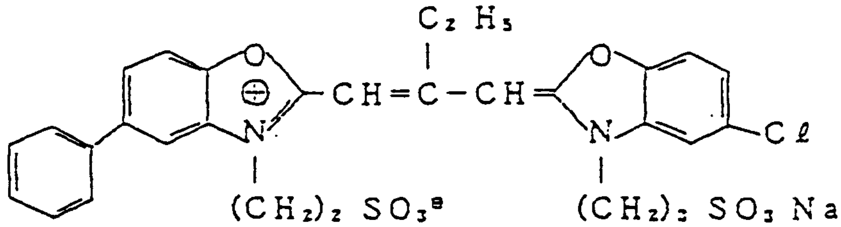

ExS-5

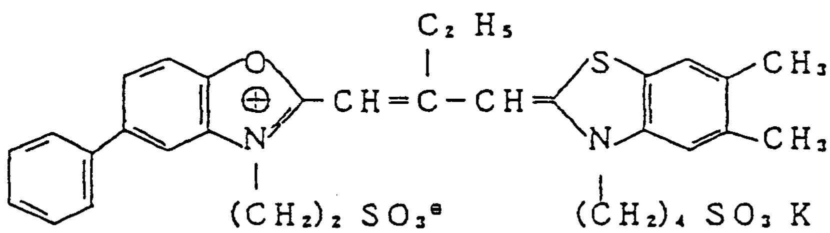

ExS-6

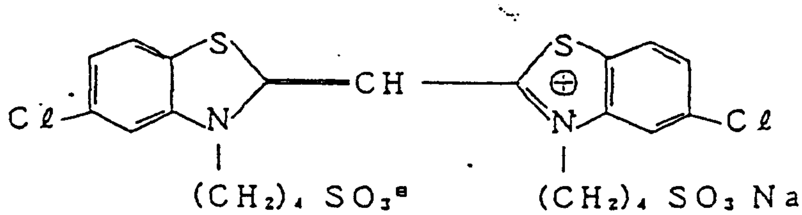

H-1

$$CH_2=CH-SO_2-CH_2-CONH-CH_2$$
$$CH_2=CH-SO_2-CH_2-CONH-CH_2$$

ExF-1

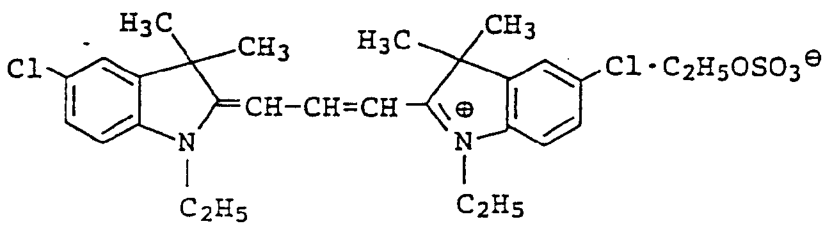

Preparation of Samples 102 to 113:

Samples 102 to 113 were prepared in the same manner as described for Sample 101 except for using the compounds shown in Table 1 below in place of Coupler ExN-1 used in the low-speed green-sensitive emulsion layer (sixth layer) of Sample 101, respectively. The couplers shown below were employed for comparison.

Ex-B

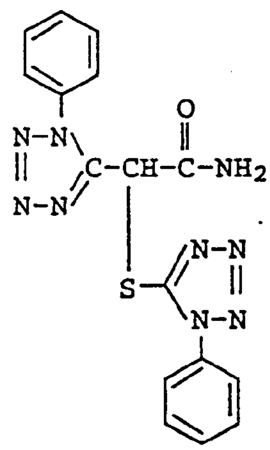

(Coupler disclosed in U.S. Patent 3,642,345)

Ex-C

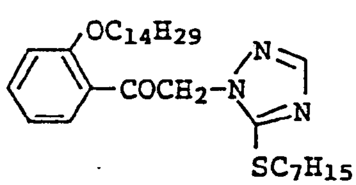

(Coupler disclosed in U.S. Patent 4,259,437)

Ex-D

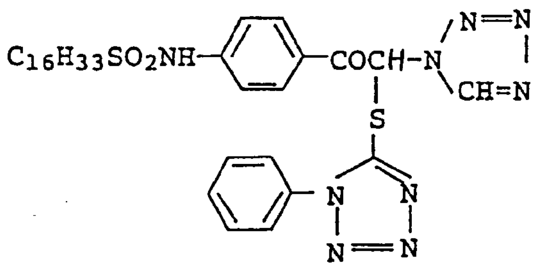

(Coupler disclosed in U.S. Patent 4,226,934)

Ex-E

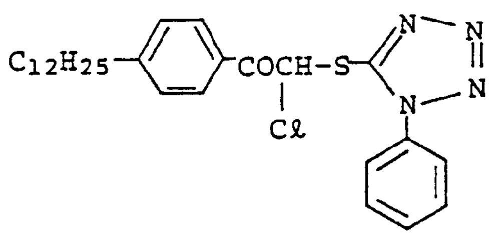

(Coupler disclosed in U.S. Patent 3,928,041)

Ex-F

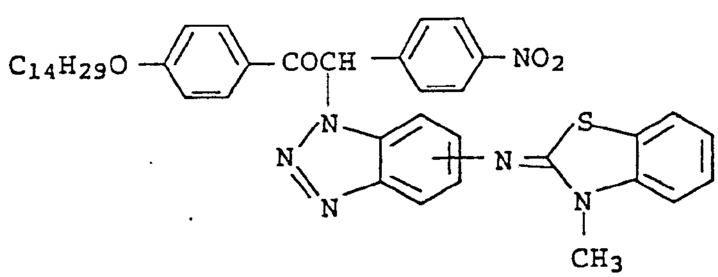

(Coupler disclosed in U.S. Patent 4,187,110)

Ex-G

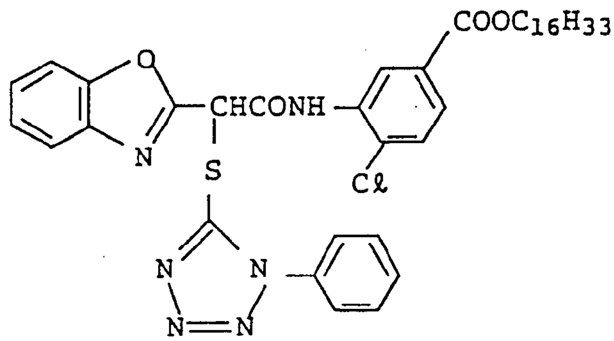

(Coupler disclosed in JP-B-52-82423)

Samples 101 to 113 thus-prepared were exposed wedgewise to white light and then subjected to color development processing according to the processing steps shown below. As the results show, the almost the same sensitivity and gradation were obtained with each of these samples.

| Processing Step | Processing Time | Processing Temperature (°C) |
|---|---|---|
| Color Development | 3 min. 15 sec. | 38 |
| Bleaching | 1 min. 00 sec. | 38 |
| Bleach-Fixing | 3 min. 15 sec. | 38 |
| Washing with Water (1) | 40 sec. | 35 |
| Washing with Water (2) | 1 min. 00 sec. | 35 |
| Stabilizing | 40 sec. | 38 |
| Drying | 1 min. 15 sec. | 55 |

The composition of each processing solution used is illustrated below.

Color Developing Solution:

| | |
|---|---|
| Diethylenetriaminepentaacetic acid | 1.0 g |
| 1-Hydroxyethylidene-1,1-diphosphonic acid | 3.0 g |
| Sodium sulfite | 4.0 g |
| Potassium carbonate | 30.0 g |
| Potassium bromide | 1.4 g |
| Potassium iodide | 1.5 mg |
| Hydroxylamine sulfate | 2.4 g |
| 4-(N-Ethyl-N-β-hydroxyethylamino)-2-methyleniline sulfate | 4.5 g |
| Water to make | 1.0 ℓ |
| pH | 10.05 |

Bleaching Solution:

| | |
|---|---|
| Ammonium iron(III) ethylenediaminetetraacetate dihydrate | 120.0 g |
| Disodium ethylenediaminetetraacetate | 10.0 g |
| Ammonium bromide | 100.0 g |
| Ammonium nitrate | 10.0 g |
| Bleach accelerating agent | 0.005 mol |
| 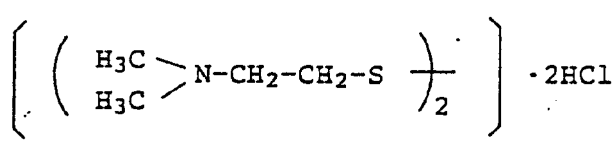 | |
| Aqueous ammonia (27%) | 15.0 ml |
| Water to make | 1.0 ℓ |
| pH | 6.3 |

Bleach-Fixing Solution:

| | |
|---|---|
| Ammonium iron(III) ethylenediamine-tetraacetate dihydrate | 50.0 g |
| Disodium ethylenediaminetetraacetate | 5.0 g |
| Sodium sulfite | 12.0 g |
| Aqueous solution of ammonium thiosulfate (70%) | 240.0 ml |
| Aqueous ammonia (27%) | 6.0 ml |
| Water to make | 1.0 ℓ |
| pH | 7.2 |

Washing Water:

City water was passed through a mixed bed type column filled with an H type strong acidic cation exchange resin (Amberlite IR-120B manufactured by Rhom & Haas Co.) and an OH type anion exchange resin (Amberlite IR-400 manufactured by Rhom & Haas Co.) to prepare water containing not more than 3 mg/ℓ of calcium ion and magnesium ion. To the water thus-treated were added sodium dichloroisocyanulate in an amount of 20 mg/ℓ and sodium sulfate in an amount of 150 mg/ℓ. The pH of the solution was in a range from 6.5 to 7.5.

stabilizing Solution:

| | |
|---|---|
| Formalin (37%) | 2.0 ml |
| Polyoxyethylene-p-monononylphenylether (average degree of polymerization: 10) | 0.3 g |
| Disodium ethylenediaminetetraacetate | 0.05 g |
| Water to make | 1.0 ℓ |
| pH | 5.0 to 8.0 |

Sharpness of the green-sensitive layer of each sample thus-processed was determined using a conventional MTF value at 25 lines/mm. The results obtained are shown in Table 1 below.

TABLE 1

| Sample | DIR Compound* in Low-Speed Green-Sensitive Layer | Amount** Added | MTF Value |
|---|---|---|---|
| 101 (Comparison) | ExN-1 | 1.0 | 0.57 |
| 102 ( " ) | Ex-B | 1.0 | 0.55 |
| 103 ( " ) | Ex-C | 1.5 | 0.56 |
| 104 ( " ) | Ex-D | 1.5 | 0.56 |
| 105 (Present Invention) | (1) | 1.0 | 0.66 |
| 106 ( " ) | (4) | 1.0 | 0.65 |
| 107 ( " ) | (5) | 1.0 | 0.67 |
| 108 ( " ) | (8) | 1.0 | 0.66 |
| 109 ( " ) | (14) | 1.0 | 0.67 |
| 110 ( " ) | (19) | 2.0 | 0.65 |
| 111 (Comparison) | Ex-E | 1.0 | 0.56 |
| 112 ( " ) | Ex-F | 1.0 | 0.57 |
| 113 ( " ) | Ex-G | 1.0 | 0.57 |

* The compound used in place of ExN-1 added to the low-speed green-sensitive emulsion layer

** A molar ratio taking the molar amount of ExN-1 added as 1

From the results shown in Table 1, it is apparent that the use of the compound according to the present invention greatly improves sharpness as compared with the use of conventional compounds.

Further, the color photographic eight-sensitive materials described above were exposed to light in the same manner as described above and then subjected to color development processing according to the processing steps shown below. Similar results to those described in Table 1 above were obtained. The samples according to the present invention exhibiting good desilvering property.

| Processing Step | Processing Time | Processing Temperature (°C) |
|---|---|---|
| Color Development | 2 min. 30 sec. | 40 |
| Bleach-fixing | 3 min. 00 sec. | 40 |
| Washing with Water (1) | 20 sec. | 35 |
| Washing with Water (2) | 20 sec. | 35 |
| Stabilizing | 20 sec. | 35 |
| Drying | 50 sec. | 65 |

The composition of each processing solution used is illustrated below.

Color Developing Solution:

| | |
|---|---|
| Diethylenetriaminepentaacetic acid | 2.0 g |
| 1-Hydroxyethylidene-1,1-diphosphonic acid | 3.0 g |
| Sodium sulfite | 4.0 g |
| Potassium carbonate | 30.0 g |
| Potassium bromide | 1.4 g |
| Potassium iodide | 1.5 mg |
| Hydroxylamine sulfate | 2.4 g |
| 4-(N-Ethyl-N-β-hydroxyethylamino)-2-methylaniline sulfate | 4.5 g |
| Water to make | 1.0 ℓ |
| pH | 10.05 |

Bleach-Fixing Solution:

| | |
|---|---|
| Ammonium iron(III) ethylenediamine-tetraacetate dihydrate | 50.0 g |
| Disodium ethylenediaminetetraacetate | 5.0 g |
| Sodium sulfite | 12.0 g |
| Ammonium thiosulfate (70% aqueous solution) | 260.0 ml |
| Acetic acid (98%) | 5.0 ml |
| Bleach accelerating agent 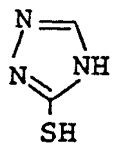 | 0.01 mol |
| Water to make | 1.0 ℓ |
| pH | 6.0 |

Washing Water:

City water was passed through a mixed bed type column filled with an H type strong acidic cation exchange resin (Amberlite IR-120B manufactured by Rhom & Haas Co.) and an OH type anion exchange resin (Amberlite IR-400 manufactured by Rhom & Haas Co.) to prepare water containing not more than 3 mg/ℓ of calcium ion and magnesium ion. To the water thus-treated were added sodium dichloroisocyanulate in an amount of 20 mg/ℓ and sodium sulfate in an amount of 1.5 g/ℓ. The pH of the solution was in a range from 6.5 to 7.5.

Stabilizing Solution:

| | |
|---|---|
| Formalin (37%) | 2.0 ml |
| Polyoxyethylene-p-monononylphenylether (average degree of polymerization: 10) | 0.3 g |
| Disodium ethylenediaminetetraacetate | 0.05 g |
| Water to make | 1.0 ℓ |
| pH | 5.0 to 8.0 |

EXAMPLE 2

Preparation of Sample 114:

On a cellulose triacetate film support, provided with a subbing layer, were coated layer, having the composition set forth below, to prepare a multilayer color photographic light-sensitive material which was designated Sample 114.

Regarding the compositions of the layers, coating amounts of silver halide and colloidal silver are shown by g/m2 units in terms of silver, those of couplers, additives and gelatin are shown by g/m2 unites, and those of sensitizing dyes are shown by molar amount per mol of silver halide present in the same layer.

First Layer: Antihalation Layer

| | |
|---|---|
| Black colloidal silver | 0.2 (as silver) |
| Gelatin | 2.2 |
| UV-10 | 0.1 |
| UV-20 | 0.2 |
| CpD-10 | 0.05 |
| Solv-10 | 0.01 |
| Solv-20 | 0.01 |
| Solv-30 | 0.08 |

Second Layer: Intermediate Layer

| | |
|---|---|
| Fine grain silver bromide (diameter of equivalent sphere: 0.07 µm) | 0.15 (as silver) |
| Gelatin | 1.0 |
| Cpd-20 | 0.2 |

Third Layer: First Red-Sensitive Emulsion layer

| | |
|---|---|
| Silver iodobromide emulsion (silver iodide: 10.0 mol%; internal high silver iodide type; diameter of equivalent sphere: 0.7 µm; coefficient of variation of diameter of equivalent sphere: 14%; tetradecahedral grain) | 0.26 (as silver) |
| Silver iodobromide emulsion (silver iodide: 4.0 mol%; internal high silver iodide type; diameter of equivalent sphere: 0.4 µm; coefficient of variation of diameter of equivalent sphere: 22%; tetradecahedral grain) | 0.2 (as silver) |

| | |
|---|---|
| Gelatin | 1.0 |
| ExS-10 | $4.5\times10^{-4}$ |
| ExS-20 | $1.5\times10^{-4}$ |
| ExS-30 | $0.4\times10^{-4}$ |
| ExS-40 | $0.3\times10^{-4}$ |
| ExC-10 | 0.33 |
| ExC-20 | 0.009 |
| ExC-30 | 0.023 |
| ExC-60 | 0.14 |

Fourth Layer: Second Red-Sensitive Emulsion Layer

| | |
|---|---|
| Silver iodobromide emulsion (silver iodide: 6 mol%; internal high silver iodide type; diameter of equivalent sphere: 1.0 µm; coefficient of variation of diameter of equivalent sphere: 25%; tabular grain; diameter/thickness ratio: 4.0) | 0.55 (as silver) |
| Gelatin | 0.7 |
| ExS-10 | $3\times10^{-4}$ |
| ExS-20 | $1\times10^{-4}$ |
| ExS-30 | $0.3\times10^{-4}$ |
| ExS-40 | $0.3\times10^{-4}$ |
| ExC-30 | 0.05 |
| ExC-40 | 0.10 |
| ExC-60 | 0.08 |

Fifth Layer: Third Red-Sensitive Emulsion Layer

| | |
|---|---|
| Silver iodobromide emulsion (silver iodide: 10.0 mol%; internal high silver iodide type; diameter of equivalent sphere: 1.2 µm; coefficient of variation of diameter of equivalent sphere: 28%; tabular grain; diameter/thickness ratio: 6.0) | 0.9 (as silver) |
| Gelatin | 0.6 |
| ExS-10 | $2\times10^{-4}$ |
| ExS-20 | $0.6\times10^{-4}$ |
| ExS-30 | $0.2\times10^{-4}$ |
| ExC-40 | 0.07 |
| ExC-50 | 0.06 |
| Solv-10 | 0.12 |
| Solv-20 | 0.12 |

Sixth Layer: Intermediate Layer

| | |
|---|---|
| Gelatin | 1.0 |
| Cpd-40 | 0.1 |

Seventh Layer: First Green-Sensitive Emulsion Layer

| | |
|---|---|
| Silver iodobromide emulsion (silver iodide: 10.0 mol%; internal high silver iodide type; diameter of equivalent sphere: 0.7 µm; coefficient of variation of diameter of equivalent sphere: 14%; tetradecahedral grain) | 0.2 (as silver) |

| | |
|---|---|
| Silver iodobromide emulsion (silver iodide: 4.0 mol%; internal high silver iodide type; diameter of equivalent sphere: 0.4 μm; coefficient of variation of diameter of equivalent sphere: 22%; tetradecahedral grain) | 0.1 (as silver) |
| gelatin | 1.2 |
| ExS-50 | $5\times10^{-4}$ |
| ExS-60 | $2\times10^{-4}$ |
| ExS-70 | $1\times10^{-4}$ |
| ExM-10 | 0.41 |
| ExN-1 (The same as used in Example 1) | 0.10 |
| ExM-50 | 0.03 |
| Solv-10 | 0.2 |
| Solv-50 | 0.03 |

Eighth Layer: Second Green-Sensitive Emulsion Layer

| | |
|---|---|
| Silver iodobromide emulsion (silver iodide: 10.0 mol%; internal high silver iodide type; diameter of equivalent sphere: 1.0 μm; coefficient of variation of diameter of equivalent sphere: 25%; tabular grain; diameter/thickness ratio: 3.0) | 0.4 (as silver) |
| Gelatin | 0.35 |
| ExS-50 | $3.5\times10^{-4}$ |
| ExS-60 | $1.4\times10^{-4}$ |
| ExS-70 | $0.7\times10^{-4}$ |

| | |
|---|---|
| ExM-10 | 0.09 |
| ExM-30 | 0.01 |
| Solv-10 | 0.15 |
| Solv-50 | 0.03 |

Ninth Layer: Intermediate Layer

| | |
|---|---|
| Gelatin | 0.5 |

Tenth Layer: Third Green-Sensitive Emulsion Layer

| | |
|---|---|
| Silver iodobromide emulsion (silver iodide: 10.0 mol%; internal high silver iodide type; diameter of equivalent sphere: 1.2 µm; coefficient of variation of diameter of equivalent sphere: 28%; tabular grain; diameter/thickness ratio: 6.0) | 1.0 (as silver) |
| Gelatin | 0.8 |
| ExS-50 | $2\times10^{-4}$ |
| ExS-60 | $0.8\times10^{-4}$ |
| ExS-70 | $0.8\times10^{-4}$ |
| ExM-30 | 0.01 |
| ExM-40 | 0.04 |
| ExC-40 | 0.005 |
| Solv-10 | 0.2 |

Eleventh Layer: Yellow Filter Layer

| | |
|---|---|
| Cpd-30 | 0.05 |
| Gelatin | 0.5 |
| Solv-10 | 0.1 |

Twelfth Layer: Intermediate Layer

| | |
|---|---|
| Gelatin | 0.5 |
| Cpd-20 | 0.1 |

Thirteenth Layer: First Blue-Sensitive Emulsion Layer

| | |
|---|---|
| Silver iodobromide emulsion (silver iodide: 10.0 mol%; internal high silver iodide type; diameter of equivalent sphere: 0.7 μm; coefficient of variation of diameter of equivalent sphere: 14%; tetradecahedral grain) | 0.1 (as silver) |
| Silver iodobromide emulsion (silver iodide: 4.0 mol%; internal high silver iodide type; diameter of equivalent sphere: 0.4 μm; coefficient of variation of diameter of equivalent sphere: 22%; tetradecahedral grain) | 0.05 (as silver) |
| gelatin | 1.0 |
| ExS-80 | $3\times10^{-4}$ |
| ExY-10 | 0.53 |
| ExY-20 | 0.02 |
| Solv-10 | 0.15 |

Fourteenth Layer: Second Blue-Sensitive Emulsion Layer

| | |
|---|---|
| Silver iodobromide emulsion (silver iodide: 19.0 mol%; internal high silver iodide type; diameter of equivalent sphere: 1.0 μm; coefficient of variation of diameter of equivalent sphere: 16%; tetradecahedral grain) | 0.19 (as silver) |
| gelatin | 0.3 |
| ExS-80 | $2\times10^{-4}$ |
| ExY-10 | 0.22 |
| Solv-10 | 0.07 |

Fifteenth Layer: Intermediate Layer

| | |
|---|---|
| Fine grain silver iodobromide (silver iodide: 2 mol%; uniform silver iodide type; diameter of equivalent sphere: 0.13 μm) | 0.2 (as silver) |
| gelatin | 0.36 |

Sixteenth Layer: Third Blue-Sensitive Emulsion Layer

| | |
|---|---|
| Silver iodobromide emulsion (silver iodide: 14.0 mol%; internal high silver iodide type; diameter of equivalent sphere: 1.5 µm; coefficient of variation of diameter of equivalent sphere: 28%; tabular grain; diameter/thickness ratio: 5.0) | 1.0 (as silver) |
| Gelatin | 0.5 |
| ExS-80 | $1.5\times10^{-4}$ |
| ExY-10 | 0.2 |
| Solv-10 | 0.07 |

Seventeenth Layer: First Protective Layer

| | |
|---|---|
| Gelatin | 1.8 |
| UV-10 | 0.1 |
| UV-20 | 0.2 |
| Solv-10 | 0.01 |
| Solv-20 | 0.01 |

Eighteenth Layer: Second Protective Layer

| | |
|---|---|
| Fine grain silver bromide (diameter of equivalent sphere: 0.07 µm) | 0.18 (as silver) |
| Gelatin | 0.7 |
| Polymethyl methacrylate particle (diameter: 1.5 µm) | 0.2 |
| W-10 | 0.02 |
| H-10 | 0.4 |
| Cpd-50 | 1.0 |

The chemical structure formulae of the compounds employed above are shown below.

ExC-10

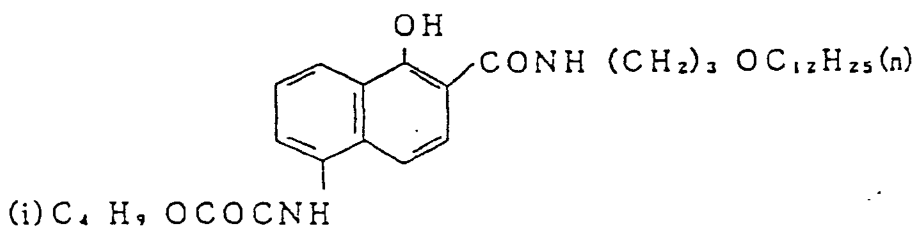

ExC-20

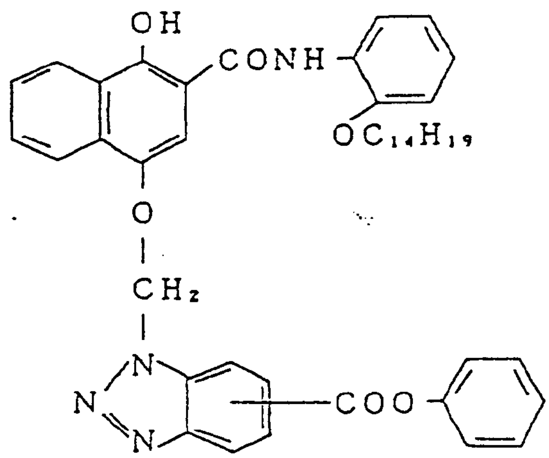

ExC-30

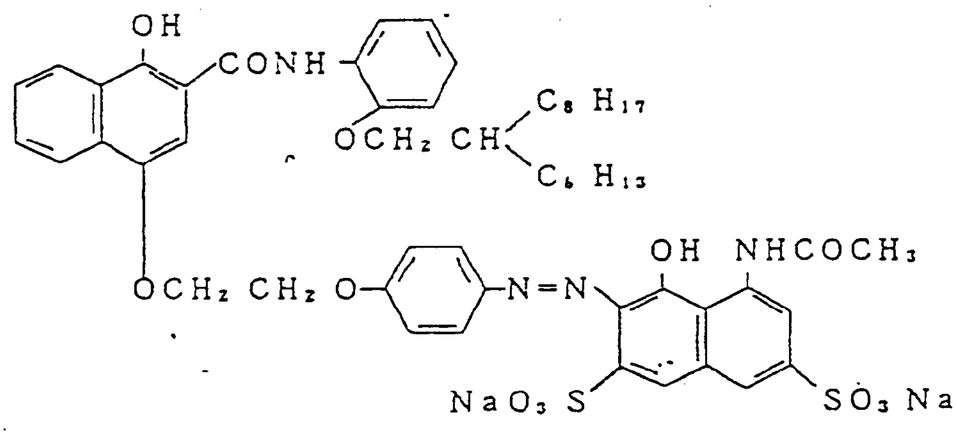

ExC-60

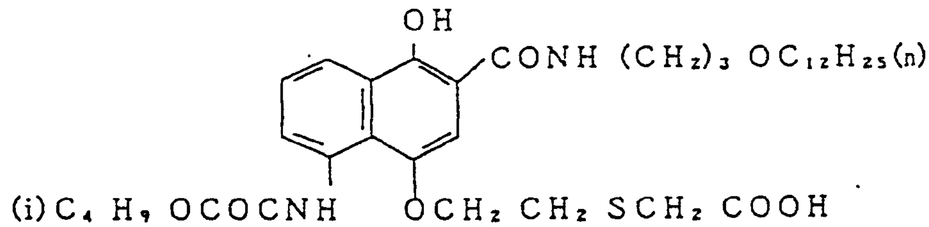

ExC-40

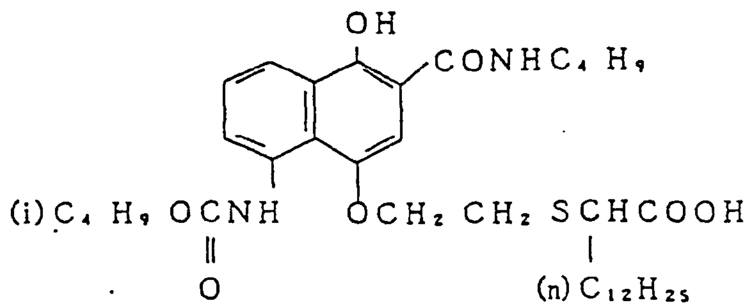

ExC-50

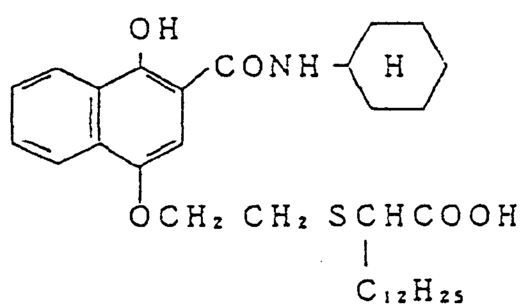

ExM-10

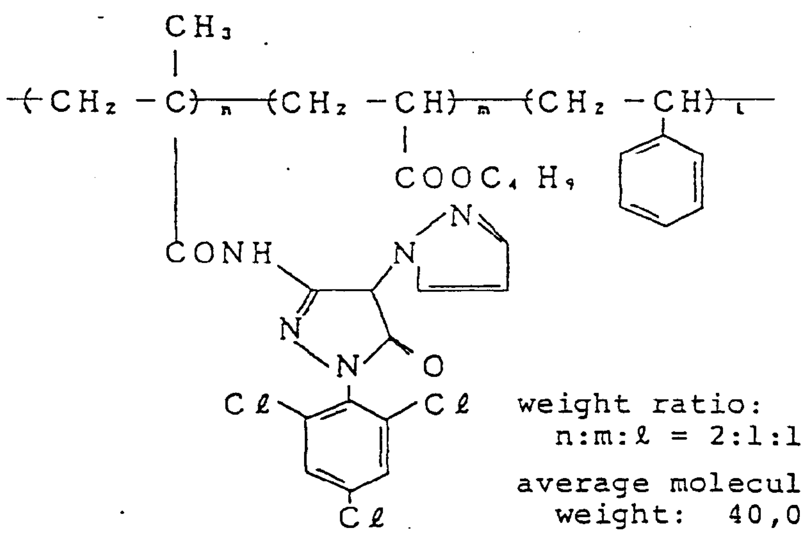

weight ratio:
n:m:ℓ = 2:1:1

average molecular weight: 40,000

ExM-40

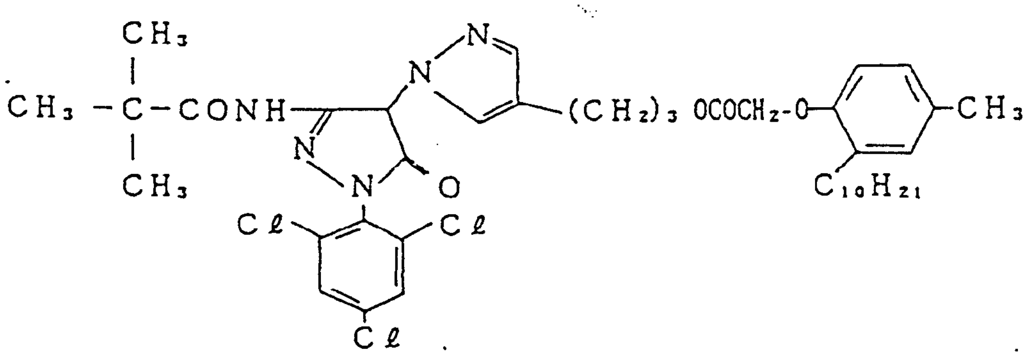

ExM-50

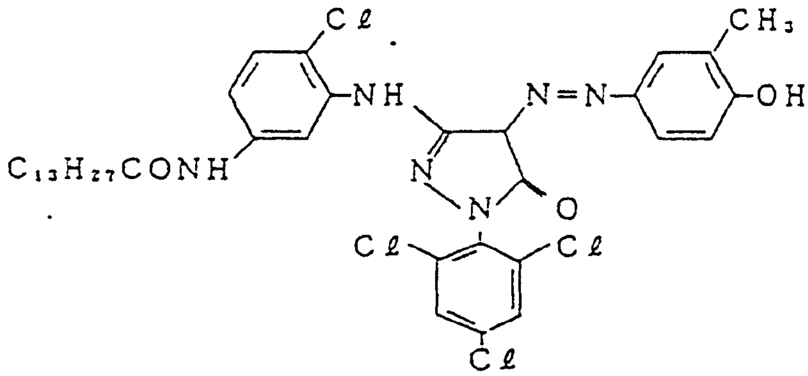

ExY-10

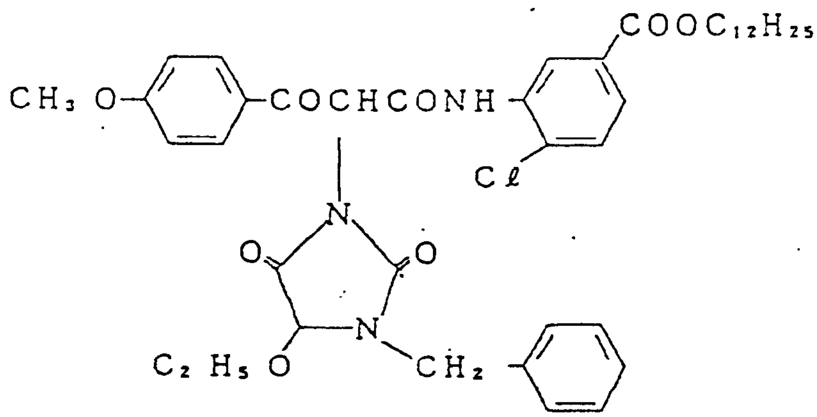

ExY-20

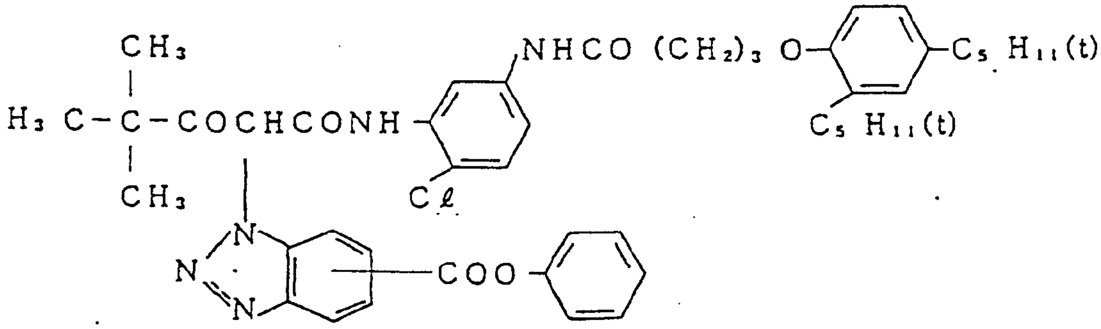

ExS-10

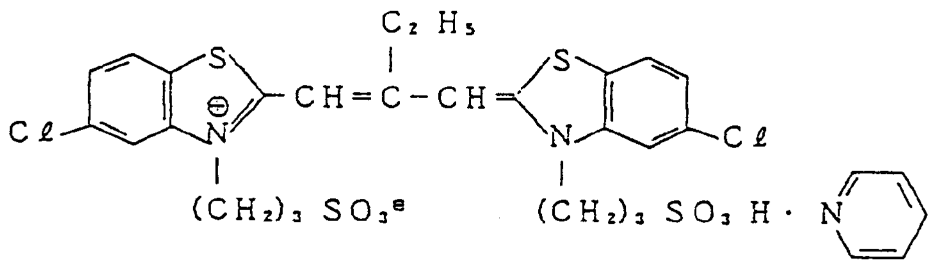

ExS-20

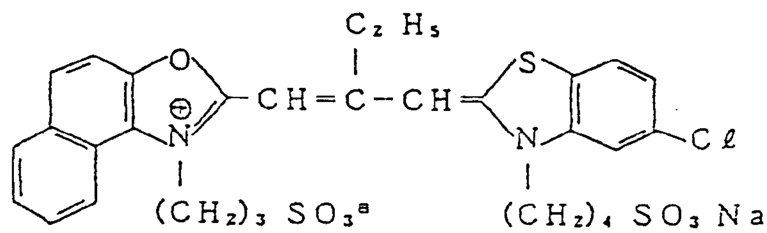

ExS-30

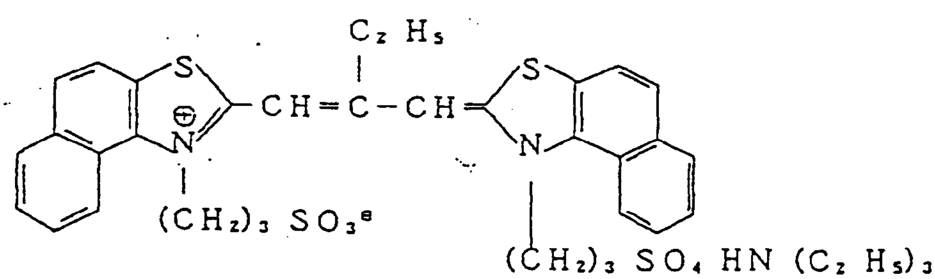

ExS-40

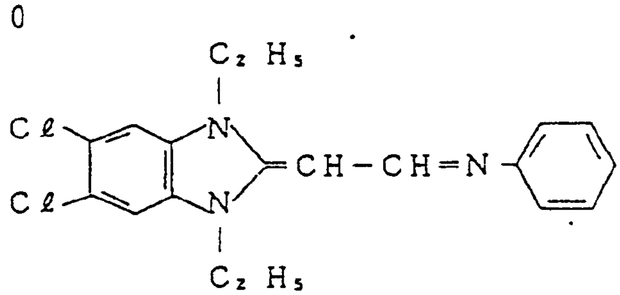

ExS-50

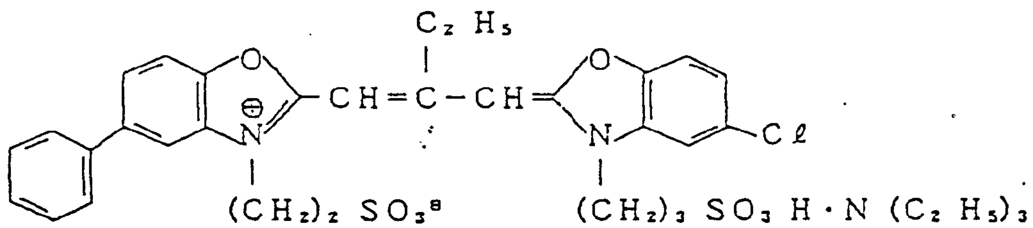

ExS-60

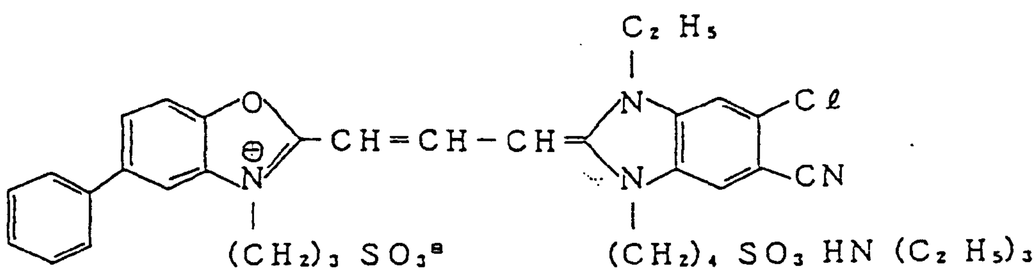

ExS-80

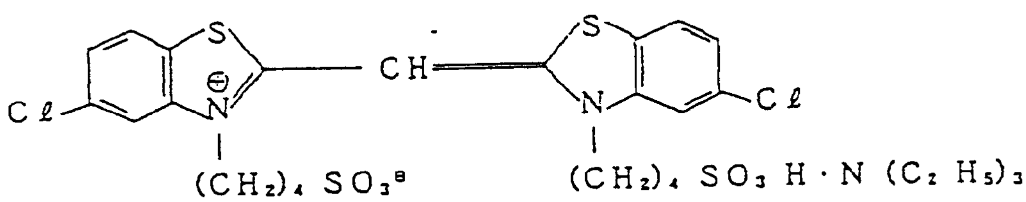

ExS-70

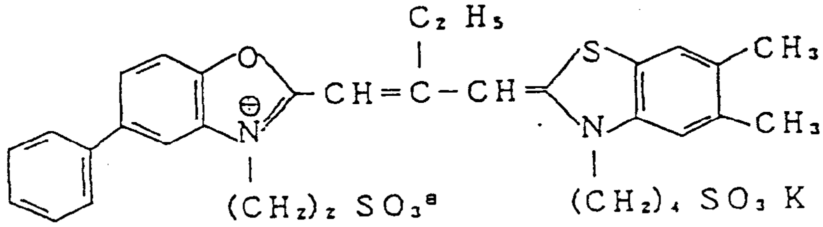

Solv-10

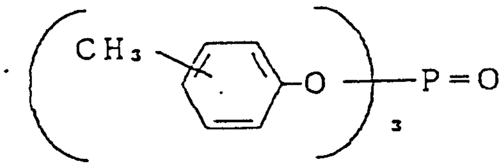

Solv-20

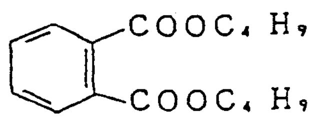

Solv-30

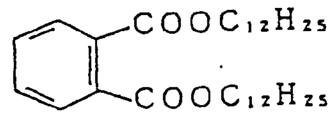

Solv-40

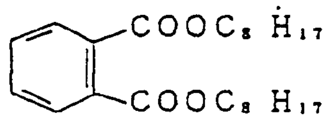

Solv-50

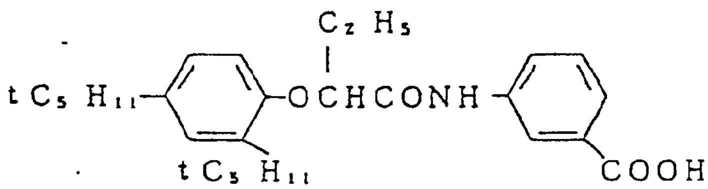

Cpd-10

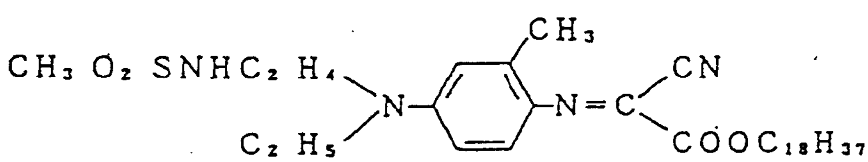

Cpd-20

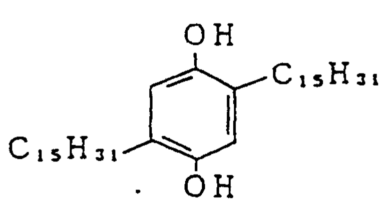

Cpd-30

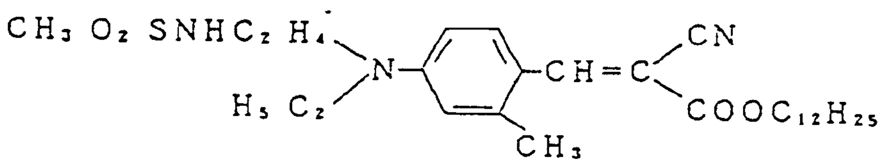

Cpd-40

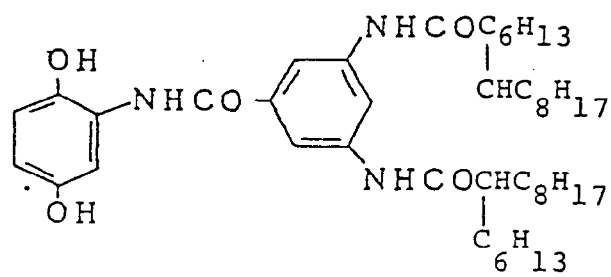

Cpd-50

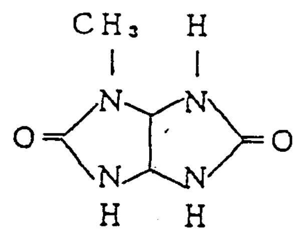

W-10

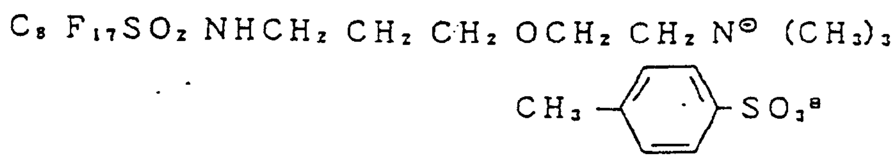

H-10

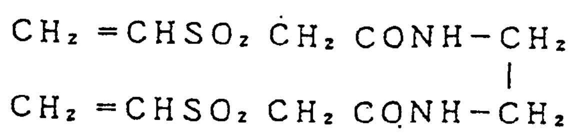

ExM-30

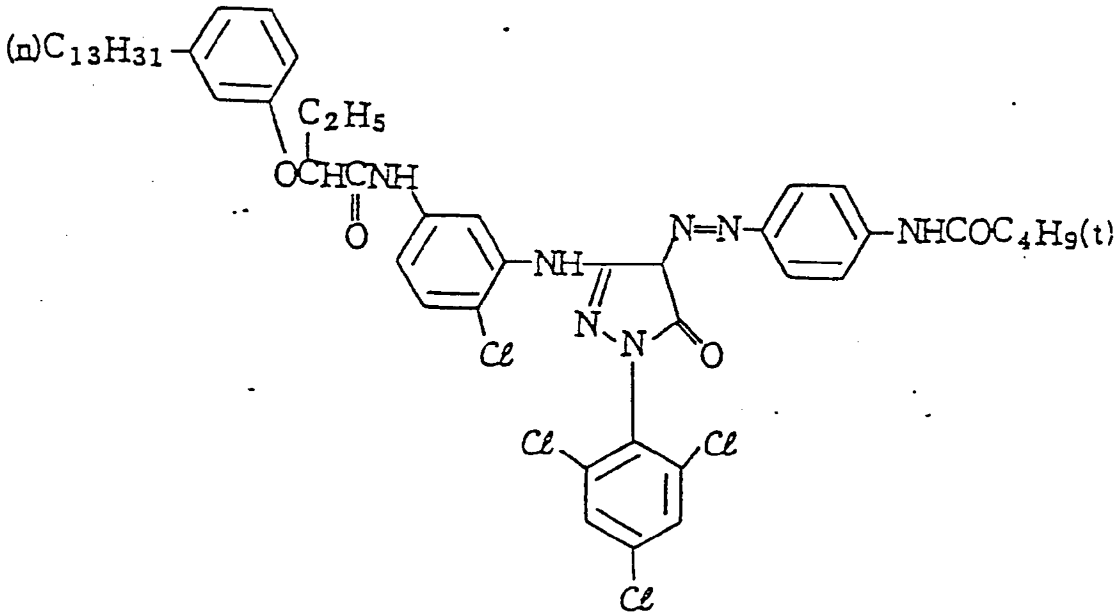

UV-10

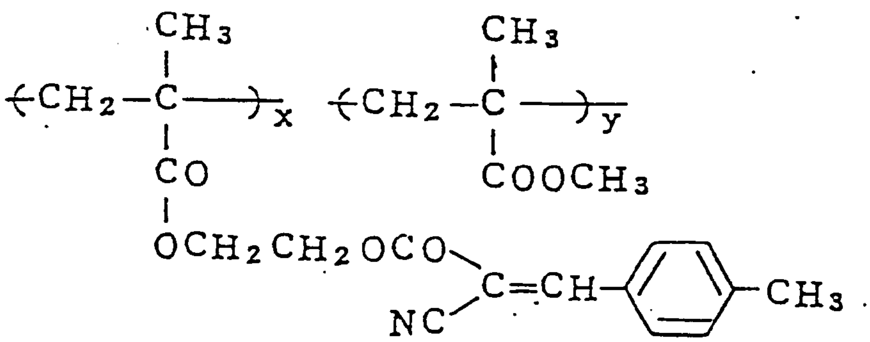

weight ratio : x/y = 7/3

UV-20

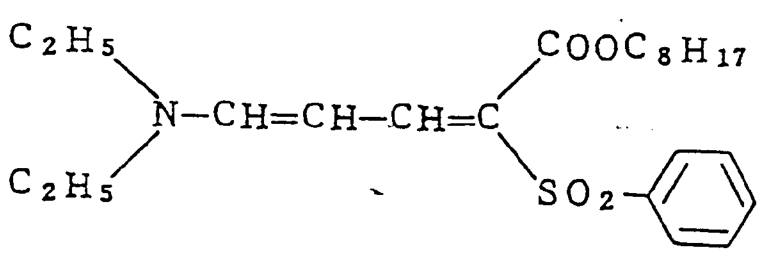

Preparation of Samples 114 to 126:

Samples 114 to 1206 were prepared in the same manner as described for Sample 111 except for using the compounds shown in Table 2 below in place of Coupler ExN-1 used in the first green-sensitive emulsion layer (seventh layer) of Sample 111, respectively. In the same manner as described in Example 1, Ex-B, Ex-C, Ex-D, Ex-E, Ex-F and Ex-G were employed for comparison.

Samples 114 to 126 thus-prepared were exposed wedgewise to white light and then subjected to color development processing in the same manner as described in Example 1. As the results show, almost the same sensitivity and gradation were obtained with each of these samples.

Sharpness of the green-sensitive layer of each sample thus-processed was determined using a conventional MTF value at 25 lines/mm. The results obtained are shown in Table 2 below.

TABLE 2

| Sample | DIR Compound* in First Green-Sensitive Layer | Amount** Added | MTF Value |
|---|---|---|---|
| 111 (Comparison) | ExN-1 | 1.0 | 0.52 |
| 112 ( " ) | Ex-B | 1.0 | 0.53 |
| 113 ( " ) | Ex-C | 1.5 | 0.52 |
| 114 ( " ) | Ex-D | 1.5 | 0.53 |

TABLE 2 (cont'd)

| Sample | DIR Compound* in First Green-Sensitive Layer | Amount** Added | MTF Value |
|---|---|---|---|
| 115 (Present Invention) | (1) | 1.0 | 0.61 |
| 116 ( " ) | (4) | 1.0 | 0.62 |
| 117 ( " ) | (5) | 1.0 | 0.63 |
| 118 ( " ) | (8) | 1.0 | 0.62 |
| 119 ( " ) | (14) | 1.0 | 0.63 |
| 120 ( " ) | (19) | 2.0 | 0.61 |
| 124 (Comparison) | Ex-E | 1.0 | 0.53 |
| 125 ( " ) | Ex-F | 1.0 | 0.53 |
| 126 ( " ) | Ex-G | 1.0 | 0.54 |

* The compound used in place of ExN-1 added to the first green-sensitive emulsion layer

** A molar ratio taking the molar amount of ExN-1 added as 1

From the results shown in Table 2, it is clear that sharpness is extremely improved in case of using compounds according to the present invention, in comparison with the use of conventional compound as is also described in Example 1.

## Claims

1. A silver halide color photographic light-sensitive material comprising a support having thereon at least one silver halide emulsion layer, wherein the silver halide color photographic material contains a compound represented by the following general formula (II):

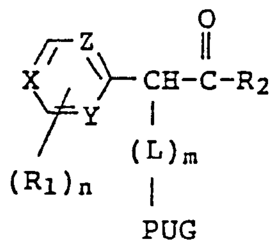

(II)

wherein X, Y and Z, which may be the same or different, each represents a methine group or a nitrogen atom provided that at least one of X, Y and Z is a nitrogen atom; $R_1$ represents a group capable of substituting on the hetero ring; $R_2$ represents an organic moiety; L represents a group capable of being cleaved from

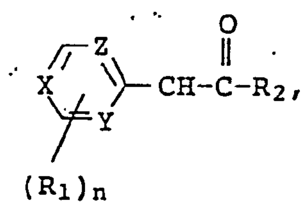

when the compound represented by the general formula (II) is reacted with an oxidation product of a developing agent and capable of releasing PUG after the cleavage from the methine group; PUG represents a photographically useful group; m represents an integer from 0 to 2; and n represents an integer from 0 to 4, when m or n represents 2 or more, two or more $R_1$'s or L's may be the same or different.

2. A silver halide color photographic light-sensitive material as claimed in Claim 1, wherein $R_1$ is selected from a halogen atom, an aliphatic group, an aromatic group, a heterocyclic group, a cyano group, an alkoxy group, an aryloxy group, an aliphatic or aromatic acyloxy group, a carbamoyloxy group, an aliphatic or aromatic sulfonyloxy group, an aliphatic or aromatic acylamino group, an aliphatic or aromatic sulfonamido group, a carbamoyl group, an aliphatic or aromatic acyl group, an aliphatic or aromatic sulfonyl group, an alkoxycarbonyl group and an aryloxycarbonyl group, and when n is 2, two $R_1$'s may combine with each other to form a cyclic structure.

3. A silver halide color photographic light-sensitive material as claimed in Claim 1, wherein $R_2$ is an aromatic group, a heterocyclic group, an aliphatic group, $-OR_3$ or

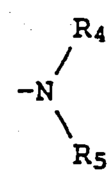

wherein $R_3$ and $R_4$ each represents an aromatic group, a heterocyclic group or an aliphatic group; $R_5$ represents an aromatic group, a heterocyclic group, an aliphatic group or a hydrogen atom; or $R_4$ and $R_5$ may combine with each other to form a cyclic structure.

4. A silver halide color photographic light-sensitive material as claimed in Claim 3, wherein the heterocyclic group is a substituted or unsubstituted heterocyclic group having from 1 to 20 carbon atoms and containing at least one of a nitrogen atom, an oxygen atom and a sulfur atom, as hetero atom.

5. A silver halide color photographic light-sensitive material as claimed in Claim 2, wherein said heterocyclic group is selected from the group consisting of a 2-pyridyl group, a 4-pyridyl group, a 2-thienyl group, a 2-furyl group, a 2-imidazolyl group, a pyrazinyl group, a 2-pyrimidinyl group, a 1-imidazolyl group, a 1-indolyl group, a phthalimido group, a 1,3,4-thiadiazol-2-yl group, a benzoxazol-2-yl group, a 2-quinolyl group, a 2,4-dioxo-1,3-imidazolidin-5-yl group, a 2,4-dioxo-1,3-imidazolidin-3-yl group, a succinimido group, a phthalimido group, a 1,2,4-triazol-2-yl group, and a 1-pyrazolyl group.

6. A silver halide color photographic light-sensitive material as claimed in Claim 3, wherein said heterocyclic group is selected from the group consisting of a 2-pyridyl group, a 4-pyridyl group, a 2-thienyl group, a 2-furyl group, a 2-imidazolyl group, a pyrazinyl group, a 2-pyrimidinyl group, a 1-imidazolyl group, a 1-indolyl group, a phthalimido group, a 1,3,4-thiadiazol-2-yl group, a benzoxazol-2-yl group, a 2-quinolyl group, a 2,4-dioxo-1,3-imidazolidin-5-yl group, a 2,4-dioxo-1,3-imidazolidin-3-yl group, a succinimido group, a phthalimido group, a 1,2,4-triazol-2-yl group, and a 1-pyrazolyl group.

7. A silver halide color photographic light-sensitive material as claimed in Claim 2, wherein at least one of the aliphatic, aromatic and heterocyclic groups is substituted and the substituent is selected from the group consisting of a halogen atom, an alkyl group, an alkoxy group, an aryloxy group, an alkoxycarbonyl group, an aryloxycarbonyl group, an aliphatic or aromatic sulfonamido group, sulfamoyl group, an amino group, a carbamoyl group, an aliphatic or aromatic acylamino group, an aliphatic or aromatic sulfonyl group, a cyano group, an aryl group, an aralkyl group, a nitro gorup, a hydroxy group, a carboxy group, an aliphatic or aromatic acyl group, or a heterocyclic group.

8. A silver halide color photographic light-sensitive material as claimed in Claim 3, wherein at least one of the aliphatic, aromatic and heterocyclic groups is substituted and the substituent is selected from the group consisting of a halogen atom, an alkyl group, an alkoxy group, an aryloxy group, an alkoxycarbonyl group, an aryloxycarbonyl group, an aliphatic or aromatic sulfonamido group, sulfamoyl group, an amino group, a carbamoyl group, an aliphatic or aromatic acylamino group, an aliphatic or aromatic sulfonyl group, a cyano group, an aryl group, an aralkyl group, a nitro gorup, a hydroxy group, a carboxy group, an aliphatic or aromatic acyl group, or a heterocyclic group.

9. A silver halide color photographic light-sensitive material as claimed in Claim 1, wherein the group represented by L is a group represented by the following general formula (T-1):

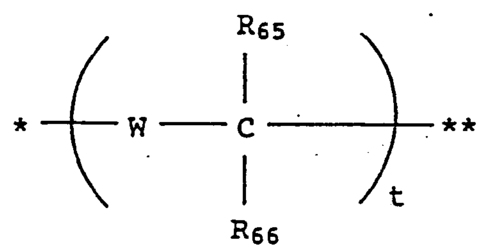

(T-1)

wherein a bond indicated by * denotes the position at which the group is connected to the upper side group in the general formula (II); a bond indicated by ** denotes the position at which the group is connected to the lower side group in the general formula (II); W represents an oxygen atom, a sulfur atom or a group of

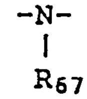

(wherein $R_{67}$ represents a substituent); $R_{65}$ and $R_{66}$ each represents a hydrogen atom or a substituent; t represents 1 or 2; when t represents 2, two

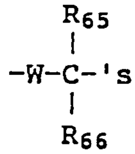

may be the same or different; and any two of $R_{65}$, and $R_{66}$ and $R_{67}$ may be connected to each other to form a cyclic structure.

**10.** A silver halide color photographic light-sensitive material as claimed in Claim 1, wherein the group represented by L is a group represented by the following general formula (T-2):

*-Nu-Link-E-** (T-2)

wherein a bond indicated by * denotes the position at which the group is connected to the upper side group in the general formula (II); a bond indicated by ** denotes the position at which the group is connected to the lower side group in the general formula (II); Nu represents a nucleophilic group; E represents an electrophilic group which is able to cleave the bond indicated by ** upon a nucleophilic attack of Nu; and Link represents a linking group which connects Nu with E in a stereochemical position capable of causing an intramolecular nucleophilic displacement reaction between Nu and E.

**11.** A silver halide color photographic light-sensitive material as claimed in Claim 1, wherein the group represented by L is a group represented by the following general formula (T-3):

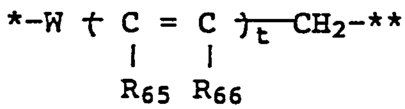 (T-3)

wherein a bond indicated by * denotes the position at which the group is connected to the upper side group in the general formula (II); a bond indicated by ** denotes the position at which the group is connected to the lower side group in the general formula (II); W represents an oxygen atom, a sulfur atom or a group of

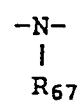

(wherein $R_{67}$ represents a substituent); $R_{65}$ and $R_{66}$ each represents a hydrogen atom or a substituent; t represents 1 or 2, when t represents 2, two

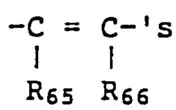

may be the same or different; and $R_{65}$ and $R_{66}$ may be connected to each other to form a cyclic structure.

**12.** A silver halide color photographic light-sensitive material as claimed in Claim 1, wherein the group represented by L is a group represented by the following general formula (T-4) or (T-5):

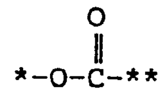

(T-4)

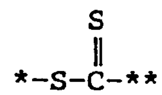

(T-5)

wherein a bond indicated by * denotes the position at which the group is connected to the upper side group in the general formula (II); and a bond indicated by ** denotes the position at which the group is connected to the lower side group in the general formula (II).

**13.** A silver halide color photographic light-sensitive material as claimed in Claim 1, wherein the group represented by L is a group represented by the following general formula (T-6):

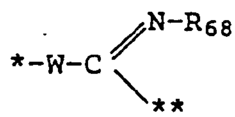

(T-6)

wherein a bond indicated by * denotes the position at which the group is connected to the upper side group in the general formula (II); a bond indicated by ** denotes the position at which the group is connected to the lower side group in the general formula (II); W represents an oxygen atom, a sulfur atom or a group of

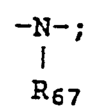

and $R_{67}$ and $R_{68}$ each represents a substituent.

**14.** A silver halide color photographic light-sensitive material as claimed in Claim 1, wherein the group represented by PUG is a development inhibitor.

**15.** silver halide color photographic light-sensitive material as claimed in Claim 14, wherein the development inhibitor is a group selected from the group consisting of groups represented by the following general formula (D-1), (D-2), (D-3), (D-4), (D-5), (D-6), (D-7), (D-8), (D-9), (D-10), (D-11) and (D-12):

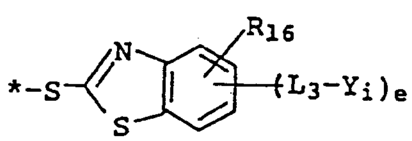

(D-1)

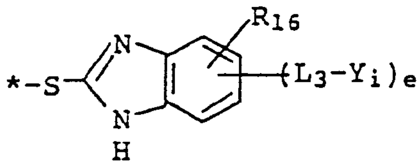

(D-2)

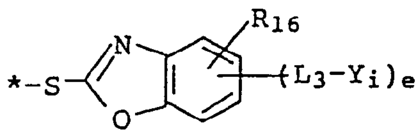

(D-3)

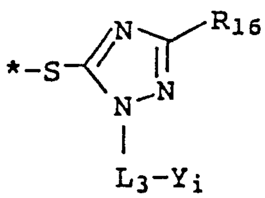

(D-4)

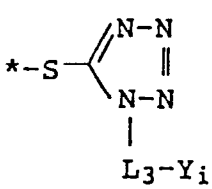

(D-5)

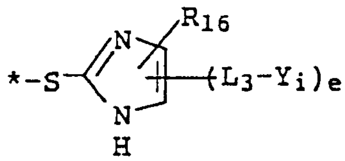

(D-6)

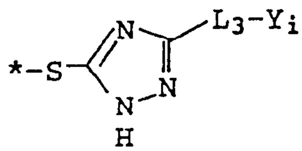

(D-7)

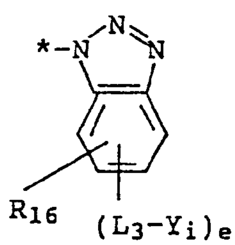

(D-8)

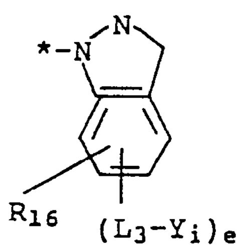

(D-9)

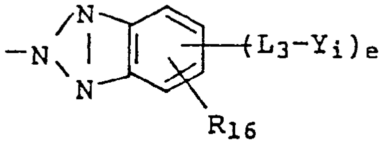

(D-10)

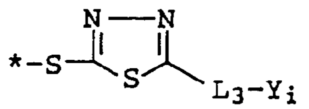

(D-11)

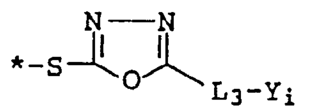

(D-12)

wherein a bond indicated by * denotes the position at which the group is connected to the methine group or the linking group (L) in the general formula (II); $R_{16}$ represents a hydrogen atom or a substituent; e represents 1 or 2; $L_3$ represents a group containing a chemical bond which is capable of being cleaved in a developing solution; and $Y_i$ represents a substituent capable of generating the development inhibiting function and is selected from an aliphatic group, an aromatic group or a heterocyclic group.

16. A silver halide color photographic light-sensitive material as claimed in Claim 15, wherein the substituent represented by $R_{16}$ is selected from an aliphatic group, an aliphatic or aromatic acylamino group, an alkoxy group, a halogen atom, a nitro group, and a sulfonamido group.

17. A silver halide color photographic light-sensitive material as claimed in Claim 15, wherein the chemical bond included in $L_3$ is selected from -COO-, -NHCOO-, $-SO_2O-$, $-OCH_2CH_2SO_2-$,

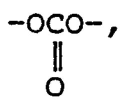

and

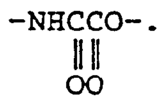

18. A silver halide color photographic light-sensitive material as claimed in Claim 1, wherein the amount of the compound is in a range from $1\times10^{-6}$ mol to 0.5 mol per mol of silver present in the same layer to which it is to be added or a layer adjacent thereto.

19. A silver halide color photographic light-sensitive material as claimed in Claim 1, wherein said silver halide emulsion layer contains a color image forming coupler in a molar ratio of the coupler to the compound represented by formular (II) of from 0.01/99.99 to 50/50.

**Patentansprüche**

1. Lichtempfindliches farbphotographisches Silberhalogenidmaterial, umfassend einen Träger und darauf mindestens eine Silberhalogenidemulsionsschicht, wobei das farbphotographische Silberhalogenidmaterial eine Verbindung der folgenden allgemeinen Formel (II) enthält:

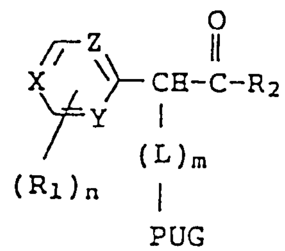

(II)

worin X, Y und Z, die gleich oder verschieden sein können, jeweils eine Methin-Gruppe oder ein Stickstoffatom darstellen, mit der Maßgabe, daß mindestens eine Gruppe aus X, Y und Z ein Stickstoffatom ist; $R_1$ eine Gruppe ist, die ein Substituent am Heteroring sein kann; $R_2$ eine organische Einheit darstellt; L eine Gruppe darstellt, die von

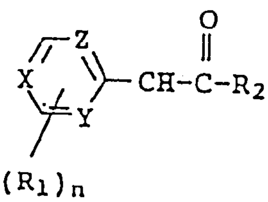

abgespalten werden kann, wenn die durch die allgemeine Formel (II) dargestellte Verbindung mit einem Oxidationsprodukt eines Entwicklungsmittels umgesetzt wird, und die imstande ist, PUG nach Abspaltung von der Methin-Gruppe freizusetzen; PUG eine photographisch verwendbare Gruppe darstellt; m eine ganze Zahl von 0 bis 2 darstellt; und n eine Ganzzahl von 0 bis 4 darstellt, wobei wenn m oder n 2 oder mehr sind, zwei oder mehr $R_1$ oder L gleich oder verschieden sein können.

2. Lichtempfindliches farbphotographisches Silberhalogenidmaterial gemäß Anspruch 1, worin $R_1$ ausgewählt ist aus einem Halogenatom, einer aliphatischen Gruppe, einer aromatischen Gruppe, einer heterocyclischen Gruppe, einer Cyano-Gruppe, einer Alkoxy-Gruppe, einer Aryloxy-Gruppe, einer aliphatischen oder aromatischen Acyloxy-Gruppe, einer Carbamoyloxy-Gruppe, einer aliphatischen oder aromatischen Sulfonyloxy-Gruppe, einer aliphatischen oder aromatischen Acylamino-Gruppe, einer aliphatischen oder aromatische Sulfonamido-Gruppe, einer Carbamoyl-Gruppe, einer aliphatischen oder aromatischen Acyl-Gruppe, einer aliphatischen oder aromatischen Sulfonyl-Gruppe, einer Alkoxycarbonyl-Gruppe und einer Aryloxycarbonyl-Gruppe, und wenn n 2 ist, können zwei $R_1$ miteinander unter Bildung einer cyclischen Struktur kombinieren.

3. Lichtempfindliches farbphotographisches Silberhalogenidmaterial gemäß Anspruch 1, worin $R_2$ eine aromatische Gruppe, eine heterocyclische Gruppe, eine aliphatische Gruppe, $-OR_3$ oder

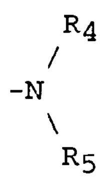

ist, worin $R_3$ und $R_4$ jeweils eine aromatische Gruppe, eine heterocyclische Gruppe oder eine aliphatische Gruppe darstellen; $R_5$ eine aromatische Gruppe, eine heterocyclische Gruppe, eine aliphatische Gruppe oder ein Wasserstoffatom darstellt; oder $R_4$ und $R_5$ miteinander unter Bildung einer cyclischen Struktur kombinieren können.

4. Lichtempfindliches farbphotographisches Silberhalogenidmaterial gemäß Anspruch 3, worin die heterocyclische Gruppe eine substituierte oder unsubstituierte heterocyclische Gruppe mit 1 bis 20 Kohlenstoffatomen ist, die mindestens ein Atom aus einem Stickstoffatom, einem Sauerstoffatom und einem Schwefelatom als Heteroatom enthält.

5. Lichtempfindliches farbphotographisches Silberhalogenidmaterial gemäß Anspruch 2, worin die heterocyclische Gruppe ausgewählt aus der Gruppe bestehend aus einer 2-Pyridyl-Gruppe, einer 4-Pyridyl-Gruppe, einer 2-Thienyl-Gruppe, einer 2-Furyl-Gruppe, einer 2-Imidazolyl-Gruppe, einer Pyrazinyl-Gruppe, einer 2-Pyrimidinyl-Gruppe, einer 1-Imidazolyl-Gruppe, einer 1-Indolyl-Gruppe, einer Phthalimido-Gruppe, einer 1,3,4-Thiadiazol-2-yl-Gruppe, einer Benzoxazol-2-yl-Gruppe, einer 2-Chinolyl-Gruppe, einer 2,4-Dioxo-1,3-imidazolidin-5-yl-Gruppe, einer 2,4-Dioxo-1,3-imidazolidin-3-yl-Gruppe, einer Succinimido-Gruppe, einer Phthalimido-Gruppe, einer 1,2,4-Triazol-2-yl-Gruppe und einer 1-Pyrazolyl-Gruppe.

6. Lichtempfindliches farbphotographisches Silberhalogenidmaterial gemäß Anspruch 3, worin die heterocyclische Gruppe ausgewählt ist aus der Gruppe bestehend aus einer 2-Pyridyl-Gruppe, einer 4-Pyridyl-Gruppe, einer 2-Thienyl-Gruppe, einer 2-Furyl-Gruppe, einer 2-Imidazolyl-Gruppe, einer Pyrazinyl-Gruppe, einer 2-Pyrimidinyl-Gruppe, einer 1-Imidazolyl-Gruppe, einer 1-Indolyl-Gruppe, einer Phthalimido-Gruppe, einer 1,3,4-Thiadiazol-2-yl-Gruppe, einer Benzoxazol-2-yl-Gruppe, einer 2-Chinolyl-Gruppe, einer 2,4-Dioxo-1,3-imidazolidin-5-yl-Gruppe, einer 2,4-Dioxo-1,3-imidazolidin-3-yl-Gruppe, einer Succinimido-Gruppe, einer Phthalimido-Gruppe, einer 1,2,4-Triazol-2-yl-Gruppe und einer 1-Pyrazolyl-Gruppe.

7. Lichtempfindliches farbphotographisches Silberhalogenidmaterial gemäß Anspruch 2, worin mindestens eine der aliphatischen, aromatischen und heterocyclischen Gruppen substituiert ist und der Substituent ausgewählt ist aus der Gruppe bestehend aus einem Halogenatom, einer Alkyl-Gruppe, einer Alkoxy-Gruppe, einer Aryloxy-Gruppe, einer Alkoxycarbonyl-Gruppe, einer Aryloxycarbonyl-Gruppe, einer aliphatischen oder aromatischen Sulfonamido-Gruppe, einer Sulfamoyl-Gruppe, einer Amino-Gruppe, einer Carbamoyl-Gruppe, einer aliphatischen oder aromatischen Acylamino-Gruppe, einer aliphatischen oder aromatischen Sulfonyl-Gruppe, einer Cyano-Gruppe, einer Aryl-Gruppe, einer Aralkyl-Gruppe, einer Nitro-Gruppe, einer Hydroxy-Gruppe, einer Carboxy-Gruppe, einer aliphatischen oder aromatischen Acyl-Gruppe oder einer heterocyclischen Gruppe.

8. Lichtempfindliches farbphotographisches Silberhalogenidmaterial gemäß Anspruch 3, worin mindestens eine der aliphatischen, aromatischen und heterocyclischen Gruppen substituiert ist und der Substituent ausgewählt ist aus der Gruppe bestehend aus einem Halogenatom, einer Alkyl-Gruppe, einer Alkoxy-Gruppe, einer Aryloxy-Gruppe, einer Alkoxycarbonyl-Gruppe, einer Aryloxycarbonyl-Gruppe, einer aliphatischen oder aromatischen Sulfonamido-Gruppe, einer Sulfamoyl-Gruppe, einer Amino-Gruppe, einer Carbamoyl-Gruppe, einer aliphatischen oder aromatischen Acylamino-Gruppe, einer aliphatischen oder aromatischen Sulfonyl-Gruppe, einer Cyano-Gruppe, einer Aryl-Gruppe, einer Aralkyl-Gruppe, einer Nitro-Gruppe, einer Hydroxy-Gruppe, einer Carboxy-Gruppe, einer aliphatischen oder aromatischen Acyl-Gruppe oder einer heterocyclischen Gruppe.

9. Lichtempfindliches farbphotographisches Silberhalogenidmaterial gemäß Anspruch 1, worin die durch L dargestellte Gruppe eine Gruppe ist, die durch die allgemeine Formel (T-1) dargestellt wird:

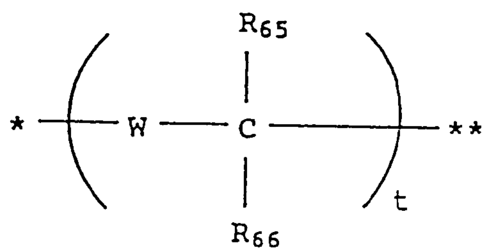

(T-1)

worin die durch * bezeichnete Bindung die Position angibt, in der die Gruppe mit der oberen Gruppe in der allgemeinen Formel (II) verbunden ist; die durch ** angegebene Bindung die Position bezeichnet, in der die Gruppe mit der unteren Gruppe in der allgemeinen Formel (II) verbunden ist; W ein Sauerstoffatom, ein Schwefelatom oder eine

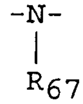

Gruppe darstellt (worin $R_{67}$ einen Substituenten darstellt); $R_{65}$ und $R_{66}$ jeweils ein Wasserstoffatom oder einen Substituenten darstellen; t 1 oder 2 darstellt; wenn t 2 darstellt, zwei

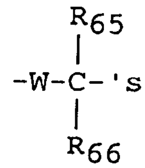

gleich oder verschieden sein können; und beliebige zwei Gruppen aus $R_{65}$, $R_{66}$ und $R_{67}$ miteinander unter Bildung einer cyclischen Struktur verbunden sein können.

10. Lichtempfindliches farbphotographisches Silberhalogenidmaterial gemäß Anspruch 1, worin die durch L dargestellte Gruppe eine Gruppe der folgenden allgemeinen Formel (T-2) ist:

*-Nu-Link-E-** (T-2)

worin eine durch * bezeichnete Bindung die Position angibt, in der die Gruppe mit der oberen Gruppe in der allgemeinen Formel (II) verbunden ist; eine durch ** bezeichnete Bindung die Position bezeichnet, in der die Gruppe mit der unteren Gruppe in der allgemeinen Formel (II) verbunden ist; Nu eine nukleophile Gruppe darstellt; E eine elektrophile Gruppe darstellt, die die durch ** bezeichnete Bindung bei einer nukleophilen Attacke von Nu abspalten kann; und Link eine verbindende Gruppe darstellt, die Nu mit E in einer stereochemischen Position verbindet, die imstande ist, eine intramolekulare nukleophile Verschiebungsreaktion zwischen Nu und E zu bewirken.

11. Lichtempfindliches farbphotographisches Silberhalogenidmaterial gemäß Anspruch 1, worin die durch L dargestellte Gruppe eine Gruppe der folgenden allgemeinen Formel (T-3) ist:

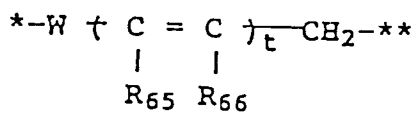 (T-3)

worin eine mit einem * bezeichnete Bindung die Position angibt, in der die Gruppe mit der oberen Gruppe in der allgemeinen Formel (II) verbunden ist; die durch ** bezeichnete Bindung die Position angibt, in der die Gruppe mit der unteren Gruppe in der allgemeinen Formel (II) verbunden ist; W ein Sauerstoffatom, ein Schwefelatom oder eine

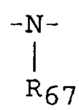

Gruppe darstellt (worin $R_{67}$ einen Substituenten darstellt); $R_{65}$ und $R_{66}$ jeweils ein Wasserstoffatom oder einen Substituenten darstellen; t 1 oder 2 darstellt, wenn t 2 darstellt, können zwei

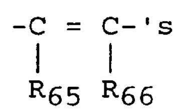

gleich oder verschieden sein; und $R_{65}$ und $R_{66}$ können miteinander unter Bildung einer cyclischen Struktur

verbunden sein.

**12.** Lichtempfindliches farbphotographisches Silberhalogenidmaterial gemäß Anspruch 1, worin die durch L dargestellte Gruppe eine Gruppe der folgenden allgemeinen Formeln (T-4) oder (T-5) ist:

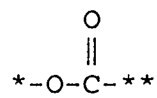

(T-4)

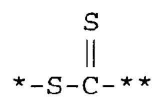

(T-5)

worin eine durch * bezeichnete Bindung die Position angibt, in der die Gruppe mit der oberen Gruppe in der allgemeinen Formel (II) verbunden ist und die durch ** bezeichnete Bindung die Position angibt, in der die Gruppe mit der unteren Gruppe in der allgemeinen Formel (II) verbunden ist.

**13.** Lichtempfindliches farbphotographisches Silberhalogenidmaterial gemäß Anspruch 1, worin die durch L dargestellte Gruppe eine Gruppe der folgenden allgemeinen Formel (T-6) ist:

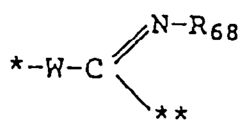

(T-6)

worin eine durch * bezeichnete Bindung die Position angibt, in der die Gruppe mit der oberen Gruppe in der allgemeinen Formel (II) verbunden ist; eine durch ** bezeichnete Bindung die Position angibt, in der die Gruppe mit der unteren Gruppe in der allgemeinen Formel (II) verbunden ist; W ein Sauerstoffatom, ein Schwefelatom oder eine

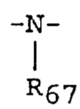

Gruppe bezeichnet; und $R_{67}$ und $R_{68}$ jeweils einen Substituenten bedeuten.

**14.** Lichtempfindliches farbphotographisches Silberhalogenidmaterial gemäß Anspruch 1, worin die durch PUG dargestellte Gruppe ein Entwicklungsinhibitor ist.

**15.** Lichtempfindliches farbphotographisches Silberhalogenidmaterial gemäß Anspruch 14, worin der Entwicklungsinhibitor eine Gruppe ist, die ausgewählt ist aus Gruppen der folgenden allgemeinen Formeln (D-1), (D-2), (D-3), (D-4), (D-5), (D-6), (D-7), (D-8), (D-9), (D-10), (D-11) und (D-12):

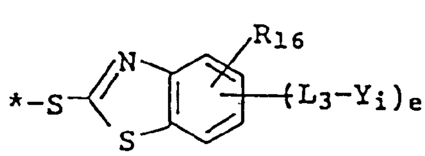

(D-1)

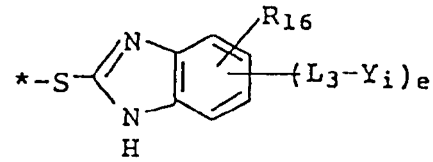

(D-2)

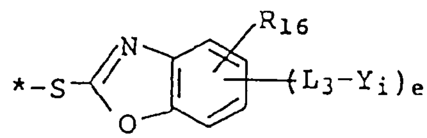

(D-3)

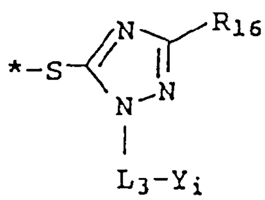

(D-4)

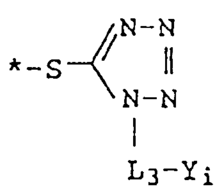

(D-5)

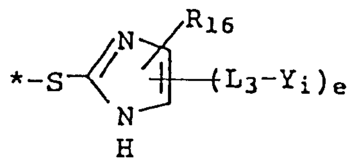

(D-6)

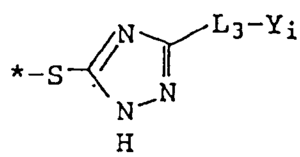

(D-7)

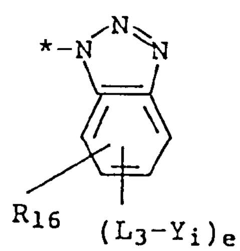

(D-8)

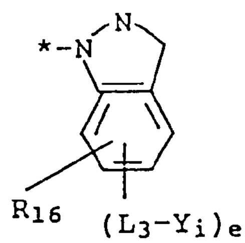

(D-9)

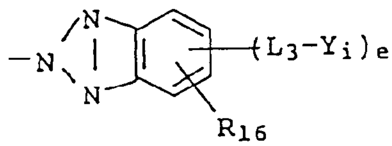

(D-10)

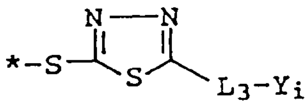

(D-11)

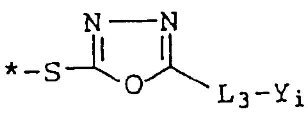

(D-12)

worin eine durch * bezeichnete Bindung die Position angibt, in der die Gruppe mit der Methin-Gruppe oder mit der verbindenden Gruppe (L) in der allgemeinen Formel (II) verbunden ist; $R_{16}$ ein Wasserstoffatom oder einen Substituenten darstellt; e 1 oder 2 darstellt; $L_3$ eine Gruppe darstellt, die eine chemische Bindung enthält, die in einer Entwicklungslösung abgespalten werden kann; und $Y_i$ einen Substituenten darstellt, der die entwicklungsinhibierende Funktion erzeugen kann und ausgewählt ist aus einer aliphatischen Gruppe, einer aromatischen Gruppe oder einer heterocyclischen Gruppe.

**16.** Lichtempfindliches farbphotographisches Silberhalogenidmaterial gemäß Anspruch 15, worin der durch $R_{16}$ dargestellte Substituent ausgewählt ist aus einer aliphatischen Gruppe, einer aliphatischen oder aromatischen Acylamino-Gruppe, einer Alkoxy-Gruppe, einem Halogenatom, einer Nitro-Gruppe und einer Sulfonamido-Gruppe.

**17.** Lichtempfindliches farbphotographisches Silberhalogenidmaterial gemäß Anspruch 15, worin die chemische Bindung, die in $L_3$ eingeschlossen ist, ausgewählt ist aus -COO-, -NHCOO-, $-SO_2O-$, $-OCH_2CH_2SO_2-$ ,

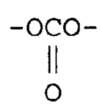

und

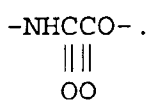

**18.** Lichtempfindliches farbphotographisches Silberhalogenidmaterial gemäß Anspruch 1, worin die Menge

der Verbindung in einem Bereich von 1 x $10^{-6}$ Mol bis 0,5 Mol pro Mol Silber, das in derselben Schicht, zu der es zugesetzt wird, oder einer benachbarten Schicht vorliegt, liegt.

19. Lichtempfindliches farbphotographisches Silberhalogenidmaterial gemäß Anspruch 1, worin die Silberhalogenidemulsionsschicht einen Farbbilderzeugenden Kuppler in einem Molverhältnis von Kuppler zu Verbindung der Formel (II) von 0,01/99,99 bis 50/50 enthält.

## Revendications

1. Matériau photographique couleur à l'halogénure d'argent sensible à la lumière, comportant un support portant au moins une couche d'émulsion d'halogénure d'argent, dans laquelle le matériau photographique couleur à l'halogénure d'argent contient un composé représenté par la formule générale (II) ci-dessous

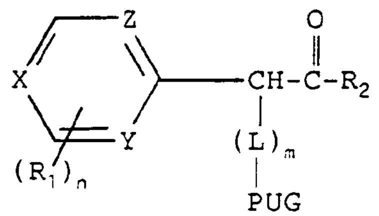

(II)

dans laquelle, X, Y, Z, qui peuvent être identiques ou différents, représentent chacun un radical méthine ou un atome d'azote, avec la condition qu'au moins un parmi X, Y et Z soit un atome d'azote; $R_1$ représente un radical capable de se substituer sur l'hétéro cycle; $R_2$ représente un radical organique; L représente un radical susceptible d'être séparé de

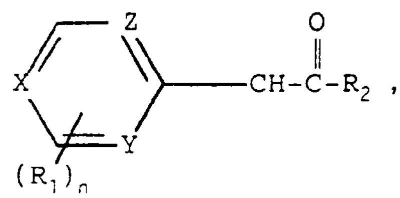

lorsque le composé représenté par la formule générale (II) est mis à réagir avec un produit d'oxydation d'un agent révélateur, et est capable de libérer un PUG après séparation du groupe méthine; PUG représente un radical photographiquement utile; m représente un entier de 0 à 2; et n représente un entier de 0 à 4, lorsque m ou n représente 2 ou d'avantage, deux ou plusieurs des $R_1$ ou des L peuvent être identiques ou différents.

2. Matériau photographique couleur à l'halogénure d'argent sensible à la lumière selon la revendication 1, dans lequel $R_1$ est choisi parmi un atome d'halogène, un radical aliphatique, un radical aromatique, un radical hétérocyclique, un radical cyano, un radical alkoxy, un radical aryloxy, un radical acyloxy aliphatique ou aromatique, un radical carbamoyloxy, un radical sulfonyloxy aliphatique ou aromatique, un radical acylamino aliphatique ou aromatique, un radical sulfonamido aliphatique ou aromatique, un radical carbamoyle, un radical acyle aliphatique ou aromatique, un radical sulfonyle aliphatique ou aromatique, un radical alkoxycarbonyle et un radical aryloxycarbonyle, et lorsque n vaut 2, deux $R_1$ peuvent se combiner l'un avec l'autre pour former une structure cyclique.

3. Matériau photographique couleur à l'halogénure d'argent sensible à la lumière selon la revendication 1, dans lequel $R_2$ est un radical aromatique, un radical hétérocyclique, un radical aliphatique, $-OR_3$ ou

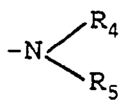
,

dans laquelle $R_3$ et $R_4$ représentent chacun un radical aromatique, un radical hétérocyclique ou un radical aliphatique; $R_5$ représente un radical aromatique, un radical hétérocyclique, un radical aliphatique ou un atome d'hydrogène; ou $R_4$ et $R_5$ peuvent se combiner l'un avec l'autre pour former une structure cyclique.

4. Matériau photographique couleur à l'halogénure d'argent sensible à la lumière selon la revendication 3, dans lequel le radical hétérocyclique est un radical hétérocyclique substitué ou non substitué possédant de 1 à 20 atomes de carbone et contenant comme hétéro atome, au moins l'un parmi un atome d'hydrogène, un atome d'oxygène et un atome de soufre

5. Matériau photographique couleur à l'halogénure d'argent sensible à la lumière selon la revendication 2, dans lequel ledit radical hétérocyclique est choisi dans le groupe constitué d'un radical 2-pyridyle, un radical 4-pyridyle, un radical 2-thiényle, un radical 2-furyle; un radical 2-imidazolyle, un radical pyrazinyle, un radical 2-pyrimidinyle, un radical 1-imidazolyle, un radical 1-indolyle, un radical phtalimido, un radical 1,3,4-thiadiazol-2-yle, un radical benzoxazol-2-yle, un radical 2-quinolyle, un radical 2,4-dioxo-1,3-imidazolidine-5-yle, un radical 2,4-dioxo-1,3-imidazolidine-3-yle, un radical succinimido, un radical phtalimido, un radical 1,2,4-triazol-2-yle, et un radical 1-pyrazolyle.

6. Matériau photographique couleur à l'halogénure d'argent sensible à la lumière selon la revendication 3, dans lequel ledit radical hétérocyclique est choisi dans le groupe constitué d'un radical 2-pyridyle, un radical 4-pyridyle, un radical 2-thiényle, un radical 2-furyle; un radical 2-imidazolyle, un radical pyrazinyle, un radical 2-pyrimidinyle, un radical 1-imidazolyle, un radical 1-indolyle, un radical phtalimido, un radical 1,3,4-thiadiazol-2-yle, un radical benzoxazol-2-yle, un radical 2-quinolyle, un radical 2,4-dioxo-1,3-imidazolidine-5-yle, un radical 2,4-dioxo-1,3-imidazolidine-3-yle, un radical succinimido, un radical phtalimido, un radical 1,2,4-triazol-2-yle, et un radical 1-pyrazolyle.

7. Matériau photographique couleur à l'halogénure d'argent sensible à la lumière selon la revendication 2, dans lequel au moins un des radicaux aliphatiques, aromatiques et hétérocycliques est substitué, et le substituant est choisi dans le groupe constitué d'un atome d'halogène, d'un radical alkyle, d'un radical alkoxy, d'un radical aryloxy, d'un radical alkoxycarbonyle, d'un radical aryloxycarbonyle, d'un radical sulfonamido aliphatique ou aromatique, d'un radical sulfamoyle, d'un radical amino, d'un radical carbamoyle, d'un radical acylamino aliphatique ou aromatique, d'un radical sulfonyle aliphatique ou aromatique, d'un radical cyano, d'un radical aryle, d'un radical aralkyle, d'un radical nitro, d'un radical hydroxy, d'un radical carboxy, d'un radical acyle aliphatique ou aromatique, ou d'un radical hétérocyclique.

8. Matériau photographique couleur à l'halogénure d'argent sensible à la lumière selon la revendication 3, dans lequel au moins un des radicaux aliphatiques, aromatiques et hétérocycliques est substitué, et le substituant est choisi dans le groupe constitué d'un atome d'halogène, d'un radical alkyle, d'un radical alkoxy, d'un radical aryloxy, d'un radical alkoxycarbonyle, d'un radical aryloxycarbonyle, d'un radical sulfonamido aliphatique ou aromatique, d'un radical sulfamoyle, d'un radical amino, d'un radical carbamoyle, d'un radical acylamino aliphatique ou aromatique, d'un radical sulfonyle aliphatique ou aromatique, d'un radical cyano, d'un radical aryle, d'un radical aralkyle, d'un radical nitro, d'un radical hydroxy, d'un radical carboxy, d'un radical acyle aliphatique ou aromatique, ou d'un radical hétérocyclique.

9. Matériau photographique couleur à l'halogénure d'argent sensible à la lumière selon la revendication 1, dans lequel le radical représenté par L est un radical représenté par la formule générale (T-1) ci-dessous:

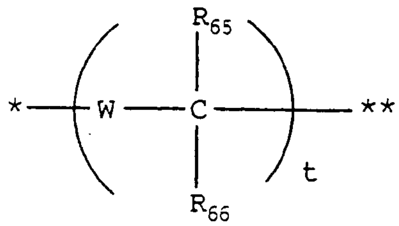 (T-1)

dans laquelle une liaison indiquée par * désigne la position dans laquelle le radical est relié au radical du côté supérieur de la formule générale (II); une liaison indiquée par ** désigne la position dans laquelle le radical est relié au radical du côté inférieur de la formule générale (II); W représente un atome d'oxygène, un atome de soufre ou un radical de

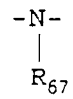

(ou $R_{67}$ représente un substituant); $R_{65}$ et $R_{66}$ représentent chacun un atome d'hydrogène ou un substituant; t représente 1 ou 2; lorsque t représente 2, deux

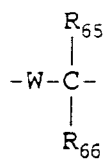

peuvent être identiques ou différents; et deux quelconques parmi $R_{65}$, et $R_{66}$ et $R_{67}$ peuvent être reliés l'un à l'autre pour former une structure cyclique.

**10.** Matériau photographique couleur à l'halogénure d'argent sensible à la lumière selon la revendication 1, dans lequel le radical représenté par L est un radical représenté par la formule générale (T-2) ci-dessous:

*-Nu-Link-E-** (T-2)

dans laquelle une liaison indiquée par * désigne la position dans laquelle le radical est relié au radical du côté supérieur de la formule générale (II); une liaison indiquée par ** désigne la position dans laquelle le radical est relié au radical du côté inférieur de la formule générale (II); Nu représente un radical nucléophile; E représente un radical électrophile susceptible de rompre la liaison indiquée par ** lors d'une attaque nucléophile de Nu; et Link représente un radical de liaison qui relie Nu à E dans une position stéréochimique capable de provoquer une réaction intramoléculaire de déplacement nucléophile entre Nu et E.

**11.** Matériau photographique couleur à l'halogénure d'argent sensible à la lumière selon la revendication 1, dans lequel le radical représenté par L est un radical représenté par la formule générale (T-3) ci-dessous:

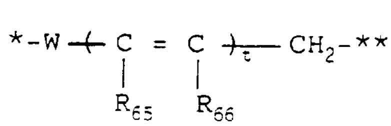 (T-3)

dans laquelle une liaison indiquée par * désigne la position dans laquelle le radical est relié au radical du côté supérieur de la formule générale (II); une liaison indiquée par ** désigne la position dans laquelle le radical est relié au radical du côté inférieur de la formule générale (II); W représente un atome d 'oxygène, un atome de soufre ou un radical de

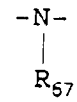

(où $R_{67}$ représente un substituant) ; $R_{65}$ et $R_{66}$ représentent chacun un atome d'hydrogène ou un substituant; t représente 1 ou 2: lorsaue t représente 2, deux

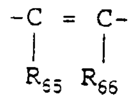

peuvent être identiques ou différents; et $R_{65}$ et $R_{66}$ peuvent être reliés l'un à l'autre pour former une structure cyclique.

12. Matériau photographique couleur à l'halogénure d'argent sensible à la lumière selon la revendication 1, dans lequel le radical représenté par L est un radical représenté par la formule générale ci-dessous (T-4) ou (T-5):

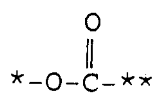

(T-4)

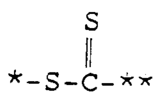

(T-5)

dans lesquelles une liaison indiquée par * désigne la position dans laquelle le radical est relié au radical du côté supérieur de la formule générale (II); une liaison indiquée par ** désigne la position dans laquelle le radical est relié au radical du côté inférieur de la formule générale (II).

13. Matériau photographique couleur à l'halogénure d'argent sensible à la lumière selon la revendication 1, dans lequel le radical représenté par L est un radical représenté par la formule générale (T-6) ci-dessous:

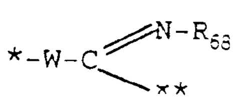

(T-6)

dans laquelle une liaison indiquée par * désigne la position dans laquelle le radical est relié au radical du côté supérieur de la formule générale (II); une liaison indiquée par ** désigne la position dans laquelle le radical est relié au radical du côté inférieur de la formule générale (II); W représente un atome d'oxygène, un atome de soufre ou un radical de

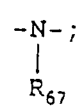

et $R_{67}$ et $R_{68}$ représentent chacun un substituant.

14. Matériau photographique couleur à l'halogénure d'argent sensible à la lumière selon la revendication 1, dans lequel le radical représenté par PUG est un inhibiteur de développement.

**15.** Matériau photographique couleur à l'halogénure d'argent sensible à la lumière selon la revendication 14, dans lequel l'inhibiteur de développement est un radical choisi dans le groupe constitué des radicaux représentés par les formules générales ci-dessous (D-1), (D-2), (D-3), (D-4), (D-5), (D-6), (D-7), (D-8), (D-9), (D-10), (D-11) et (D-12):

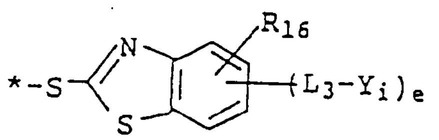
(D-1)

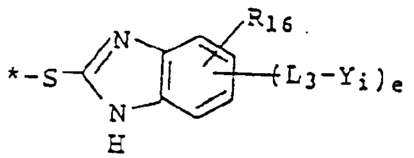
(D-2)

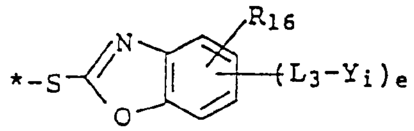
(D-3)

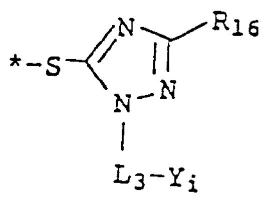
(D-4)

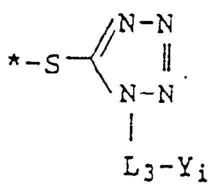
(D-5)

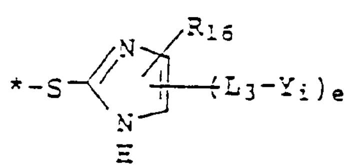
(D-6)

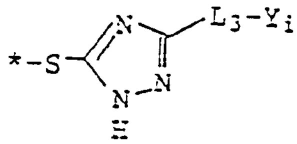

(D-7)

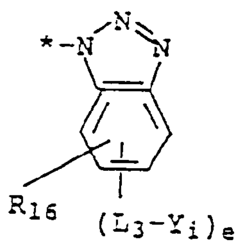

(D-8)

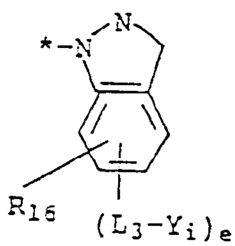

(D-9)

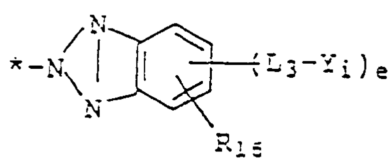

(D-10)

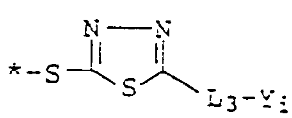

(D-11)

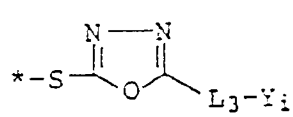

(D-12)

dans lesquelles une liaison indiquée par * désigne la position dans laquelle le radical est relié au radical méthine ou au radical de liaison (L) de la formule générale (II); $R_{16}$ représente un atome d'hydrogène ou un substituant; e représente 1 ou 2; $L_3$ représente un radical contenant une liaison chimique capable d'être rompue dans une solution de développement; et $Y_i$ représente un substituant capable de générer la fonction d'inhibition du développement, et est choisi parmi un radical aliphatique, un radical aromatique ou un radical hétérocyclique.

**16.** Matériau photographique couleur à l'halogénure d'argent sensible à la lumière selon la revendication 15, dans lequel le substituant représenté par $R_{16}$ est choisi parmi un radical aliphatique, un radical acylamino aliphatique ou aromatique, un radical alkoxy, un atome d'halogène, un radical nitro, et un radical sulfonamido.

**17.** Matériau photographique couleur à l'halogénure d'argent sensible à la lumière selon la revendication 15, dans lequel la liaison chimique comprise dans $L_3$ est choisie parmi -COO-, -NHCOO-, $-SO_2O-$, $-OCH_2CH_2SO_2-$,

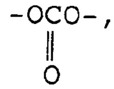

et

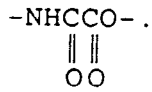

**18.** Matériau photographique couleur à l'halogénure d'argent sensible à la lumière selon la revendication 1, dans lequel la quantité du composé se situe dans la plage de $1x10^{-6}$ mole à 0,5 mole par mole d'argent présente dans la même couche que celle à laquelle il doit être ajouté ou dans une couche adjacente à celle-ci.

**19.** Matériau photographique couleur à l'halogénure d'argent sensible à la lumière selon la revendication 1, dans lequel ladite couche d'émulsion d'halogénure d'argent contient un copulant formateur d'image couleur, dans un rapport molaire de 0,01/99,99 à 50/50, du copulant au composé représenté par la formule (II).